# EUROPEAN PATENT SPECIFICATION

(11) **EP 3 997 464 B1**
(45) Date of publication and mention of the grant of the patent: **09.09.2026**
(21) Application number: 20736378.9
(22) Date of filing: 09.07.2020
(51) Int. Cl.: G01N 33/58

(54) **LIGANDS FOR CAPTURING MICROVESICLES AND USES THEREOF**
LIGANDEN ZUR ERFASSUNG VON MIKROVESIKELN UND DEREN VERWENDUNGEN
LIGANDS POUR LA CAPTURE DE MICROVÉSICULES ET LEURS UTILISATIONS

(30) Priority: 10.07.2019 EP 19185572
(43) Date of publication of application: 18.05.2022
(73) Proprietor: Université de Bordeaux, 33000 Bordeaux (FR); Institut Polytechnique de Bordeaux, 33400 Talence (FR); Centre National de la Recherche Scientifique, 75016 Paris (FR)
(72) Inventor: DURRIEU, Marie-Christine, 33000 Bordeaux (FR); NLATE, Sylvain, 33600 Pessac (FR); CHANSEAU, Christel, 33000 Bordeaux (FR); JIANG, Jian-Qiao, Lanzhou Gansu, 730000 (CN)
(74) Representative: Santarelli
(86) International application number: PCT/EP2020/069406
(87) International publication number: WO 2021/005167

(56) References cited:
- US-A1- 2008 171 351
- US-A1- 2011 159 481
- US-A1- 2014 011 219
- US-A1- 2015 209 450
- ADI DAHAN ET AL: "Pd Catalysis on Dendronized Solid Support: Generation Effects and the Influence of the Backbone Structure", JOURNAL OF THE AMERICAN CHEMICAL SOCIETY, vol. 129, no. 18, 2007, pages 5860 - 5869, XP055668940
- SHUZHANG XIAO ET AL: "Convenient synthesis of multivalent zinc(II)-dipicolylamine complexes for molecular recognition", TETRAHEDRON LETTERS, vol. 54, no. 8, 2013, pages 861 - 864, XP055605113
- JIAN-QIAO JIANG ET AL: "Dendron-Functionalized Surface: Efficient Strategy for Enhancing the Capture of Microvesicles", ISCIENCE, vol. 21, 22 November 2019 (2019-11-22), pages 110 - 123, XP055669199
- W. MATTHEW LEEVY ET AL: "Selective recognition of bacterial membranes by zinc(ii)-coordination complexes", CHEMICAL COMMUNICATIONS, no. 15, 2006, pages 1595, XP055426777
- BRYAN A. SMITH ET AL: "Optical Imaging of Mammary and Prostate Tumors in Living Animals using a Synthetic Near Infrared Zinc(II)-Dipicolylamine Probe for Anionic Cell Surfaces", JOURNAL OF THE AMERICAN CHEMICAL SOCIETY, vol. 132, no. 1, 2010, pages 67 - 69, XP055668329
- KASEY J. CLEAR ET AL: "Phenoxide-Bridged Zinc(II)-Bis(dipicolylamine) Probes for Molecular Imaging of Cell Death", BIOCONJUGATE CHEMISTRY, vol. 27, no. 2, 2016, US, pages 363 - 375, XP055668543
- UMME AYESA ET AL: "Liposomes Containing Lipid-Soluble Zn(II)-Bis-dipicolylamine Derivatives Show Potential To Be Targeted to Phosphatidylserine on the Surface of Cancer Cells", MOLECULAR PHARMACEUTICS, vol. 14, no. 1, 2017, pages 147 - 156, XP055564205
- PICHANDI ASHOKKUMAR ET AL: "A fluorogenic BODIPY molecular rotor as an apoptosis marker", CHEMICAL COMMUNICATIONS, vol. 55, no. 48, 11 June 2019 (2019-06-11), pages 6902 - 6905, XP055668551
- BRYAN A. SMITH ET AL: "Enhanced Cell Death Imaging Using Multivalent Zinc(II)-bis(dipicolylamine) Fluorescent Probes", MOLECULAR PHARMACEUTICS, vol. 10, no. 9, 2013, pages 3296 - 3303, XP055390934

## Description

### Field of the invention

The invention relates to synthetic ligands for capturing microvesicles and their use in the diagnostic and prognostic of diseases in a subject.

### Background of the invention

Microvesicles (also known as cellular microparticles) are submicron extracellular vesicles that are found both in the interstitial space between cells and in body fluids. Microvesicles are released from the surface or plasma membrane of cells by the outward budding and fission of the plasma membrane. The budding process involves multiple signaling pathways including the elevation of intracellular calcium and reorganization of the cell's structural scaffolding. The budding takes place at unique locations on the cell membrane that are enriched with specific lipids and proteins reflecting their cellular origin. At these locations, proteins, lipids, and nucleic acids are selectively incorporated into microvesicles and released into the surrounding environment. Microvesicles play a role in intercellular communication and can transport molecules such as mRNA, miRNA, mitochondrial DNA and proteins between cells.

Microvesicles are released during cell activation or during apoptosis, in the course of various pathological conditions, including cancer, inflammation or and a variety of diseases such as diabetes, Parkinson disease, cardiovascular disease with increased risk of thrombosis or even neurovascular ischemic conditions.

Microvesicles have a high density of phosphatidylserines exposed on the outer leaflet. They also expose a variety of cell surface proteins according to their cell origin and the type of pathology or physiological event inducing their release. Moreover, it was shown that an increase in microvesicles can be correlated with the onset and presence of diseases in patients. For instance, increased leukocyte-derived microvesicles levels have been reported in the blood of patients with sepsis or with high atherothrombotic risk. It is also believed that microvesicles play an important role in the regulation of intercellular communication, inflammatory response and vascular function.

Thus, it was proposed to detect, quantify and/or analyze microvesicles present in body fluids so as to diagnose and assess the risk of developing various diseases and associated disorders, in particular thrombotic, inflammatory and/or metabolic disorders in patients.

Methods for detecting and quantifying microparticles have been described in the prior art. Flow cytometry is the most widely used. This technique allows the measurement and the identification of the phenotype of the microvesicles by using (CD)-specific antibodies. It is also possible to use Annexin A5 as a probe to mark microvesicles due to the high affinity of A5 for phosphatidylserine molecules present in the out layer of microvesicles.

Methods for capturing/quantifying microparticles have been also proposed and are based on the use of solid supports such as microplates or magnetic beads having thereon a ligand able to specifically capture microvesicles. These ligands include Annexin 5, antibodies as well as synthetic ligands.

For instance, Cointe et al. (J Extracell vesicles, 2018, 7(1):1494482) describe a bio-assay for capturing leukocyte-derived microvesicles and the measurement of their plasmin generation capacity, for instance for the prognostic of the outcome of sepsis in patients.

The International patent application WO 2012/127175 describes dinuclear metal complexes which can be used as ligands to specifically capture microvesicles and their uses in the diagnosis of diseases.

US 2014/011219 A1 discloses solid-phase chelators and electronic biosensors.

However, there is still a need for alternative methods for capturing microvesicles from biological samples, in particular in the context of diagnosis of diseases.

### Summary of the invention

The invention is as defined in the appended claims.

The disclosure relates to a molecule able to bind to microvesicles which comprises at least one, preferably two moieties of formula (I):

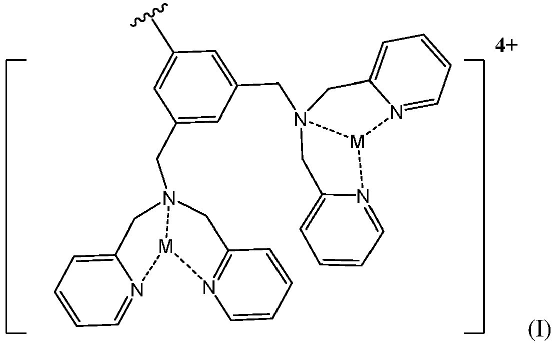

Wherein M is a metal cation preferably selected from the group consisting of Zn²⁺, Mn²⁺, Co²⁺, Ni²⁺, Cu²⁺ and Fe²⁺,

The molecule of the invention comprises at least one moiety of formula (Ia) as follows:

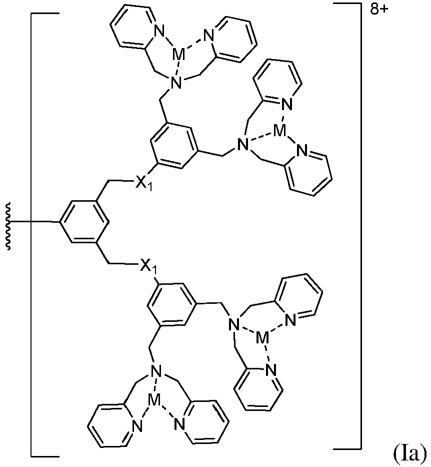

For instance, the molecule of the invention may have at least one moiety of formula (Ib): 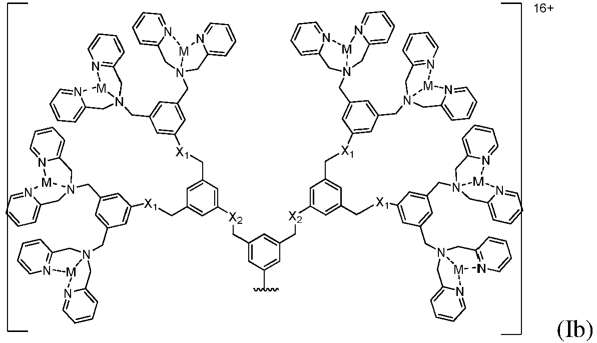 the moiety of formula (Ia) or (Ib) is such that :
- each X₁ is independently O, S, or NH,
- each X₂, when present, is independently O, S or NH,
- M is selected from Zn²⁺, Co²⁺, Cu²⁺ and Fe²⁺, preferably Zn²⁺.

In some embodiments, the molecule of the invention, as defined in claim 8 or 9 as appended, is a dendrimer comprising a branched core bearing a plurality of moieties of formula (Ia) or (Ib), said branched core preferably comprising a group selected from 3,5-di(hydroxymethyl) phenol, 3,5-di(thiomethyl)phenol, 3,5-di(thiomethyl) thiophenol, 3,5-dialkylphenol, 3,5-di(aminomethyl)phenol, and 3,5-di(aminomethyl) phenylamine as building block, more preferably comprising 3,5-di(hydroxymethyl) phenol as building block. In some alternative or additional embodiments, the molecule of the invention may further comprise a mean for immobilization on a support attached at the extremity of a spacer chain.

For instance, the molecule of the disclosure may be of formula (II) 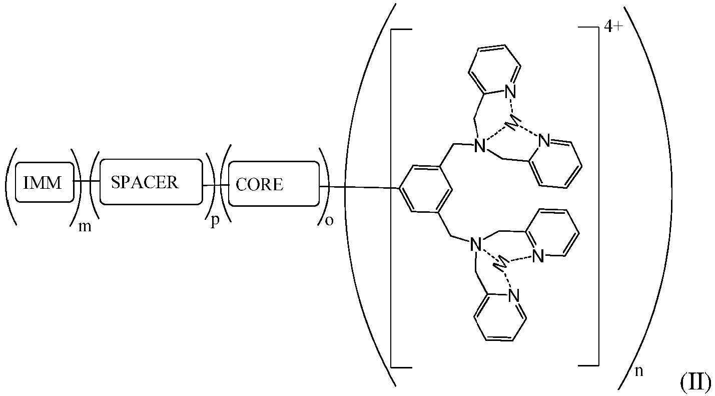 wherein:
- n is an integer from 1 to 10, preferably from 2 to 6, more preferably 2 or 4,
- m, p and o are independently 0 or 1,
- M is a divalent metal cation,
- [CORE] is a chemical entity bearing the at least one moiety of formula (I) and having a molecular weight of at most 25 000 g.mol⁻¹, preferably of at most 10 000 or 5000 g.mol⁻¹
- [IMM] is a mean for covalently or non-covalently immobilization on a support, and
- [SPACER] is selected from the group consisting of a peptide, a polypeptide, an oligo- or polysaccharide, a saturated or unsaturated hydrocarbon chain optionally interrupted by one or several heteroatoms (e.g. S, O or NH), optionally having an heteroatom such as S, O and NH on at least one of its ends, and optionally substituted by one or several substituents such as hydroxyl, halogens, C₁-C₃ alcoxy, -CN, -CF₃, or C₁-C₃ alkyl, polymers including homopolymers, copolymers and block polymers, and combinations thereof.

Said molecule may further be characterized by one, several or all of the following features:
- m, p and o are 1,
- M is selected from Zn²⁺, Co²⁺, Cu²⁺ and Fe²⁺, preferably Zn²⁺.,
- [SPACER] is selected from C₂-C₂₀ saturated or unsaturated hydrocarbon chains optionally substituted, polyamide chains, polyester chains and polyether chains, such as polyethylene glycol, comprising from 2 to 20 monomers and combinations thereof.
- [IMM] comprises, or consists of, a moiety selected from the group consisting of an amino group, preferably -NH₂, OH, -COOH, an activated carboxylic acid, -SH, iodoacetyl group, a carbonyl, a hydrazide group, an azido, and a strained alkyne.
- [CORE] is a chemical moiety made of at least one 3,5-di(hydroxymethyl) phenol.

In particular, it is disclosed a compound of formula (IIa):

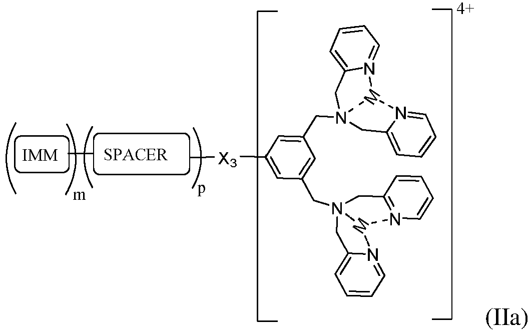

In some embodiments, the molecule of the invention may be a compound of formula (IIb) or (IIc): 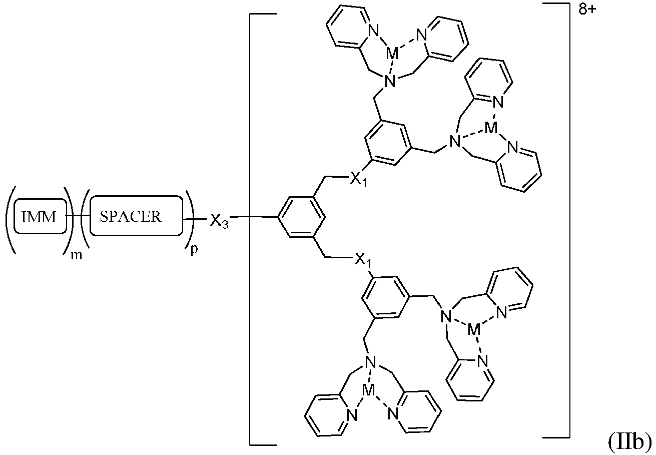 -or 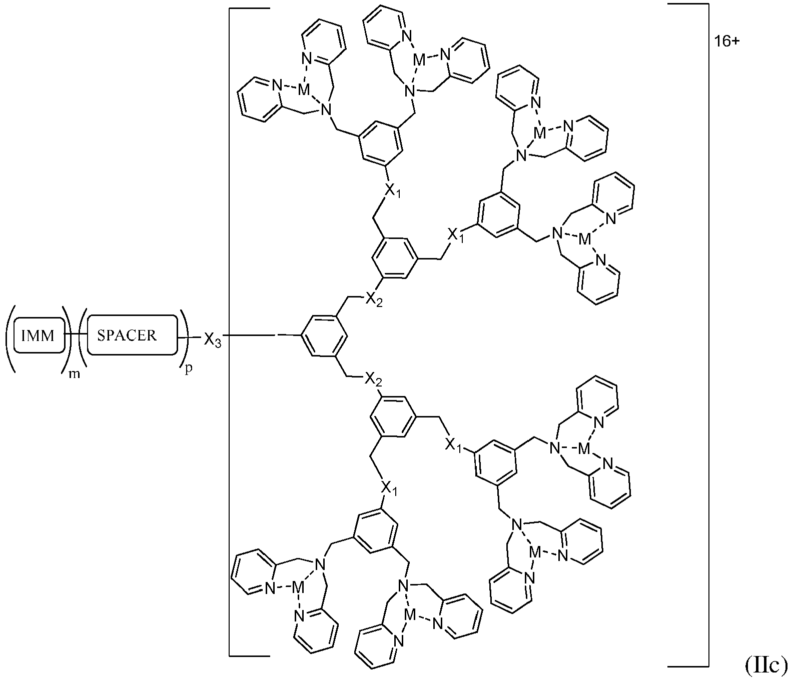 wherein
- M, m, p, [IMM] and [SPACER] are as defined above for formula (II),
- each X₁ and X₂, when present, are selected from the group consisting of O, NH, and S,
- X₃ is selected from -O-, C(=O) -OC(O)-, -C(O)O-, -OC(O)O-, -S-, -SS-, -SC(O)-, - OC(S)-, NR₁-, -NR₁C(O)-, -C(O)NR₁-, NR₁C(S)-, C(S)NR₁-, -OC(O)S-, -OC(S)O-, - SC(O)O-, -OC(S)S-, -SC(O)S-, -SC(S)O-, -SC(S)S-, OC(O)NR₁-, -OC(S)NR₁-, - NR₁C(S)O-, -NR₁C(O)S-, NR₁C(O)NR₂-, -NR₁C(S)NR₂-, -SC(O)S-, -SC(S)O-, -S(O)-, -S(O)₂-, - -P(O)(R₁)-, -P(O)(OR1)-, P(O)(R₁)O-, OP(O)(OR₁)-, OP(O)(R₁)O-, NR₁P(O)(R₂)-, -NR₁P(O)(OR₂)-, NR₁P(O)(R₂)O-, OP(O)(OR₁₎₋ and OP(O)(R₁)O-wherein R₁ and R₂ are independently H or CH₃.

For instance said molecule of formula (IIa), (IIb) or (IIc) may be characterized by:
- m and p are 1,
- M is Zn²⁺, Co²⁺, Cu²⁺ or Fe²⁺, preferably Zn²⁺.,
- X₁ and X₂, when present, are O,
- X₃ is O, NH, NHCO, CONH, O(C=O), (O=C)O, O(C=O)O, or NHCONH,
- [IMM]-[SPACER] is NH₂-(CH₂)ᵣ- or or NH₂-[(CH₂)₂-O]ᵣ- with r an integer from 2 to 10, and
- Said molecule comprises one or several counter-anions selected from the group consisting of perchlorate, tosylate, nitrate, sulphate, sulphonate, thiosulfate, halide, hexafluorophosphate, tetraphenylborate, carbonate, and tetrafluoroborate.

Preferably the molecule is a compound of formula (IIb) or (IIc).

Examples of molecules of the invention are ligands of formula (IIIb) and (IIIc): 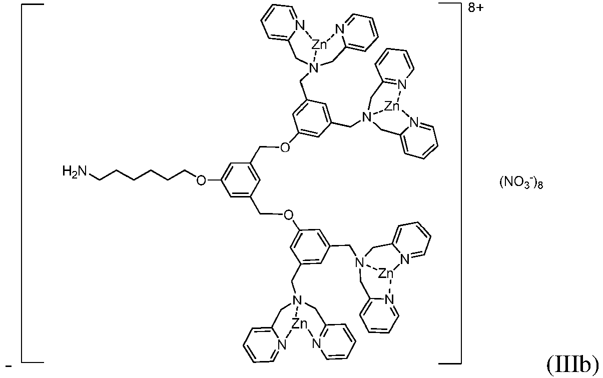 and

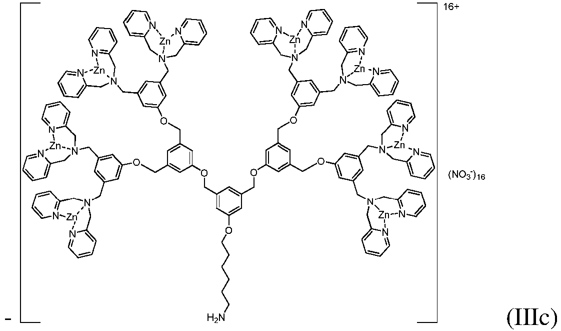

The invention also relates to a support for capturing microvesicles, which comprises a molecule according to formula (1a), (1b), (IIb), (IIc), (IIIb) or (IIIc) on its surface, and a support comprising such a surface.

The invention also relates to an *in vitro* method for capturing microvesicles from a sample, which comprises contacting the sample with a molecule or a support as defined above. Said *in vitro* method of may further comprise at least one step selecting from:
- a step of providing a sample susceptible to contain the microvesicles of interest from a body fluid of a subject, and/or
- a step of washing the support after the step of contacting the support with the sample, and/or
- a step of detecting the formation of the complex between the microvesicles of interest and said support or molecule of the invention and/or
- a step of recovering the complex formed between the microvesicles and the molecules of the invention, and/or
- a step of quantification of said microvesicles of interest, and/or
- a step of characterization of the captured microvesicles, e.g. by detecting and/or quantifying a biomarker present on the surface or within the microvesicles, and/or
- a step of releasing the microvesicles from the complex, and/or
- a step of recovering the microvesicles of interest.

The invention also relates to an *in vitro* method for the diagnosis, the differential diagnosis, the prognosis, the assessment of the risk of, and/or the monitoring a disorder in a subject which comprises:
(a) providing a support or a molecule of the invention,
(a) contacting said support or said molecule with a sample obtained from the subject, in conditions suitable for the formation of a complex between the molecules of the invention and the microvesicles possibly present in the sample, and
(b) detecting, quantifying and/or characterizing the microvesicles captured in step (b) by complexation.

Typically, the disorder may be selected from the group consisting of cancer, parasitic diseases, diabetes and related disorders such as diabetic nephropathy, diabetic neuropathy, diabetic retinopathy, and diabetic foot syndrome, multiple sclerosis, cancer, Alzheimer's disease, Parkinson's disease, aneurysm, cerebral vasospasm, stroke, and coronary artery disease, and/or the sample may be or derive from, blood, blood plasma and urine.

A further object of the invention is the use of a molecule, or a support as defined above as means for capturing microvesicles from a sample of a subject, preferably for the diagnosis, the prognostic or the monitoring of a disease in a subject such cancers, parasitic diseases, as diabetes and related disorders including diabetic nephropathy, diabetic neuropathy, diabetic retinopathy, and diabetic foot syndrome, multiple sclerosis, cancer, Alzheimer's disease, Parkinson's disease, aneurysm, cerebral vasospasm, stroke, and coronary artery disease.

### Figures

Figure 1 PWR titration of different complex solutions to PSPC membrane. A, B: Fitting of Cplx1-PSPC membrane titration in s and p polarization, respectively; C, D: Fitting of Cplx2-PSPC membrane titration in s and p polarization, respectively; E, F: Fitting of Cplx4-PSPC membrane titration in s and p polarization, respectively. PSPC membrane composition: PS 1 equivalent, PC 5 equivalent. The fitting curves were acquired using GraphPad Prism 7.
Figure 2: TBO calibration curve of carboxyl group density plotted against solution absorbance at 633 nm.
Figure 3: Carboxyl group density on treated PET surfaces (unit: pmol/mm²) determined by TBO experiment.
Figure 4: Cplx1 grafted PET surface incubated with microvesicles. A: fluorescence micrograph, B: cryo-SEM micrograph. Scale bars are provided within the images.
Figure 5: Cplx2 grafted PET surface incubated with microvesicles. A: fluorescence micrograph, B: cryo-SEM micrograph. Scale bars are provided within the images.
Figure 6: Cplx4 grafted PET surface incubated with microvesicles. A: fluorescence micrograph, B: cryo-SEM micrograph. Scale bars are provided within the images.
Figure 7: PET surface grafted with cplx8 incubated with microvesicles. A: fluorescence micrograph, B: cryo-SEM micrograph. Scale bars are provided within the images.

### Detailed description of the invention

The Inventors conceived new synthetic ligands able to selectively bind to microvesicles with high affinity. These ligands are metal complex comprising a plurality of metal(II)-bis(dipicolylamine) (M-BDPA) moieties able to interact with phosphatidylserine present in the outer leaflet of the microvesicles. Without to be bound by any theory, the Inventors are of the opinion that the number of M-BDPA and their proximity in the ligands of the invention enable to optimize the binding to phospholipid-enriched microvesicles with high specificity.

As illustrated in the examples, the ligands of formula (IIIa), (IIIb) and (IIIc) have dissociation constant (Kd) for enriched-phosphastidylserine microvesicles in the 10⁻⁶ M range. When immobilized on a support, these ligands were able to capture microvesicles. In contrast, support grafted with the ligand of formula (IIId), which comprises one dipicolylamine moiety only, was shown to have very limited ability to capture microvesicles.

The ligands of the invention were shown to be effective ligands to capture microvesicles on a support, enabling the subsequent detection, quantification and characterization of microvesicles. Noteworthy, the multivalent ligand of formula (IIIb) (also called Cplx4 in the Example section) was able to capture the microvesicles while maintaining their shape, making it possible to detect and quantify biomarkers of interest present within the membrane of the microvesicles but also present inside the microvesicles. ³¹P NMR analyses confirmed that Cplx4 was the complex having the highest interaction with POPS among the tested compounds. Of note, the interactions were higher with Cplx4 than with Cplx8, showing a negative dendritic effect for large ligands.

The possibility to detect biomarkers in the membrane and/or inside the microvesicles is of high interest in the conception of diagnostic methods, because it was shown that the microvesicle proteome reflect the pathological status of the origin cells.

Thus, the ligands of the invention find applications in *in vitro* diagnosis and prognostic of various disorders.

### - Ligands according to the invention

In a first aspect, the disclosure relates to a ligand binding to microvesicles, preferably phosphatidylserine-enriched microvesicles, comprising a plurality of metal(II)-bis(dipicolylamine) (M-BDPA) moieties.

More precisely, the disclosure relates to a molecule able to bind to microvesicles comprising at least one moiety (e.g. at least 2, 3, or 4 moieties) of formula (I): 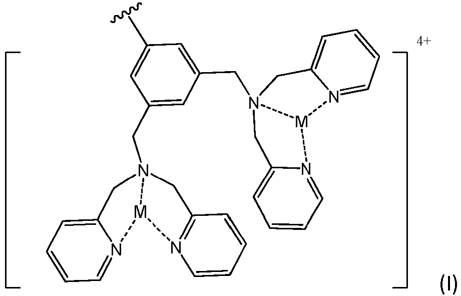 wherein M is a metal cation, preferably a divalent metal cation.

In some embodiments, M is selected from the group consisting of Zn²⁺, Mn²⁺, Co²⁺, Ni²⁺, Cu²⁺ and Fe²⁺, preferably Zn²⁺, Fe²⁺, Co²⁺ and Cu²⁺, more preferably Zn²⁺.

In a preferred embodiment, the molecule of the disclosure comprises at least two moieties of formula (I), e.g. 2, 3, or 4 moieties of formula (I).

Typically, the molecule of the disclosure has a molecular weight of at most 25000 g.mol⁻¹, preferably of at most 20000, e.g. of at most 15000, 10000, 7000, 5000 or 3000 g.mol⁻¹.

As explained in the background of the invention, *"Microvesicles"* (also known as cellular microparticles) refer to submicron extracellular vesicles that are found both in the interstitial space between cells and in body fluids. Microvesicles are released from the surface or plasma membrane of cells by the outward budding and fission of the plasma membrane. Microvesicles play a role in intercellular communication and can transport molecules such as mRNA, miRNA, and proteins between cells.

Phosphatidylserine (PS) roughly accounts for 2-10% of total cellular phospholipids in a typical mammalian cell plasma membrane. In healthy cells, PS is almost exclusively sequestered to the inner leaflet of the cell. Under certain conditions, in particular in cell death pathway, PS appears in the outer leaflet of the plasma membrane, due to the attenuation of the active transport mechanisms that maintains the asymmetric transmembrane distribution of PS. It was shown that microvesicles exhibit phosphatidylserine in the outer leaflet of their membrane.

As used herein, *"phosphatidylserine-enriched microvesicles or phosphatodylserine-rich microvesicles"* refer to microvesicles in which PS can be found in the outer leaflet of the membrane of the vesicles. In other words, *"phosphatidylserine-enriched microvesicles"* refer to microvesicles having PS present in the outer leaflet of the bilayer lipid membrane. For example, such microvesicles may derive from cells in which a cell death pathway is activated. Synthetic vesicles made of POPC (1-palmitoyl-2-oleoyl-sn-glycro-3phosphocholine) and POPS (1-palmitoyl-2-oleoyl-sn-glycro-3phosphoserine) with a weight ratio of 5:1 (w/w) can be used as a model of *"PS-enriched microvesicles"* in order to assess the ability of molecules to bind to microvesicles in vitro

As used herein, *"a molecule binds to a microvesicle"* means that the molecule is able to bind to a microvesicle, preferably a phosphatidylserine-enriched microvesicle. Such a molecule herein also refers to *"ligand of microvesicles"* or *"ligand".*

Preferably *"a molecule binds to a microvesicle"* refers to a molecule able to bind a microvesicle with high affinity e.g. with a dissociation constant (Kd) of at most 10⁻³ M such as at most 10⁻⁴ or 10⁻⁵ M.

The Kd is preferably determined by plasmon waveguide resonance spectroscopy, as illustrated in Example 2. Such assay is performed by using vesicles prepared by mixing POPC with POPS with a weight ratio of 5:1 (w/w) and 1x Dulbecco's Phosphate Buffered Saline (pH 7.1-7.5) as medium.

Alternatively, it is possible to identify whether a molecule is able to bind to microvesicles by a capture assay using e.g. cryo-scanning electron microscopy (Cryo-SEM) as illustrated in Example 4. Typically, the molecule to assess is immobilized on a support and then incubated with a solution comprising microvesicles in phosphate saline buffer. After washing and cryofixation, the surface of support is analyzed by Cryo-SEM so as to determine whether microvesicles are immobilized thereon.

In some embodiments, the molecule of the invention binds to microvesicles with specificity, which means that their affinity for microvesicles (i.e. phosphatidylserine-enriched microvesicles) is higher than that for other cellular particles such as exosomes and more generally for cellular particles which do not exhibit PS in the outleaflet of their membrane. For instance, the molecule of the invention may have a Kd for a microvesicle which is at least 5-fold, preferably, at least 10, 20, 30, 40, 50, 100, 200, 500, or 1000-fold lower than its Kd for another cellular microparticles such as exosomes. In some embodiments, the molecule of the invention do not cross-react with cellular particles other than microvesicles, i.e. has relatively little detectable reactivity with other molecules which may be present in the sample.

The ligand of the invention can comprise one or several counter-anions to counterbalance the positive charge of the moieties of formula (I). The counter-anion may be selected from the group consisting of perchlorate, tosylate, nitrate, sulphate, sulphonate, thiosulfate, halide, hexafluorophosphate, tetraphenylborate, carbonate, and tetrafluoroborate, more particularly from perchlorate, nitrate, sulphate, halide and carbonate anions. In some preferred embodiments, the counter-anion(s) is/are selected from nitrate and perchlorate.

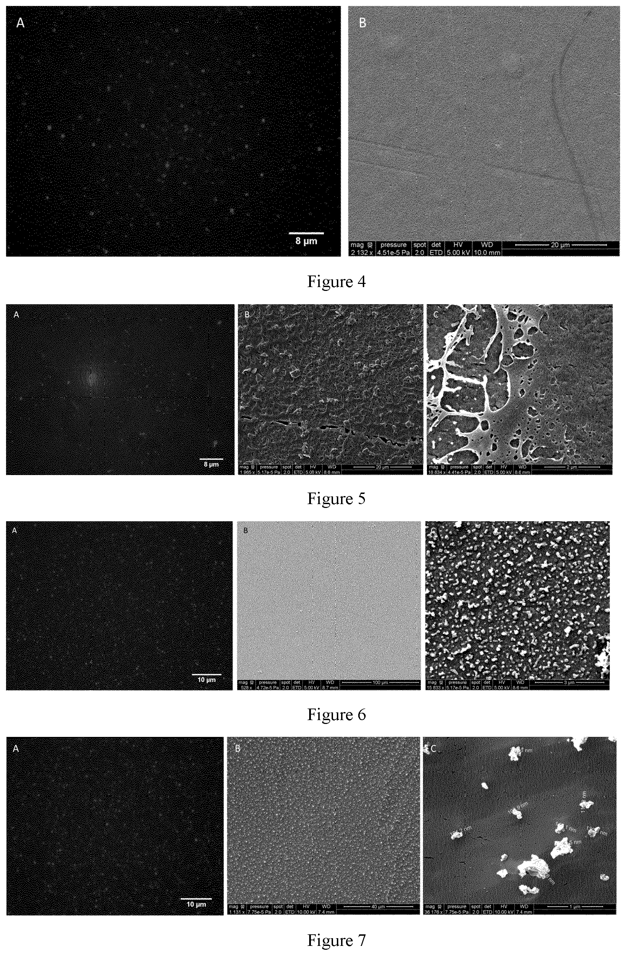

As shown in Example 3, the ligand of formula (IIIb) (called Cplx4 in the Example section) is particularly effective to capture microvesicles while maintaining their integrity. Indeed, Figure 6 shows that the captured microvesicles uniformly covered the surface functionalized with Cplx4 without any significant fusion or aggregates between them, whereby the integrity of microvesicles is maintained.

Noteworthy, the ligand of formula (IIIb) bears two moieties of formula (I).

Thus, in a preferred embodiment, the molecule of the description comprises at least two moieties of formula (I).

For instance, the ligand of the disclosure comprises at least one moiety of formula (1c) (e.g. 1, 2, 3 or 4 moieties): 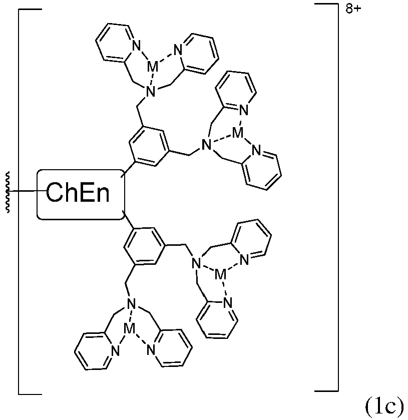 Wherein:
- M is a divalent metal cation. M may be selected from the group consisting of Zn²⁺, Mn²⁺, Co²⁺, Ni²⁺, Cu²⁺ and Fe²⁺, preferably Zn²⁺, Fe²⁺, Co²⁺ and Cu²⁺, more preferably Zn²⁺, and
- [ChEn] is a chemical entity having a molecular weight of at most 20000 g.mol⁻¹, preferably of at most 10000, 7000, 5000, 3000 or 2000 g.mol⁻¹. [ChEn] may comprise acyclic and/or cyclic moieties. For instance, [ChEn] may be selected among bifunctional, trifunctional and tetrafunctional linkers having a backbone of 1 to 60 carbon atoms and at least two heteroatoms independently selected from N, S and O. Alternatively or additionally, [ChEn] may comprise one or several cyclic groups (e.g. at least 2, 3, 4, 5, or 6) preferably selected from pyrrole, furan, thiophene, pyrazole, imidazole, oxazole, triazole, triazine, phenyl, naphthalene, pyridine, piperidine, pyridazine, pyrimidine, pyrazine, oxazine, dioxine, piperazine, morpholine, and thiazine.

In some embodiments, "ChEn" may be of formula: 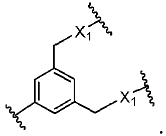 In such a case, the ligand of the invention comprises at least one moiety of formula (1a): 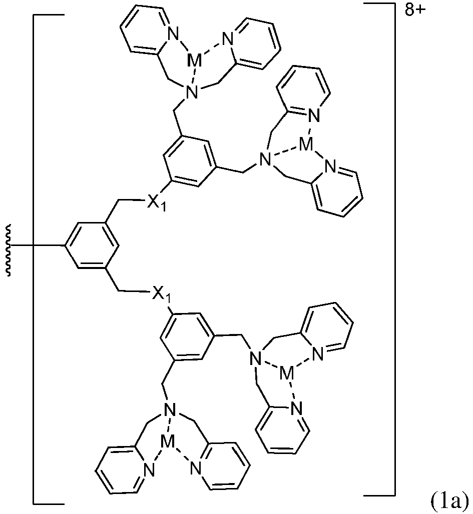 wherein:
- each X₁ is independently selected from S, O and NH. Preferably, all the X₁ are identical, preferably O, and
- M is as defined above, preferably Zn^{2+.}

In another embodiment, the ligand of the invention comprises two moieties of formula (1a). For instance, the ligand of the invention comprises the moiety of formula (1b): 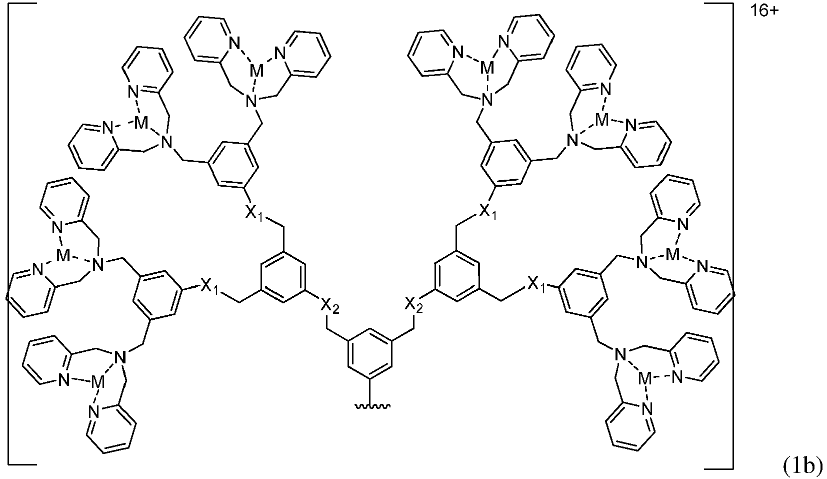 wherein
- M is as defined above
- X₁ are as defined above, and
- each X₂ is independently O, NH, or S,

In some embodiments, all X₁ and X₂ are the same, e.g. S, O or NH, preferably O.

In a particular embodiment, the ligand of the disclosure is a dendrimer comprising a branched core bearing a plurality of moieties of formula (I), preferably at least 2, 4, 6 or 8 moieties of formula (I). Said dendrimer comprises from 1 to 8, preferably from 1 to 4, e.g. 1, 2 or 3 layers of molecules that emanate radially from a central core. The outermost layer is the layer bearing the moieties of formula (I). The building block of the dendrimer may be selected from the group consisting of 3,5-di(hydroxymethyl) phenol, 3,5-di(thiomethyl)phenol, 3,5-di(thiomethyl) thiophenol, 3,5-dialkylphenol, 3,5-di(aminomethyl)phenol, 3,5-dialkylphenol and 3,5-di(aminomethyl) phenylamine.

In some embodiments, the building block of said dendrimer is 3,5-di(hydroxymethyl) phenol. Accordingly, the ligand of the invention may comprise a branched core based on the following repeated unit:

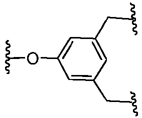

As mentioned above, the molecule of the invention finds applications in the detection, the quantification and the analysis of microvesicles. Consequently, the molecule of the invention may comprise one or several additional moieties enabling to retrieve and/or to detect the complex formed between the molecules of the invention and microvesicles. For instance, the molecule of the invention may comprise one or several moieties selected from the group consisting of a reporter moiety and/or a mean for immobilization on a support.

A mean for immobilization on a support is described further below (*see the definition given for [IMM]*). On the other hand, the report moiety may be selected from the group consisting of a fluorescent molecule, a dye, a biotin, a radioactive agent, a quantum dot, an enzyme such as horseradish peroxidase and the like.

In a particular embodiment, the molecule of the invention is to be immobilized on a support. In that case, the molecule of the invention may further comprise a mean for immobilization attached on a spacer chain. More precisely, the molecule of the disclosure may comprise or may be of formula (II): 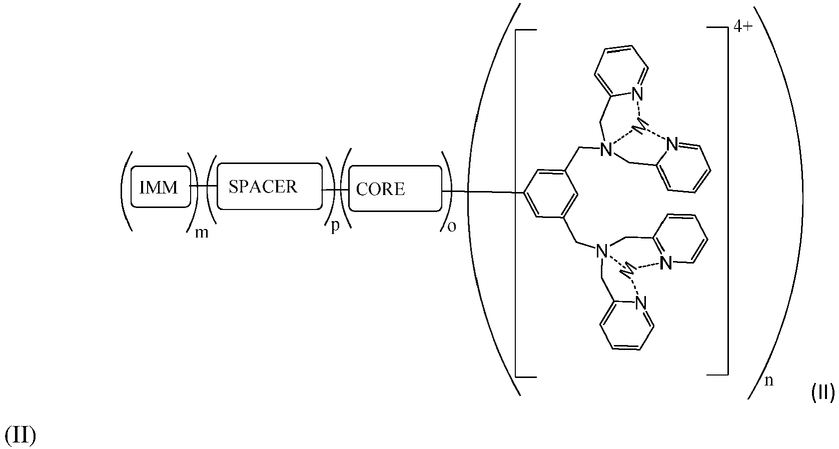 Wherein:
- n is an integer from 1 to 10, preferably from 2 to 6, more preferably 2, 3 or 4,
- m, p and o are independently 0 or 1,
- M is a divalent metal cation as defined above,
- [CORE] is a chemical entity having a molecular weight of at most 25000 g.mol⁻¹,
- [IMM] is a mean for covalently or non-covalently immobilization on a support.
- [SPACER] is selected from the group consisting of a peptide, a polypeptide, an oligo- or polysaccharide, a saturated or unsaturated hydrocarbon chain optionally interrupted by one or several heteroatoms (e.g. S, O or NH), optionally having at least one of its extremity an heteroatom such as S, O and NH, and optionally substituted by one or several substituents such as hydroxyl, halogens, C₁-C₃ alcoxy, -CN, -CF₃, or C₁-C₃ alkyl, polymers including homopolymers, copolymers and block polymers, and combinations thereof.

"m is 0" means that IMM is absent and when "m is 1" means that IMM is present.

"p is 0" means that SPACER is absent and when "p is 1" means that SPACER is present.

"o is 0" means that CORE is absent and when "o is 1" means that CORE is present.

The molecule of the invention may have a molecular weight of at most 25000 g.mol⁻¹, preferably of at most 20000, e.g. of at most 15000, 10000, 7000, 5000 or 3000 g.mol⁻¹.

As mentioned above, said molecule may comprise one or several counter-anions to counterbalance the positive charges of the metallic complexes. Said counter-anions are as defined above and are preferably selected from the group consisting perchlorate, tosylate, nitrate, sulphate, sulphonate, thiosulfate, halide, hexafluorophosphate, tetraphenylborate, carbonate, and tetrafluoroborate, more particularly from perchlorate, nitrate, sulphate, halide and carbonate anions. In some preferred embodiments, the counter-anion(s) is/are selected from nitrate and perchlorate.

[CORE] is typically a chemical moiety having a molecular weight of at most 20000 g.mol-1, preferably of at most 10000, 7000, 5000, 3000 or 2000 g.mol⁻¹. [CORE] may comprise acyclic and/or cyclic moieties. For instance, [CORE] may be selected among O, NH, S, and polyfunctional linkers, such as bifunctional, trifunctional tetrafunctional, penta-functional, octo-functional and even deca-functional linkers, having a backbone of 1 to 60 carbon atoms and at least two heteroatoms independently selected from N, S and O. Alternatively or additionally, [CORE] may comprise one or several cyclic groups (e.g. at least 2, 3, 4, 5, or 6) preferably selected from pyrrole, furan, thiophene, pyrazole, imidazole, oxazole, triazole, triazine, phenyl, naphthalene, pyridine, piperidine, pyridazine, pyrimidine, pyrazine, oxazine, dioxine, piperazine, morpholine, and thiazine.

In some embodiments, [CORE] is composed of one or several 3,5-dimethoxyphenol moieties. In other embodiments [CORE] is a branched core, preferably a dendritic core, made of 3,5-di(hydroxymethyl) phenol 3,5-di(thiomethyl)phenol, 3,5-di(thiomethyl) thiophenol, 3,5-dialkylphenol, 3,5-di(aminomethyl)phenol, and 3,5-di(aminomethyl) phenylamine and all related oxo, thio and amino derivatives as building block.

As used herein, *"combinations"* means that [SPACER] (hereafter "the spacer") may comprise several hydrocarbon chains, oligomer chains or polymeric chains (e.g. 2, 3, 4, 5 or 6) linked by any appropriate group, such as -O-, -S-, -NHC(O)-, -OC(O)-, -NH-, -NH-CO-NH-, -O-CO-NH-, phosphodiester or phosphorothioate groups.

Typically, the spacer may comprise from 2 to 200 carbon atoms, preferably from 2 to 40, such as 2 to 20 carbon atoms. The length and the chemical nature of the spacer may be optimized depending on the support on which the molecule is to be immobilized and/or in order to optimize the interaction between the molecule and the microvesicles.

In some embodiments, the spacer may be selected from the group consisting of polyethers such as polyethylene glycol (PEG) or polypropylene glycol, polyvinyl alcohol, polyesters such as polylacte, polyacrylate polymethacrylate, polysilicone, polyamide such as polycaprolactone, unsaturated or saturated, branched or unbranched, hydrocarbon chains optionally having an heteroatom such as O, NH and N on at least one end, and combinations thereof.

In some preferred embodiments, [SPACER] is selected from C₂-C₂₀ saturated or unsaturated hydrocarbon chains optionally having an heteroatom such as O, NH and N on at least one extremity, polyether chains comprising from 2 to 20 monomers and combinations thereof.

For instance, the spacer comprises at least one polyethylene glycol moiety comprising from 2 to 20 monomers. For illustration only, the spacer may comprise from 2 to 10 triethyleneglycol blocks linked together by linkers. As another example, the spacer may be a C₁₂ hydrophilic triethylene glycol ethylamine derivative. Alternatively, the spacer may be a C₂-C₄₀ hydrocarbon chain, in particular a C₁₀-C₂₀ alkyl chain or a C₂-C₁₀ alkyl chain such as a C₆ alkyl chain. The alkyl chain may have a group such as NH, S or O on at least one end.

In a particular embodiment, [SPACER] is selected from linear or branched C₂-C₂₀ alkyl chains and a polymer such as a polyethyleneglycol, a polyester, or a polyamide comprising from 2 to 10 monomers. In a particular embodiment, the spacer is selected from C₂-C₂₀ alkyl chains and a polyethyleneglycols having from 2 to 10 monomers. The alkyl chain may have a group such as NH, S or O on at least one of its ends.

[IMM] (hereunder "mean for immobilization") is to be selected depending on the support for immobilization and the type of binding (covalent or non-covalent binding) which is sought.

For instance, [IMM] may comprise a moiety enabling non-covalent binding with a support.

For instance, [IMM] may comprise a biotin while the support may comprise streptavidin or avidin thereon or *vice versa.* [IMM] may comprise a ligand moiety such as a short oligosaccharides, e.g. comprising from 1 to 6 saccharides, a peptide, protein fragments in particular an antigenic fragment while the support has a binding moiety able to specifically binds to said ligand such as antibodies, antibodies fragments or constructs e.g. as Fab, Fc fragment, or ScFv, aptamers, and the like, or *vice versa.*

Preferably [IMM] comprises a chemical reactive group for immobilizing the molecule of the invention by a covalent bound on the support. Said chemical reactive is typically selected so as to react with a chemical entity present on the support of interest in conditions which would not alter the binding specificity of the molecule of the invention, in particular the ability of the molecule to specifically bind to the microvesicles of interest. In other words, [IMM] and the chemical entity present on the support may be selected so as to react together in conditions which are not likely to alter the structure and/or the conformation of the ligand of the invention. For instance, [IMM] may comprise an amino group, preferably a primary amino group (-NH₂), or a primary hydroxyl group and the support may comprise a chemical reactive group able to form a covalent bond with said amino or hydroxyl group or *vice versa.* Chemical reactive groups able to form a covalent bond with an amino or hydroxyl group encompasses, without being limited to, a carboxylic acid group, an activated carboxylic acid group, a lactone group, and an imidazole carbamate.

The term *"activated carboxylic acid group"* is intended to mean a chemical function derived from the *"carboxylic acid"* group capable of reacting with a nucleophile such as a primary amino group. *"Activated carboxylic acid"* groups are well known to those skilled in the art and encompass acyl chlorides, mixed anhydrides and esters.

In some embodiments, the activated carboxylic acid group is in the form of an ester. This ester may result from the reaction of a carboxylic acid group with a compound selected from 1-hydroxybenzotriazole (HOBt), 1-Hydroxy-7-azabenzotriazole (HOAt) and N-hydroxysuccinimide, or a derivative thereof, preferably N-hydroxysuccinimide, or a derivative thereof such as sulfo-NHS or N-bromosuccinimide. In a particular embodiment, the activated carboxylic acid group is a N-hydroxysuccinimidyl ester of the following formula

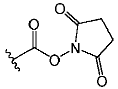

In some other embodiments, [IMM] comprises a sulfhydryl group (-SH) and the chemical entity of the support comprises a chemical reactive group able to form a covalent bond with said sulfhydryl group such as an iodoacetyl group, or *vice versa.* The resulting bond is a thioether bond.

In other embodiments, [IMM] comprises a carbonyl group (-C(=O)-H) and the chemical entity of the support comprises a chemical reactive group able to form a covalent bond with said carbonyl group such as a hydrazide group, or *vice versa.* The resulting bond formed is a hydrazine bond.

In some embodiments, [IMM] may comprise a chemical reactive moiety suitable to create a covalent bond by click-chemistry or by bioconjugation reaction.

Bioconjugation reactions encompass reactions between amino acids such as lysine, cysteine or tyrosine with reactive groups as detailed in Koniev, O., Wagner, A, Chem. Soc. Rev., 44, 5495 (2015). For instance, the functional moiety may comprise a maleimide group or a squarane moiety, which can react with cysteine or tyrosine residues, respectively. The maleimide or the squarane moiety may be present in [IMM] while the amino acid residue may be present on the support and *vice versa.* Bioconjugation reactions encompass, without being limited to:

| | |
|---|---|
| a) Amine conjugation | c) Thiol conjugation |
| 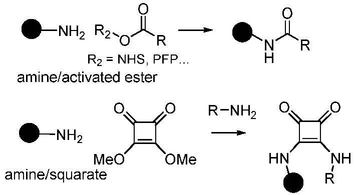 | 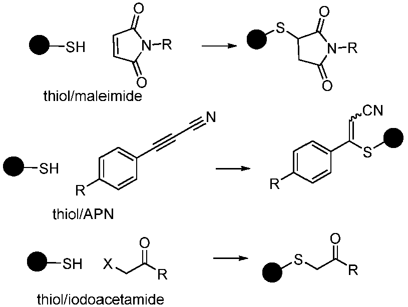 |
| b) Bioconjugation via carbon-nitrogen double bonds. | |
| 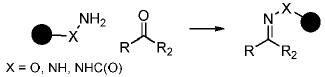 | |

On the other hand, "Click-reaction" or "Click-chemistry" refers to chemical reactions characterized by high yields, high chemoselectivity, which are simple to conduct and which generate inoffensive by-products. Click reactions are typically used to create covalent heteroatom links (C-X-C) between two entities of interest. For review about click chemistry, one can refer to Kolb et al., Angew. Chem. Int. Ed. 2001, 40, 2004-2021 and to Rudolf et al., Current opinion in Chemical Biology, 2013, 17:110-117.

Examples of click reactions encompass, without being limited to, copper-catalyzed azide-alkyne dipolar cycloadditions (CuAAC), strain-promoted alkyne-azide cycloaddition (SPAAC), Diels-Alder reactions with tetrazines and strained alkynes or alkenes, tetrazine-isonitrile cycloadditions, thiol-alkene click reactions such as maleimide-cysteine cycloadditions, Staudinger azide-triarylphosphine conjugation, and sydnone-alkyne cycloadditions.

In some embodiments, the chemical reactive group of [IMM] and the chemical entity of the support may be selected so as to promote a free-metal click reaction. Preferred free-metal click reaction is strain-promoted alkyne-azide 1,3-dipolar cycloaddition (SPAAC). This reaction refers to the reaction between an azido group and a strained alkyne moiety which leads to the formation of a triazole moiety. The azido group may be present in[IMM] and the strained alkyne moiety may be present on the support, and *vice versa.*

Preferred strained alkynes are C₆-C₃₀ alkynes, preferably C₈-C₂₀ alkynes, wherein the triple bond is sterically strained, in particular in a cyclooctyne scaffold. The strained alkyne may comprise a cyclooctyne scaffold which may be optionally substituted by one or several substituents such as halogens and/or fused to one or several cycles, including heterocycles. For instance, the strained alkyne may comprise one of the following cyclooctyne scaffolds (a)-(f):

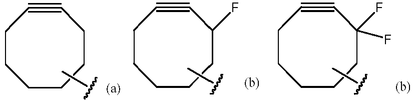

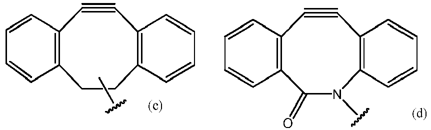

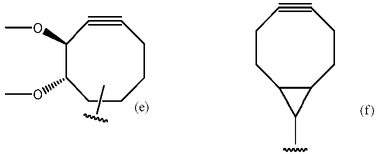

In some other embodiments, [IMM] may comprise a photoreactive group. A photoreactive group, also called photoreactive crosslinker, refers to a chemically inert compound that becomes reactive when exposed to ultraviolet or visible light. Photoreactive groups encompass, without being limited to, aryl azides, azido-methyl-coumarins, benzophenones, anthraquinones, diazo compounds, diazirines, and psoralen derivatives. One can cite as examples of photoreactive groups phenyl azide, ortho-hydroxyphenyl azide, meta-hydroxyphenyl azide, tetrafluorophenyl azide, diazirine, azido-methylcourmarin, benzophenone, and psoralen. A photoreactive group can react with many amino acids. Accordingly, when [IMM] comprises a photoreactive group, the chemical moiety on the support may be an amino acid and *vice versa.* In a particular embodiment, [IMM] comprises, or consists of, a moiety selected from an amino [IMM] comprises, or consists of, a moiety selected from an amino group, preferably -NH₂, -COOH, OH, an activated carboxylic acid, -SH, iodoacetyl group, a carbonyl, a hydrazide group, an azido, and a strained alkyne. For instance, [IMM] comprises, or consists of, a moiety selected from -NH₂-COOH and activated carboxylic acid groups for instance hydroxy-succinimidyle ester.

In a further embodiment, the molecule of the disclosure is of formula (II) wherein:
- n is an integer from 1 to 6, preferably from 2 to 4,
- m and p are 1,
- [SPACER] and [IMM] are as described above, and
- [CORE] is a chemical moiety made of at least one (e.g. at least 2, 3, 4, 5, or 6) 3,5-di(hydroxymethyl) phenol moiety.

In a particular aspect, it is disclosed a molecule of formula (IIa):

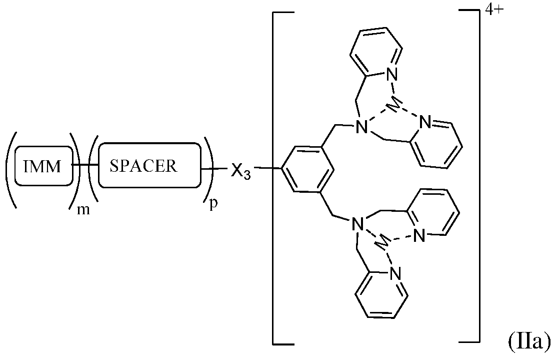

In a particular embodiment, the molecule of the invention is selected from the group consisting of (IIb) and (IIc): 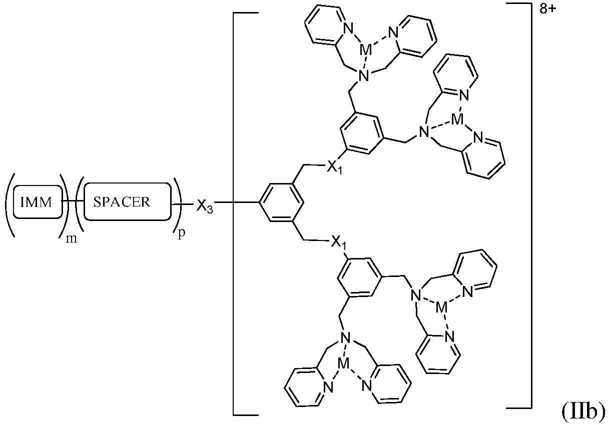
- and 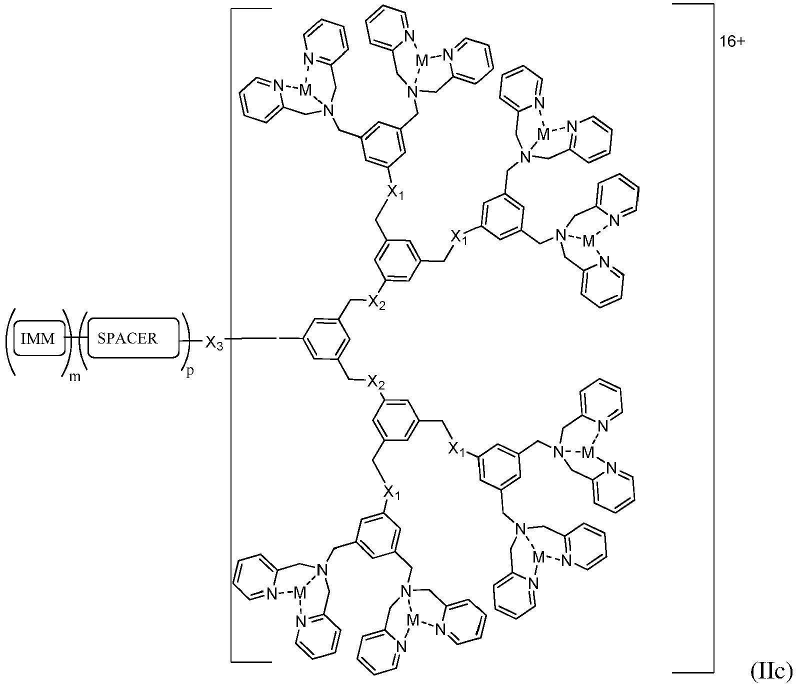 wherein
   - M, m, p, [IMM] and [SPACER] are as defined above for formula (II),
   - each X₁ and X₂, when present, are independently selected from the group consisting of O, NH, or S. Preferably, all X₁ and X₂ are identical, e.g. O,
   - X₃ is selected from -O-, C(=O) -OC(O)-, -C(O)O-, -OC(O)O-, -S-, -SS-, -SC(O)-, - OC(S)-, NR₁-, -NR₁C(O)-, -C(O)NR₁-, NR₁C(S)-, C(S)NR₁-, -OC(O)S-, -OC(S)O-, - SC(O)O-, -OC(S)S-, -SC(O)S-, -SC(S)O-, -SC(S)S-, OC(O)NR₁-, -OC(S)NR₁-, - NR₁C(S)O-, -NR₁C(O)S-, NR₁C(O)NR₂-, -NR₁C(S)NR₂-, -SC(O)S-, -SC(S)O-, -S(O)-, -S(O)₂-, - -P(O)(R₁)-, -P(O)(OR1)-, P(O)(R₁)O-, OP(O)(OR₁)-, OP(O)(R₁)O-, NR₁P(O)(R₂)-, -NR₁P(O)(OR₂)-, NR₁P(O)(R₂)O-, OP(O)(OR₁₎₋ and OP(O)(R₁)O-wherein R₁ and R₂ are independently H or CH₃, preferably H. Preferably, X₃ is O, NH, NHCO, CONH, O(C=O), (O=C)O, O(C=O)O, or NHCONH

In some embodiments, the molecule of the invention is a molecule of formula (IIb) or (IIc), wherein:
- m and p are 1,
- M is Fe^{2+,} Cu²⁺ or Zn^{2+,} preferably Zn^{2+,}
- X₁ and X₂ are O,
- X₃ is O, NH, NHCO, CONH, O(C=O), (O=C)O, O(C=O)O, or NHCONH,
- -[SPACER] is selected from the group consisting of unsaturated or saturated C₂-C₂₀ hydrocarbon chains, optionally substituted, polyamides, polyesters, polyethers such as polyethylene glycol (PEG) or polypropylene glycol, polyvinyl alcohol, polyacrylate, polymethacrylate, polysilicone, unsaturated or saturated, branched or unbranched, hydrocarbon chains and combinations thereof, and
- [IMM] comprises, or consists of, a moiety selected from an amino group, preferably -NH₂, -SH, -OH, an activated carboxylic acid, preferably N-hydroxysuccinimidyl ester, -SH, an azido, and a strained alkyne.

In some embodiments, [SPACER] is selected from polyethylene glycol (PEG) or polypropylene glycol comprising from 2 to 20 monomers, preferably from 3 to 10 monomers.

In preferred embodiments, [SPACER] is selected from C₂-C₂₀ saturated or unsaturated hydrocarbon chains optionally having an heteroatom such as O, NH or S on at least one of its extremities, polyether chains comprising from 2 to 20 monomers and combinations thereof. In an additional embodiment, the molecule of the invention is a molecule of formula (IIa), (IIb) or (IIc), wherein:
- m and p are 1,
- M is Fe^{2+,} Cu²⁺ or Zn^{2+,} preferably Zn^{2+,},
- X₁ and X₂ are O,
- X₃ is O, NH, NHCO, CONH, O(C=O), (O=C)O, O(C=O)O, or NHCONH, and
- [IMM]-[SPACER] is NH₂-(CH₂)ᵣ- with r an integer from 2 to 10. Alternatively, [IMM]-[SPACER] may be also or NH₂-[(CH₂)₂-O]ᵣ-.

The counter-anions present in the molecules of formula (IIa), (IIb) and (IIc) are preferably selected from perchlorate, nitrate, sulphate, halide and carbonate.

In a particular aspect, it is disclosed the molecule of formula (IIIa) : 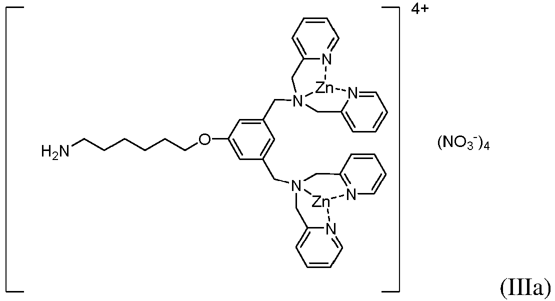 - also called herein Cplx2

In a particular embodiment, the molecule of the invention is selected from (IIIb) and (IIIc): 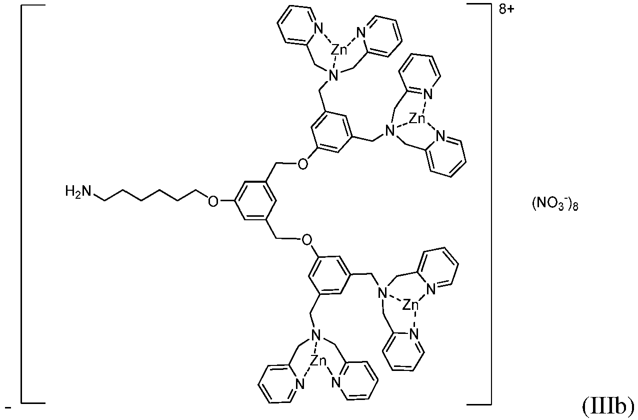 - also called herein Cplx4 And 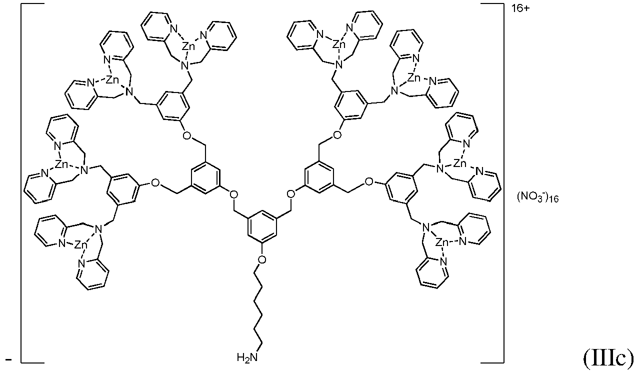 - also called herein Cplx8.

Preferably, the molecule of the invention is of formula (IIIb).

It goes without saying that the counteranions shown in formula (IIIa), (IIIb), and (IIIc) can be replaced by any other anions, in particular biological compatible anions such as perchlorate, tosylate, nitrate, sulphate, sulphonate, thiosulfate, halide, hexafluorophosphate, tetraphenylborate, carbonate, and tetrafluoroborate, more particularly from perchlorate, nitrate, sulphate, halide and carbonate anions. In some preferred embodiments, the counter-anion(s) is/are selected from nitrate and perchlorate.

The molecules of the invention can be synthesized by conventional chemical reactions and can be adapted from the synthesis of molecules of formula (IIIa), (IIIb) and (IIIc) as shown in Example 1.

### - Support and devices comprising the ligands of the invention

As mentioned above, the molecule of the invention may be immobilized on a support. Accordingly, the invention also relates to a support having thereon a ligand of the invention as well as devices comprising such a support.

In other words, the invention relates to supports functionalized, e.g. grafted, with a ligand of the invention. Preferred ligands are those shown in formula (II), (IIa), (IIb), (IIc), (IIIa), (IIIb) and (IIIc). In some embodiments, the support of the invention comprises at least one ligand of formula (IIb) or (IIIb).

The ligand of the invention may be covalently or non-covalently bound to the support.

The support may be of any type with proviso that the ligand can be covalently or non-covalently immobilized on it. The support may be pre-functionalized, e.g. chemically pre-treated or functionalized with a specific binding entity to allow the immobilization of the ligand of the invention.

The constituent of the solid support may be of any type and encompasses glass, metals for instance steel, gold, silver, aluminum, or copper, ceramic, hydroxyapatite, silica, bentonite, polysaccharide such as cellulose, carboxy methylcellulose, hydroxypropyl methylcellulose, diethylaminocellulose (DEAE), dextran, cross-linked dextran agarose, cross-linked agarose, starches, alginate, chitosan and derivatives thereof, plastics and polymers such as polyethylene, polypropylene, polyamide, polyvinylidene fluoride, polyacrylamide, polyesters such as polyethylene terephthalate (PET), polyglycolic acid (PLA), polycaprolactone (PCL), polyethylene adipate (PEA), polybuthylene terephthalate (PBT), or Poly(3-hydroxybutyrate-co-3-hydroxyvalerate), commonly known as PHBV, polymers and copolymers based on acrylic acid and derivatives thereof, polyamide, polystyrene (PS), organopolysiloxane, polyacrylate, polyvinyl polyacrilin, derivatives or combinations thereof. Such supports are commercially available.

In some embodiments, the support is a polymeric support, preferably a support made of polyvinyl chloride (PVC), polystyrene (PS) or polyethylene terephthalate (PET).

For illustration only, when the ligand of the invention comprises a primary amine group as mean for immobilization, the support can be, for instance, a polystyrene support pre-functionalized with N-oxysuccinimide (NOS) or a pre-functionalized PET. The PET can be pre-functionalized by a treatment comprising the hydrolysis of the ester groups present at the surface of the support, the increase of COOH surface density by oxidation (e.g. with potassium permanganate) and activation of COOH groups e.g. with N-hydroxysuccinimide as shown in Example 3.

The device comprising such a support may be of any type and any form. For instance, the device may be a film, a strip, a sheet, a chip, a microchip, a sensor, a reactor, for instance a microreactor a microfluidic chamber or channel, a cartridge, a plate or a microplate, a chromatography column resin or gel, a support for lateral flow immunochromatography assay, a support for ELISA-type assay, a filtration membrane, a filter, a bead such as a polymeric bead or a magnetic bead

### - Methods and uses according to the invention

Typically, the molecule, the support and the device of the invention can be used to capture, immobilize, detect, quantify and/or analyze microvesicles present in a sample. More generally, the Invention relates to the use of the molecule, support and device of the invention for capturing microvesicles present in a sample for the *in vitro* diagnosis of the pathological status of a subject.

In a particular aspect, the Invention relates to a method for capturing microvesicles from a sample, said method comprising:
- providing a support or a molecule of the invention,
- contacting said support or said molecule with a sample susceptible to contain the microvesicles of interest, in conditions suitable for the formation of a complex between the molecules of the invention and the microvesicles, whereby the microvesicles are captured.

The sample may be any composition which potentially comprises the microvesicles of interest. Typically, the sample may be, or may derive from, a body fluid. The body fluid is typically retrieved from a human being, preferably a human being suffering from, or at risk of developing a disease as described further below.

As used herein, the term *"biological fluid"* refers to any extractable or retrievable body fluid, including for example, blood, blood plasma, cerebrospinal fluid, bronchoalveolar fluid, urine, synovial fluid, breast milk, saliva, tears, seminal fluid, ascitic fluids, amniotic fluid and effusions (pleural or other). In the context of the invention, preferred biological fluids are blood, blood plasma and urine.

As used herein, "a *sample derives from a body fluid"* means that the sample is obtained from said body fluid by subjecting the body fluid to one or several treatment steps, e.g. in order to remove contaminants and/or separate the microvesicles from certain constituents of the body fluid. For instance, the body fluid may be subjected to one or several treatments such as a precipitation step e.g. salt precipitation, cryo-precipitation or flocculation, a filtration step such as depth filtration or ultrafiltration, centrifugation, clarification, chromatography, an extraction step such as a liquid-liquid or a solid-liquid extraction, viral inactivation, pasteurization, concentration, dialysis, freezing/thawing steps and the like.

The sample typically comprises contaminants from which microvesicles of interest is to be separated. The contaminants may be of any type and depend on the nature of the starting composition. The contaminants encompass proteins, salts, hormones, vitamins, nutriments, lipids, cells, cell debris such as cell membrane fragments and the like.

In some embodiments, the methods of the invention are performed on a non-treated body fluid, preferably on non-treated urine or plasma blood.

In some embodiments, the method may further comprise one or several steps selected from:
- a step of providing a sample susceptible to contain the microvesicles of interest from a body fluid of a subject, and/or
- a step of washing the support after contacting the support with the sample, e.g. so as to remove contaminants, and/or
- a step of detecting the formation of the complex between the microvesicles of interest and said support or molecule of the invention and/or
- a step of recovering the complex formed between the microvesicles and the molecules of the invention, and/or
- a step of quantification of said microvesicles of interest, and/or
- a step of detection of said microvesicles, and/or
- a step of characterization of the captured microvesicles, e.g. by detecting and/or quantifying a biomarker present on the surface or within the microvesicles, and/or
- a step of releasing the microvesicles from the complex, and/or
- a step of recovering the microvesicles of interest.

As mentioned above, the molecules, supports and devices of the invention find applications in the *in vitro* diagnosis.

Indeed, the detection, the quantification and/or the characterization of microvesicles in a sample may be useful in the context of the diagnosis and prognostic of diseases in a patient. The level of microvesicles in the body fluid as well as their compositions in terms of lipids, proteins and genetic materials such as mRNA and mitochondrial RNA is indicative of the pathological status of the subject.

Besides, the determination of microvesicle proteome may enable to determine the physiological and/or pathological status of the cell from which they originate, and can therefore represent a tool of choice for the early detection of a pathological state.

Accordingly, the detection, quantification and/or characterization of the captured microvesicles may allow the diagnosis of a pathology, the evaluation of the risk of developing a pathology, the prognosis of a pathology, the differential diagnosis of a pathology, the follow-up of the evolution of a pathology, and/or the monitoring of the therapy effectiveness in the patient. Thus, in an additional aspect, the invention relates to the use of a molecule, support or device of the invention in an *in vitro* method for the diagnosis, the differential diagnosis, the prognosis, the assessment of the risk of, and/or the monitoring of the evolution of a disorder in a subject. The Invention also relates to the use of a molecule, support or device of the invention in an *in vitro* method for monitoring the efficacy of therapeutic treatment in a patient.

The disease or disorder of interest may be of any type and includes thrombotic, inflammatory and/or metabolic disorders, as well as cardiovascular or neurovascular diseases. For instance, disorders of interest include diabetes and related disorders such as diabetic nephropathy, diabetic neuropathy, diabetic retinopathy, and diabetic foot syndrome, multiple sclerosis, cancer, Alzheimer's disease, Parkinson's disease, aneurysm, cerebral vasospasm, stroke, coronary artery disease, parasitic diseases, cancers and other pathologies.

In a particular embodiment, the invention relates to an *in vitro* method for the diagnosis, the differential diagnosis, the prognosis, the assessment of the risk of, and/or the monitoring a disorder in a subject which comprises:
(a) providing a support or a molecule of the invention,
(b) contacting said support or said molecule with a sample obtained from the subject, in conditions suitable for the formation of a complex between the molecules of the invention and the microvesicles possibly present in the sample,
(c) detecting, quantifying and/or characterizing the microvesicles captured in step (b) by complexation.

As mentioned above, preferred disorders include thrombotic, inflammatory and/or metabolic disorders, as well as cardiovascular or neurovascular diseases. In a preferred embodiment, the disorder is selected from the group consisting of diabetes and related disorders such as diabetic nephropathy, diabetic neuropathy, diabetic retinopathy, and diabetic foot syndrome, multiple sclerosis, cancer, Alzheimer's disease, Parkinson's disease, aneurysm, cerebral vasospasm, stroke, coronary artery disease, parasitic diseases, cancers and other pathologies.

Said method may further comprise a step of comparing the result obtained in step (c) with that obtained from one or several control samples in similar conditions.

The control sample(s) may be obtained from a healthy subject or from a subject suffering from the disorder to diagnose. Alternatively, the sample is from the same subject, but obtained earlier. Comparison with earlier samples from the same subject may enable to monitor the evolution of the disease and/or the efficacy of the treatment and/or to predict the outcome of the disease or treatment in the subject.

The detection, quantification and/or characterization of the microvesicles can be carried by standard methods known by the skilled artisan.

As used herein *"the characterization"* of microvesicles refer to the detection, the determination of the presence or the absence and/or the quantification of at least one biomarker susceptible to be present in the microvesicles and correlated with the disorder to diagnose.

The biomarker may be any molecule of interest such as proteins, lipids, hormones, mitochondrial DNA, miRNA or mRNA. Such biomarkers can be detected and/or quantified by methods well-known by the skilled artisan.

For instance, proteins can be detected and quantified by standard immunological assays, e.g. by ELISA or Western blot techniques. Detection and characterization of nucleic acids may include specific nucleic acid detection methods such as PCR /qPCR, RT-PCR / RT-qPCR and sequencing.

In some embodiments, the method comprises a step of normalization of the result obtained in step (c). For instance, said normalization can be carried out on the basis of the quantification of a marker present on or in the microvesicles such as annexin-A5 and beta-actin.

Thus, in certain embodiments, step (c) of the method may comprise:
- a step of quantifying a biomarker of interest in the captured microvesicles,
- a step of quantifying a normalization biomarker in the captured microvesicles,
- a step of normalizing the amount detected for the biomarker by the amount obtained for the normalization biomarker (e.g. by calculating the ratio of the amount of biomarker to that of the normalization biomarker), and
- optionally comparing the resulting normalized amount of the biomarker of interest with that obtained for a control sample in similar conditions.

The comparison with a normalized amount obtained for a control sample, e.g. a sample obtained from a healthy subject, or a sample obtained from a subject suffering from the disorder to diagnose, may enable to determine the diagnosis or prognostic of the disease in the subject.

In the method of the invention, it goes without saying that the biomarker(s) to detect or quantify the microvesicles is selected depending on the disorder of interest.

For illustration only:
- For the diagnosis, in particular of early diagnosis, of nephropathy in a subject, the biomarker may be podocalyxin.
- For the diagnosis, in particular of early diagnosis, of Parkinson's disease, the biomarker may be alpha-synuclein.

### - kits

The disclosure also relates to a kit comprising a molecule, a support or a device according to the disclosure. Such a kit is typically dedicated for implementing, at least in part, a method of the invention, e.g. the method for capturing microvesicles from a sample, and/or the *in vitro* method for the diagnosis, the differential diagnosis, the prognosis, the assessment of the risk of, and/or the monitoring a disorder in a subject.

Typically, the kit comprises a molecule of the disclosure, for instance as shown in any one of formula II, (IIa), (IIb), (IIc), (IIIa), (IIIb) or (IIIc) or a support or device having thereon said molecules as described above and at least one additional mean useful to implement a method of the invention.

For instance, the at least one additional mean may be selected from:
- means for providing the sample to analyze, for instance mean for pre-treating the body fluid obtained from the subject,
- buffers for implementing the methods of the invention, e.g. suspension buffers, washing buffers, elution buffers (e.g. for the dissociation of the complex form between microvesicles and the molecules of the invention)
- a mean for the detection of the captured microvesicles,
- a mean for the characterization of the captured microvesicles, for instance a mean for detecting, and/or quantifying at least one biomarker possibly present in the microvesicles
- a mean for detecting and/or quantifying at least one normalization biomarker in the captured microvesicles such as annexin V or beta-actin
- a control sample for the normalization of the biomarker quantified in the microvesicles
- a control sample, such as a positive or negative control sample, for validation

As used herein, "a *mean for the detection or quantification of a biomarker"* refers to any mean known by the skilled artisan for detecting or quantifying said biomarker. Said mean depends on the nature of the biomarker. For instance, a protein biomarker may be detected/quantified by immunological techniques (in particular ELISA and Western-blot) while nucleic acid biomarkers can be detected by specific amplification techniques, which can be qualitative or quantitative.

Moreover, the kit according to the disclosure may comprise a notice providing its user with instructions for implementing the method according to the invention by means of the kit.

Further aspects and advantages of the present invention are disclosed in the following experimental section, which should be regarded as illustrative and not limiting the scope of the present patent.

### Examples

### Example 1: Synthesis of ligands according to the invention

### A. Synthesis of Tert-butyl (6-iodohexyl)carbamate (intermediate)

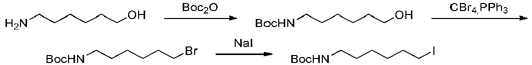

Tert-butyl (6-hydroxyhexyl)carbamate: 3.72 g (17 mmol) of Di-tert-butyl-dicarbonate was added into a solution of 2 g (17mmol) 6-amino-1-hexanol in 40 mL tetrahydrofuran at 0 °C under stirring. The reacting mixture was allowed to slowly warm up to room temperature. After 24 h, the solvent was evaporated. The residue was dissolved in dichloromethane 50 mL and was washed with 50 mL of water for 3 times. The organic phase was gathered and dried over sodium sulfate, and then the solvent was removed with a rotary evaporator. The product is colorless oil, and the yield was 91 %. ¹H NMR (CDCl₃, 300 MHz, 25°C): δ(ppm) 3.60 (t, 2H), 3.08 (t, 2H), 1.59-1.28 (m, 17H).

Tert-butyl (6-bromohexyl)carbamate: To a solution of 1 g (4.6 mmol) tert-butyl (6-hydroxyhexyl)carbamate and 2.1 g (8.1 mmol) of triphenylphosphine in 12 mL tetrahydrofuran, a solution of 2.7 g (8.1 mmol) of tetrabromomethane in 8 mL tetrahydrofuran was added dropwise at 0 °C under stirring. The reacting mixture was allowed to slowly warm up to room temperature. After 24 h of reaction, the solvent was removed. The residue was added into a solvent mixture of 50 mL petroleum ether and 10 mL ethyl acetate. A precipitate formed instantly and then was filtered off while the filtrate was collected and evaporated to leave colorless oil behind. The oil went through a flash column of silica using an eluent of petroleum ether: ethyl acetate= 10:1. The solvent was removed with a rotary evaporator. The product is colorless oil, and the yield was 93 %. ¹H NMR (CDCl₃, 300 MHz, 25°C): δ(ppm) 3.44 (t, 2H), 3.14 (t, 2H), 1.91-1.86 (m, 2H), 1.52-1.33 (m, 15H).

Tert-butyl (6-iodohexyl)carbamate: 1.5 g (5.4 mmol) of tert-butyl (6-bromohexyl)carbamate was dissolved in 15 mL acetonitrile. 4.02 g (26.8 mmol) of sodium iodide was added into the solution. The mixture was stirred under room temperature and kept in darkness for 48 h. The solvent was removed under vacuum, and the residue was extracted with 50 mL of dichloromethane and 50 mL water. The organic phase was washed with a saturated solution of sodium thiosulfate and then dried over sodium sulfate. The solvent was removed with rotary evaporator. The product is colorless oil, and the yield was 96 %. ¹H NMR (CDCl₃, 300 MHz, 25°C): δ(ppm) 3.21 (t, 2H), 1.90-1.80(m, 2H), 1.56-1.30 (m, 15H).

### B. Synthesis of complex of formula (IIId) (comparative - Cplx1)

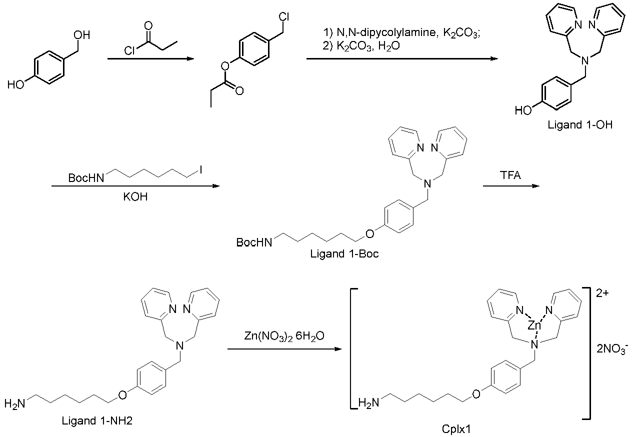

4-(chloromethyl)phenyl propionate: Under stirring, 30 mL propionyl chloride was cooled to 0°C. 5.1 g (41 mmol) of 4-(hydroxymethyl)phenol was added slowly into the flask so as to control the generation of hydrogen chloride at a moderate rate. After the addition, the mixture was allowed to slowly warm up to room temperature. After 12h, the mixture was poured into 500 mL ice-cold water, and then neutralized with sodium bicarbonate. The mixture was then extracted with 50 mL diethyl ether for 3 times. The organic phase was combined and washed with 30 mL water for 3 times. The solution was then dried over sodium sulfate, and the solvent was removed using a rotary evaporator. The product is pale yellow oil, and the yield was 97%. ¹H NMR (CDCl₃, 300 MHz, 25°C): δ(ppm) 7.42 (d, 2H), 7.12 (d, 2H), 4.60 (s, 2H), 2.62 (q, 2H), 1.30 (t, 3H).

Ligand 1-OH: 199 mg (1 mmol) of 4-(chloromethyl)phenyl propionate and 199 mg (1 mmol) of di-(2-picolyl)amine was dissolved in 10 mL dimethyl sulfoxide under stirring. After adding 414.6 mg (3 mmol) of potassium carbonate, the mixture was then heated to 60 °C. 24 h later, the reaction was cooled to room temperature, and then a solution of 0.6 g potassium carbonate in 3 mL water was added at room temperature. Another 24 h later, the reaction mixture was dissolved in 30 mL dichloromethane, then washed with water (30 mL, 3 times), dried over sodium sulfate. The solvent was removed under vacuum to give the product as white solid. The yield was 74%. ¹H NMR (DMSO, 300 MHz, 25°C): δ(ppm) 9.31 (s, 1H), 8.51-8.49 (m, 2H), 7.83-7.77 (m, 2H), 7.58 (d, 2H), 7.28-7.24 (m, 2H), 7.20 (d, 2H), 6.73 (d, 2H), 3.68 (s, 4H), 3.51 (s, 2H); ¹³C NMR (DMSO, 75MHz, 25°C): δ(ppm) 159.80, 156.87, 149.26, 137.08, 130.38, 128.98, 122.87, 122.59, 115.48, 59.33, 57.35.

Ligand 1-Boc: 305 mg (1 mmol) of ligand 1-OH was dissolved in 4 mL dimethylformamide under stirring. 112 mg (2 mmol) of potassium hydroxide was added to the solution at room temperature. 1 h later, the mixture was cooled to -20 °C, then 392 mg (1.2 mmol) of tert-butyl (6-iodohexyl)carbamate was added into the mixture. The reaction was allowed to slowly warm up to room temperature. 3 h later, the reaction was quenched with water. The mixture was extracted three times with 20 mL dichloromethane, then the combined organic phase was washed two times with 20 mL brine, and dried over sodium sulfate. The solvent was removed under vacuum to afford a brown oil. The crude product was then purified by silica gel column chromatography with an eluent of ethyl acetate: acetonitrile (5:1). The product is obtained as a pale yellow solid in 75% yield. ¹H NMR (MeOD, 300 MHz, 25°C): δ(ppm) 8.43 (m, 2H), 7.86-7.71 (m, 2H), 7.70 (d, 2H), 7.32-7.28 (m, 4H), 6.89 (d, 2H), 3.99 (t, 2H), 3.77 (s, 4H), 3.61 (s, 2H), 3.06 (t, 2H), 1.81-1.76 (m, 2H), 1.54-1.44 (m, 15H); ¹³C NMR (MeOD, 75MHz, 25°C): δ(ppm) 159.34, 154.18, 147.94, 137.34, 129.86, 123.88, 123.32, 122.39, 113.99, 67.45, 66.43, 59.12, 57.81, 28.94, 27.39, 25.48.

Ligand 1-NH₂: 802.2 mg (1.6 mmol) of ligand 1-Boc was dissolved in 15 mL tetrahydrofuran. Trifluoroacetic acid was then added into the solution slowly at room temperature. 5 min after the addition of trifluoroacetic acid, the solution was heated to 60 °C. 30 h later, the solution was cooled to room temperature and then poured into an ice-cold sodium bicarbonate solution. The product was extracted with dichloromethane (30 mL, under vacuum and the compound was obtained with 85% yield. ¹H NMR (MeOD, 300 MHz, 25°C): δ(ppm) 8.54 (d, 2H), 7.71-7.66 (m, 2H), 7.60 (d, 2H), 7.19-7.14 (m, 2H), 6.87 (d, 2H), 3.96 (t, 2H), 3.82 (s, 4H), 3.64 (s, 2H), 2.73 (t, 2H), 1.83-1.77 (m, 2H), 1.53-1.41 (m, 6H); ¹³C NMR (MeOD, 75MHz, 25°C): δ(ppm) 159.94, 158.25, 148.96, 136.44, 130.69, 130.06, 122.81, 121.94, 114.31, 67.86, 59.84, 57.88, 41.88, 33.24, 29.27, 26.66, 25.94;

### Complex of formula (IIId) - comparative - also referred as Cplx1 herein)

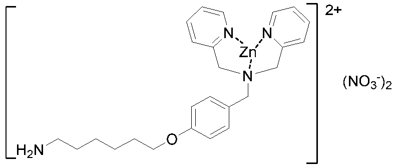

108 mg (0.27 mmol) of ligand 1-NH₂ was dissolved in 10 mL methanol at room temperature under stirring. 79.4 mg (0.27 mmol) of zinc nitrate hexahydrate was dissolved in 5 mL methanol and was added in to the solution of ligand dropwise. 12 h later, the solvent was removed under vacuum to give quantitatively the final product as pale yellow powder (yield 100%). ¹H NMR (DMSO, 300 MHz, 25°C): δ(ppm) 8.69 (d, 2H), 8.12 (t, 2H), 7.66 (m, 4H), 7.30 (d, 2H), 7.02 (d, 2H), 4.25 (d, 2H), 4.02 (t, 2H), 3.71 (t, 4H), 2.80 (t, 2H), 1.78-1.73 (m, 2H), 1.60-1.56 (m, 2H), 1.48-1.39 (m, 6H); ¹³C NMR (MeOD, 75MHz, 25°C): δ(ppm) 159.34, 154.81, 148.40, 141.26, 133.32, 125.33, 125.19, 123.98, 114.90, 67.88, 56.40, 55.68, 29.22, 29.01, 28.09, 26.10;

### C. Synthesis of complex of formula (IIIa) (hereafter Cplx2)

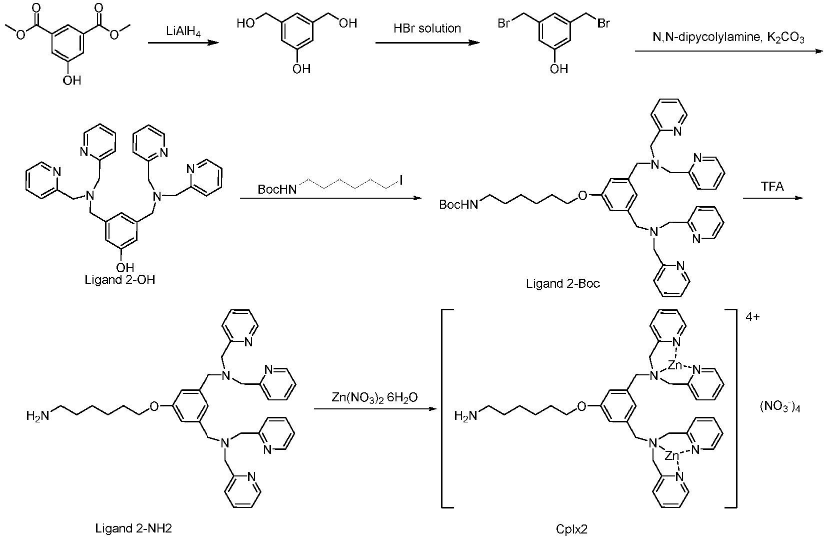

5-hydroxyphenyl-1,3-dimethanol: To a dispersion of 1.3 g (34.3 mmol) of lithium aluminum hydride in 40 mL anhydrous tetrahydrofuran at 0 °C, a solution of 3 g (14.3 mmol) of dimethyl 5-hydroxyisophthalate in 60 mL anhydrous tetrahydrofuran was added dropwise. The reaction was allowed to warm up to room temperature during 3 hours. After 12 h, the mixture was cooled to 0 °C again and quenched with 10% hydrochloric acid. The solvent was removed under reduced pressure. The residue was diluted in 50 mL brine and ethyl acetate was used to extract the product from the aqueous solution (6x50 mL). The organic layers were combined, dried over sodium sulfate and the solvent was removed under vacuum. The product is obtained as a white solid in 94% yield. ¹H NMR (CDCl₃, 300 MHz, 25°C): δ(ppm) 6.69 (s, 1H), 6.61 (s, 2H), 4.41 (s, 4H).3,5-bis(bromomethyl)phenol

3,5-bis(bromomethyl)phenol: 8.2 mL of 33% hydrogen bromide solution was added dropwise at 0 °C to a solution of 2 g (13 mmol) of 5-hydroxyphenyl)-1,3-dimethanol in 20 mL acetic acid. The mixture was extracted with 3 x70 mL dichloromethane. The combined organic phases were washed with 2x100 mL water, then 2x100 mL saturated sodium bicarbonate solution and again 100 mL water. The organic phase was dried with sodium sulfate, filtered and concentrated. The crude product was purified with a flash column silica chrommtography, with a mixture of petroleum ether: ethyl acetate (9:1) to afford white solids (yield 90%).. ¹H NMR (CDCl₃, 300 MHz, 25°C): δ(ppm) 3.21 (t, 2H), 1.90-1.80(m, 2H), 1.56-1.30 (m, 15H).

Ligand 2-OH: 672 mg (2.4 mmol) of 3,5-bis(bromomethyl)phenol, 1196 mg (6 mmol) of N,N-dipycolylamine and 398 mg (2.88 mmol) of potassium carbonate was added into 10 mL dimethylformamide under nitrogen protection. The mixture was stirred under room temperature. After 3 hours, 50 mL water was added into the mixture, and was extracted with 2x50 mL of dichloromethane. The organic layers were combined and washed with 2x50 mL of water, dried over sodium sulfate, filtered and evaporated using rotary evaporator. The remainder was purified using silica gel column chromatography to give the final product as white solid (yield 80%).¹H NMR (CDCl₃, 300 MHz, 25°C): δ(ppm) 8.48-8.46 (m, 4H), 7.60-7.58 (m, 8H), 7.11 (q, 4H), 6.96 (s, 1H), 6.86 (s, 2H), 3.79 (s, 8H), 3.57 (s, 4H);

Ligand 2-Boc : 520 mg (1 mmol) of ligand 2-OH was dissolved in 4 mL dimethylformamide under stirring. 120 mg (2 mmol) of potassium hydroxide was added to the solution at room temperature. 1 h later, the mixture was cooled to -20 °C, then 400 mg (1.2 mmol) of tert-butyl (6-iodohexyl)carbamate was added into the mixture. The reaction was allowed to slowly warmup to room temperature. 3 h later, the reaction was quenched with water. The mixture was extracted with 20 mL dichloromethane for three times, then the combined organic phase was washed with 20 mL brine twice, and dried with sodium sulfate. The solvent was removed under vacuum to afford brown oil. The crude product was then purified by silica gel column chromatography with an eluent of ethyl acetate: acetonitrile (5:1). The product is pale yellow solid with. 75% yield. ¹H NMR (DMSO, 300 MHz, 25°C): δ(ppm) 8.43 (d, 4H), 7.79 (ddd, 4H), 7.28 (tt, 4H), 7.04 (s, 1H), 3.97 (t, 2H), 3.80 (s, 8H), 3.65 (s, 4H), 3.07 (t, 2H), 1.79 (t, 2H), 1.58-1.48 (m, 15H);

Ligand 2-NH₂ : 1350 mg (1.89 mmol) of ligand 2-Boc was dissolved in 15 mL tetrahydrofuran. 7.2 mL (94.3 mmol) of trifluoroacetic acid was then added into the solution slowly at room temperature. 5 min after the addition of trifluoroacetic acid, the solution was heated to 60 °C. 30 h later, the solution was cooled to room temperature and then poured into an ice-cold sodium bicarbonate solution. The product was extracted with dichloromethane (3x30 mL). The organic phase was then dried over sodium sulfate and the solvent was removed using rotary evaporator. The yield was 85%. ¹H NMR (DMSO, 300 MHz, 25°C): δ(ppm) 8.49 (d, 4H), 7.74 (ddd, 4H), 7.57 (d, 4H), 7.25 (dd, 4H), 7.07 (s, 1H), 6.82 (s, 2H), 3.93 (t, 2H), 3.71 (s, 8H), 3.60 (s, 4H), 3.32 (b, 4H), 1.69 (t, 2H), 1.42-1.34 (m, 6H); ¹³C NMR (MeOD, 75MHz, 25°C): δ(ppm) 159.79, 159.20, 148.97, 140.49, 136.42, 122.76, 121.95, 121.48, 113.56, 67.85, 60.09, 58.63, 41.31, 29.07, 26.81, 26.58, 26.13.

### Complex of formula (IIIa) - (hereunder Cplx2)

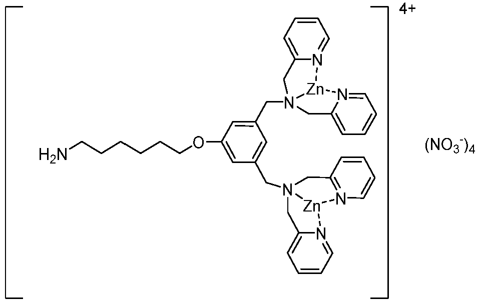

1110 mg (1.8 mmol) of ligand 2-NH₂ was dissolved in 10 mL methanol at room temperature under stirring. 1072 mg (3.6 mmol) of zinc nitrate hexahydrate was dissolved in 5 mL methanol and was added in to the solution of ligand dropwise. 12 h later, the solvent in the mixture was removed under vacuum to give the final product as pale yellow powder (yield 100%). ¹H NMR (DMSO, 300 MHz, 25°C): δ(ppm) 8.71 (b, 4H), 8.11 (t, 4H), 7.67 (t, 4H), 7.60 (d, 4H), 7.01 (b, 2H), 6.89 (s, 1H), 4.35 (d, 2H), 4.07 (b, 2H), 3.81 (d, 8H), 2.84 (t, 2H), 1.79 (b, 2H), 1.65-1.46 (m, 6H); ¹³C NMR (MeOD, 75MHz, 25°C): δ(ppm) 162.85, 159.27, 155.04, 148.50, 141.37, 134.32, 127.12, 125.32, 117.96, 68.03, 57.51, 56.15, 39.86, 36.26, 31.24, 29.05, 27.79, 26.11, 25.62.

### D. Synthesis of 2-(trimethylsilyl)ethyl (6-iodohexyl)carbamate (intermediate)

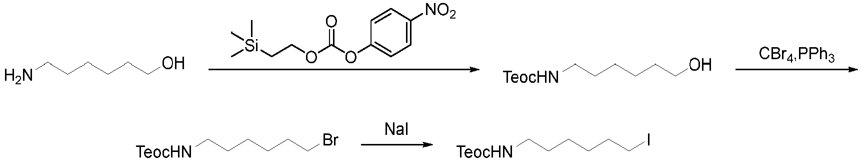

2-(trimethylsilyl)ethyl (6-hydroxyhexyl)carbamate : 352 mg (3 mmol) of 6-amino-1-hexanol was dissolved in 5 mL dichloromethane. 0.9 mL (6 mmol) triethylamine was added into the solution and then 850 mg (3 mmol) of 4-Nitrophenyl 2-(trimethylsilyl)ethyl carbonate in 1.4 mL dichloromethane was also added into the mixture at room temperature under stirring. After 24 h, the solvent was evaporated. The residue was dissolved in dichloromethane 50 mL and washed with 3x50 mL of saturated NaHCO₃ solution and then 3x50 mL of NAOH (2M) solution. The organic phase was gathered and dried over sodium sulfate, and the solvent was removed with a rotary evaporator. The product is obtained as a colorless oil in98 % yield. ¹H NMR (CDCl₃, 300 MHz, 25°C): δ(ppm) 4.64 (b, 1H), 4.17 (t, 2H), 3.67 (t, 2H), 3.20 (q, 2H), 1.68-1.34 (m, 8H), 1.00 (t, 2H), 0.07 (s, 9H);

2-(trimethylsilyl)ethyl (6-bromohexyl)carbamate : To a solution of 748 mg (2.86 mmol) of 2-(trimethylsilyl)ethyl (6-hydroxyhexyl)carbamate and 1311 mg (5 mmol) of triphenylphosphine in 12 mL tetrahydrofuran, a solution of 1.66 g (5 mmol) of tetrabromomethane in 8 mL tetrahydrofuran was added dropwise at 0 °C under stirring. The reacting mixture was allowed to slowly warm up to room temperature. After 24 h of reaction, the solvent was removed. The residue was added into a solvent mixture of 50 mL petroleum ether and 10 mL ethyl acetate. A Precipitate formed instantly and filtered off. The filtrate was collected and evaporated to give a colorless oil. The oil went through a flash chromatography column of silica gel using an eluent of petroleum ether: ethyl acetate (10:1). The solvent was removed under vacuum and a colorless oil in obtained in , 84 % yield. ¹H NMR (CDCl₃, 300 MHz, 25°C): δ(ppm) 4.61 (b, 1H), 4.18 (t, 2H), 3.43 (t, 2H), 3.19 (q, 2H), 1.94-1.84 (m, 2H), 1.62-1.34 (m, 6H), 1.00 (t, 2H), 0.07 (s, 9H);

2-(trimethylsilyl)ethyl (6-iodohexyl)carbamate : 846 mg (2.61 mmol) of 2-(trimethylsilyl)ethyl (6-bromohexyl)carbamate was dissolved in 15 mL acetonitrile. 2 g (13 mmol) of sodium iodide was added into the solution. The mixture was stirred under room temperature and kept in darkness for 48 h. The solvent was removed under vacuum, and the residue was extracted with 50 mL of dichloromethane and 50 mL water. The organic phase was washed with a saturated solution of sodium thiosulfate and then dried over sodium sulfate. The solvent was removed under vacuum and the product was obtained as a colorless oil in 96 % yield. ¹H NMR (CDCl₃, 300 MHz, 25°C): δ(ppm) 4.61 (b, 1H), 4.18 (t, 2H), 3.24-3.16 (m, 4H), 1.89-1.80 (m, 2H), 1.58-1.33 (m, 6H), 1.00 (t, 2H), 0.07 (s, 9H);

### E. Synthesis of complex of formula (IIIb) (hereafter Cplx4)

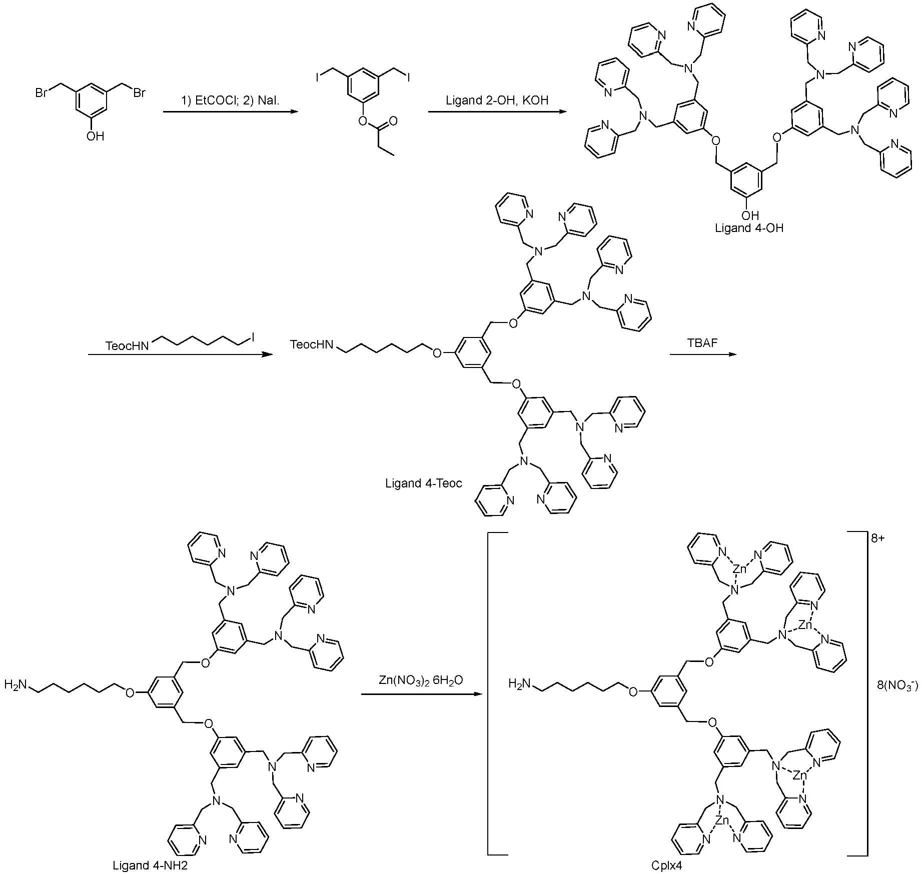

3,5-bis(iodomethyl)phenylpropionate : 1.15 mL (13 mmol) of propionyl chloride was dissolved in 30 mL of dichloromethane. At 0°C, 2.8 g (10 mmol) of 3,5-bis(bromomethyl)phenol dissolved in 10 mL dichloromethane was added into the solution under stirring. 5 min later, 3 mL (13 mmol) of triethylamine in 10 mL dichloromethane was also added into the solution and the cold bath was removed. 2h after the addition of triethylamine, the reaction was quenched with 10 mL water. The mixture was extracted with 50 mL dichloromethane twice, and the organic phase was combined and washed first with 15 mL saturated sodium bicarbonate solution, then with 15 mL brine. The organic phase was dried with sodium sulfate before the solvent was removed under vacuum. The product was dissolved in 15 mL acetonitrile. 7.5 g (50 mmol) sodium iodide was added into the solution. The mixture was stirred under room temperature and kept in darkness for 48 h. The solvent was removed under vacuum, and the residue was extracted between 50 mL of dichloromethane and 50 mL water. The organic phase was washed with a saturated solution of sodium thiosulfate before the organic phase was collected and dried over sodium sulfate. The solvent was removed with a rotary evaporator. The product is a colorless solid (96 %). ¹H NMR (CDCl₃, 300 MHz, 25°C): δ(ppm) 7.27 (t, 1H), 7.04 (d, 2H), 4.41 (s, 4H), 2.62 (q, 2H), 1.30 (t, 3H);

Ligand 4-OH : 1284 mg (2.5 mmol) of ligand 2-OH was dissolved in 4 mL dimethylformamide under stirring. 126 mg (2.5 mmol) of potassium hydroxide was added to the solution at room temperature. 1 h later, the mixture was cooled to -20 °C, then 520 mg (1.2 mmol) of 3,5-bis(iodomethyl)phenyl propionate was added into the mixture. The reaction was allowed to slowly warm up to room temperature. 3 h later, the reaction was quenched with water. The mixture was extracted with 20 mL dichloromethane for three times, then the combined organic phase was washed with 2x20 mL brine, and dried with sodium sulfate. The solvent was removed with rotary evaporator to afford brown oil. The crude product was then purified by silica gel column chromatography with an eluent of ethyl acetate: acetonitrile= (5:1). The product is pale yellow solid. The yield was 75%. ¹H NMR (DMSO, 300 MHz, 25°C): δ(ppm) 8.47 (tt, 8H), 7.71 (ddd, 8H), 7.54-7.51 (m, 8H), 7.20 (qq, 8H), 7.06 (s, 2H), 6.96 (s, 1H), 6.90 (s, 4H), 6.83 (s, 2H), 5.01(s, 4H), 3.70 (s, 16H), 3.57 (s, 8H); ¹³C NMR (CDCl₃, 75MHz, 25°C): δ(ppm) 159.68, 158.63, 157.79, 148.87, 140.61, 139.88, 138.99, 136.64, 122.84, 122.49, 122.05, 117.89, 114.94, 115.18, 69.96, 59.87, 58.33.

Ligand 4-Teoc : 930 mg (0.8 mmol) of ligand 4-OH was dissolved in 10 mL dimethylformamide under stirring. 90.5 mg (1.6 mmol) of potassium hydroxide was added to the solution at room temperature. 1 h later, the mixture was cooled to -20 °C, then 360 mg (0.97 mmol) of 2-(trimethylsilyl)ethyl (6-iodohexyl)carbamate was added into the mixture. The reaction was allowed to slowly warm up to room temperature. 3 h later, the reaction was quenched with water. The mixture was extracted with 3x20 mL dichloromethane, then the combined organic phase was washed with 2x20 mL brine, and dried with sodium sulfate. The solvent was removed with rotary evaporator to afford brown oil. The crude product was then purified by silica gel column chromatography with an eluent of ethyl acetate: acetonitrile (5:1). The product is pale yellow solid. The yield was 75%. ¹H NMR (CDCl₃, 300 MHz, 25°C): δ(ppm) 8.54-8.51 (m, 8H), 7.65-7.55 (m, 16H), 7.16-7.11 (m, 11H), 7.00-6.98 (m, 6H), 5.05 (s, 4H), 4.17 (t, 2H), 3.97 (t, 2H), 3.82 (s, 16H), 3.68(s, 8H), 3.18 (q, 2H), 1.83-1.72 (m, 2H), 1.54-1.34 (m, 6H), 0.99 (t, 2H), 0.06 (s, 9H); ¹³C NMR (CDCl₃, 75MHz, 25°C): δ(ppm) 162.55, 159.75, 159.67, 159.02, 148.99, 140.73, 139.09, 136.42, 122.73, 121.96, 121.88, 118.52, 113.82, 113.11, 69.91, 67.92, 60.10, 58.57, 40.86, 36.49, 30.94, 30.05, 29.19, 26.54, 25.79, 17.80, -1.44;

Ligand 4-NH₂ : 900 mg (0.65 mmol) of ligand 4-Teoc was dissolved in 10 mL tetrahydrofuran under stirring. 6.5 mL (6.5 mmol) of 1 M tetrabutylammonium fluoride solution was then added at room temperature. The reaction mixture was then kept at 60 °C overnight. After cooled to room temperature, the solvent was removed in vacuo. The residue was dissolved in dichloromethane, and then washed with 50 mL 0.5 M NaOH solution once and 50 mL saturated NaHCO₃ solution twice. The organic phase was dried with sodium sulfate. The solvent was removed with rotary evaporator to afford brown solid in 90% yield. ¹H NMR (DMSO, 300 MHz, 25°C): δ(ppm) 8.48-8.45 (t, 8H), 7.70 (ddd, 8H), 7.51 (d, 8H), 7.22 (ddd, 8H), 7.11 (s, 1H), 7.06 (s, 2H), 6.97 (s, 2H), 6.92 (s, 4H), 5.07 (s, 4H), 3.89 (t, 2H), 3.69 (s, 16H), 3.57 (s, 8H), 1.61 (t, 2H), 1.37-1.19 (m, 6H); ¹³C NMR (DMSO, 75MHz, 25°C): δ(ppm) 159.61, 159.29, 158.79, 149.79, 149.26, 140.72, 139.49, 136.95, 122.81, 122.57, 121.69, 114.10, 113.30, 69.48, 59.66, 57.94, 55.39, 41.52, 32.60, 29.05, 26.48, 25.80.

### 1) Complex of formula IIIb - (hereafter Cplx4)

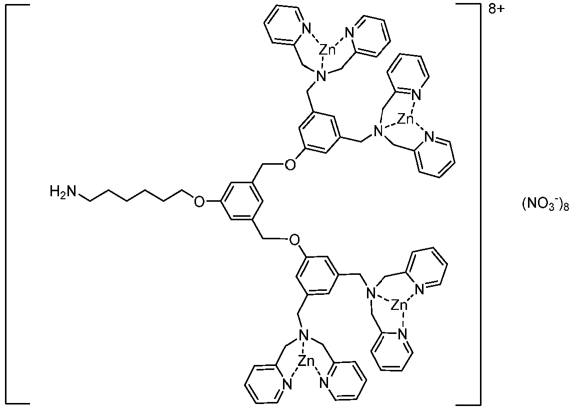

125 mg (0.1 mmol) of ligand 4-NH₂ was dissolved in 10 mL methanol at room temperature under stirring. 119 mg (0.4 mmol) of zinc nitrate hexahydrate was dissolved in 5 mL methanol and was added in to the solution of ligand dropwise. 12 h later, the solvent in the mixture was removed under vacuum to give quantitatively the final product as brown powder (yield 100%). ¹H NMR (DMSO, 300 MHz, 25°C): δ(ppm) 8.70 (d, 8H), 8.10 (t, 8H), 7.67 (t, 8H), 7.58 (d, 8H), 7.27 (s, 1H), 7.17 (s, 4H), 7.06 (s, 1H), 6.96 (s, 2H), 5.27 (s, 4H), 4.38 (d, 8H), 4.10 (q, 2H), 3.83 (t, 16H), 2.81 (t, 2H), 2.22 (t, 2H), 1.55 (t, 2H), 1.44-1.32 (m, 4H); ¹³C NMR (DMSO, 75MHz, 25°C): δ(ppm) 159.44, 159.08, 154.85, 148.45, 141.27, 139.20, 134.29, 127.73, 125.32, 125.13, 118.35, 114.09, 69.86, 68.05, 57.42, 56.14, 49.09, 29.27, 26.05, 25.55, 20.34, 19.72;

### F. Synthesis of complex of formula (IIIc) (hereafter Cplx8)

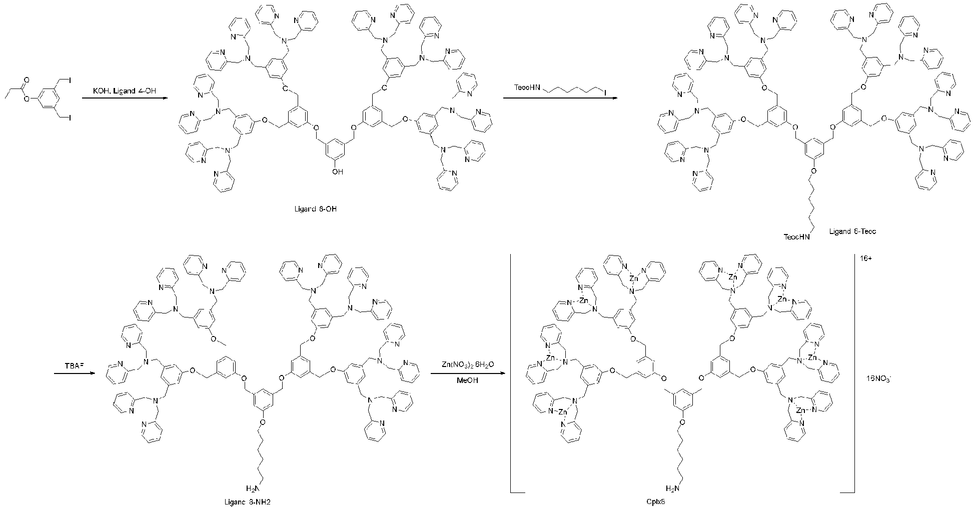

Ligand 8-OH : 1151 mg (1 mmol) ligand 4-OH was dissolved in 4 mL dimethylformamide under stirring. 126 mg (2.25 mmol) potassium hydroxide was added to the solution at room temperature. 1 h later, the mixture was cooled to -20 °C, then 220 mg (0.51 mmol) 3,5-bis(iodomethyl)phenyl propionate was added into the mixture. The reaction was allowed to slowly warm up to room temperature. 3 h later, the reaction was quenched with water. The mixture was extracted with 3x20 mL dichloromethane, the combined organic phase was washed with 2x20 mL brine, and dried with sodium sulfate. The solvent was removed with rotary evaporator to afford brown oil. The crude product was then purified by silica column chromatography with an eluent of ethyl acetate: acetonitrile= 5:1. The product is obtained as a pale yellow solid in 75% yield. ¹H NMR (DMSO, 300 MHz, 25°C): δ(ppm) 8.48-8.44 (m, 16H), 7.74-7.64 (m, 16H), 7.54-7.51 (m, 16H), 7.22-7.14 (m, 16H), 7.07 (d, 6H), 6.96 (s, 1H), 6.90 (d, 8H), 6.83 (s, 2H), 6.79 (s, 1H), 6.75 (s, 1H), 6.71 (s, 1H), 6.66 (s, 1H), 5.05-4.97(m, 12H), 3.69 (d, 32H), 3.57 (d, 16H); ¹³C NMR (CDCl₃, 75MHz, 25°C): δ(ppm) 159.68, 158.63, 157.79, 148.87, 140.61, 139.88, 138.99, 136.64, 122.84, 122.49, 122.05, 117.89, 114.94, 115.18, 69.96, 59.87, 58.33.

Ligand 8-Teoc : 605 mg (0.25 mmol) ligand 8-OH was dissolved in 10 mL dimethylformamide under stirring. 30 mg (0.54 mmol) potassium hydroxide was added to the solution at room temperature. 1 h later, the mixture was cooled to -20 °C, then 95 mg (0.256 mmol) 2-(trimethylsilyl)ethyl (6-iodohexyl)carbamate was added into the mixture. The reaction was allowed to slowly warm up to room temperature. After 3 h, the reaction was quenched with water. The mixture was extracted with 3x20 mL dichloromethane, and the combined organic phase was washed with 2x20 mL brine, and dried with sodium sulfate. The solvent was removed with rotary evaporator to afford brown oil. The crude product was then purified by silica gel column chromatography with an eluent of ethyl acetate: acetonitrile (5:1). The product is pale yellow solid obtained in 73% yield. ¹H NMR (DMSO, 300 MHz, 25°C): δ(ppm) 8.47-8.43 (m, 16H), 7.72-7.63 (m, 16H), 7.53-7.47 (m, 16H), 7.23-7.13 (m, 16H), 7.00 (b, 2H), 6.93-6.88 (m, 9H), 6.81 (s, 1H), 6.78 (s, 1H), 5.13-4.99 (m, 12H), 4.05-3.98 (m, 2H), 3.67 (d, 32H), 3.55 (d, 16H), 1.71-1.58 (m, 2H), 1.43-1.20 (m, 8H), 0.94-0.84 (m, 2H), 0.00(s, 9H); ¹³C NMR (CDCl₃, 75MHz, 25°C): δ(ppm) 162.55, 159.75, 159.67, 159.02, 148.99, 140.73, 139.09, 136.42, 122.73, 121.96, 121.88, 118.52, 113.82, 113.11, 69.91, 67.92, 60.10, 58.57, 40.86, 36.49, 30.94, 30.05, 29.19, 26.54, 25.79, 17.80, -1.44;

Ligand 8-NH₂ : 400 mg (0.15 mmol) ligand 8-Teoc was dissolved in 10 mL tetrahydrofuran under stirring. 1.5 mL of 1 M tetrabutylammonium fluoride solution was then added at room temperature. The reaction mixture was then kept at 60 °C overnight. After cooled to room temperature, the solvent was removed in vacuo. The residue was dissolved in dichloromethane, and then washed with 50 mL 0.5 M NaOH solution once and 50 mL saturated NaHCO₃ solution twice. The organic phase was dried with sodium sulfate. The solvent was removed with rotary evaporator to afford brown solid. The yield was 90%. ¹H NMR (DMSO, 300 MHz, 25°C): δ(ppm) 8.45-8.43 (m, 16H), 7.73-7.58 (m, 16H), 7.56-7.45 (m, 16H), 7.24-7.11 (m, 18H), 7.10-6.97 (m, 10H), 6.93-6.88 (m, 9H), 5.05-4.99 (m, 12H), 3.91-3.84 (m, 2H), 3.67 (b, 32H), 3.55 (b, 16H), 1.64-1.56 (m, 2H), 1.37-1.23 (m, 8H); ¹³C NMR (DMSO, 75MHz, 25°C): δ(ppm) 159.61, 159.29, 158.79, 149.79, 149.26, 140.72, 139.49, 136.95, 122.81, 122.57, 121.69, 114.10, 113.30, 69.48, 59.66, 57.94, 55.39, 41.52, 32.60, 29.05, 26.48, 25.80;

### Complex 8-NH₂ (molecule of formula IIIc - hereafter Cp1x8 ):

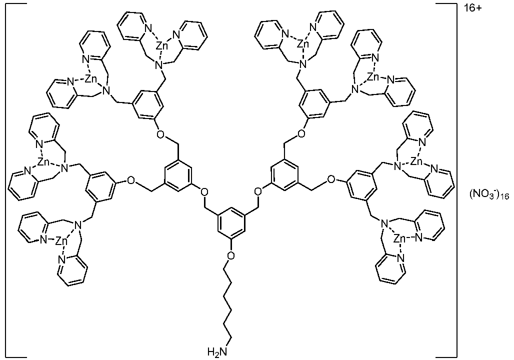

250 mg (0.1 mmol) ligand 8-NH₂ was dissolved in 10 mL methanol at room temperature under stirring. 30 mg (1 mmol) zinc nitrate hexahydrate was dissolved in 5 mL methanol and was added in to the solution of ligand dropwise. 12 h later, the solvent was removed under vacuum to give the final product as brown powder (yield 100%). ¹H NMR (DMSO, 300 MHz, 25°C): δ(ppm) 8.45 (b, 16H), 7.67 (b, 16H), 7.51 (b, 16H), 7.20 (b, 18H), 7.07 (b, 9H), 6.90 (b, 10H), 5.05 (b, 12H), 3.90 (b, 2H), 3.67 (b, 32H), 3.56 (b, 16H), 1.58 (b, 2H), 1.25 (b, 8H); ¹³C NMR (DMSO, 75MHz, 25°C): δ(ppm) 159.44, 159.08, 154.85, 148.45, 141.27, 139.20, 134.29, 127.73, 125.32, 125.13, 118.35, 114.09, 69.86, 68.05, 57.42, 56.14, 49.09, 29.27, 26.05, 25.55, 20.34, 19.72.

### Example 2: Ligand-PS-enriched microvesicle interaction study by PWR spectroscopy

The detailed setup of PWR and lipid bilayer constitution used herein is described in Calmet, et al., (Sci Rep, 2016. 6: p. 36181).

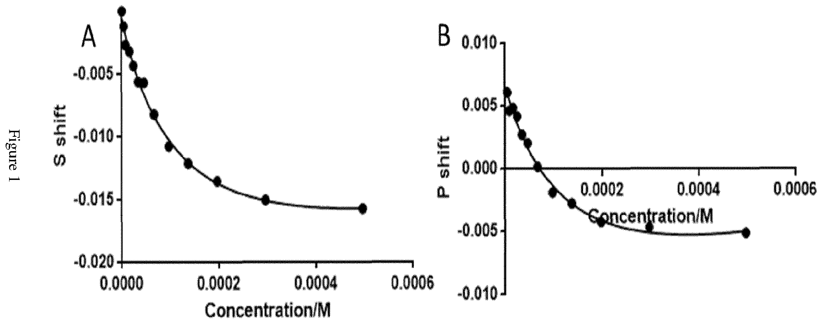

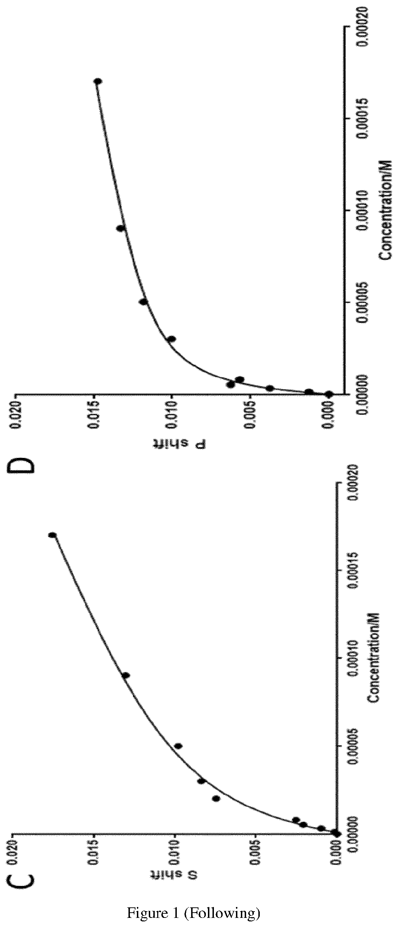

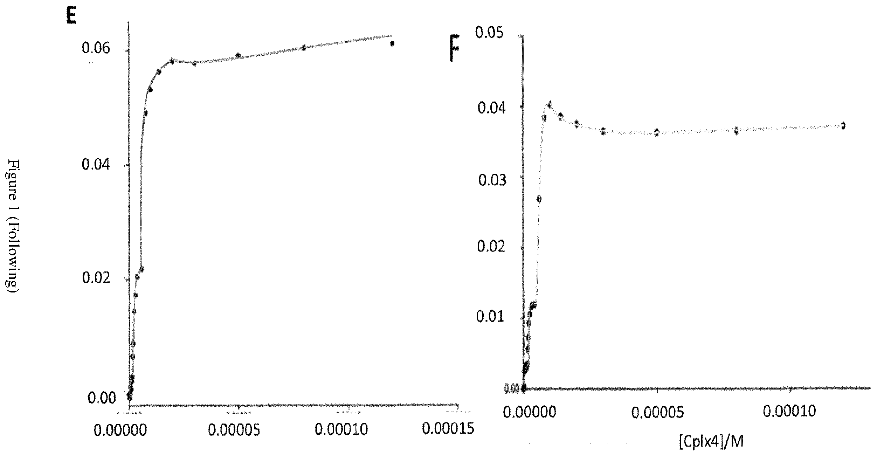

After the formation of POPC:POPS = 5:1 (w/w) bilayer on the prism surface, a titration experiment is performed for each complex. Briefly, each complex was dissolved in PBS buffer, and then titrated into the Teflon sample chamber. As equilibrium is reached in each addition, the complex concentration and the spectral shifts of s-polarized light (parallel to the membrane surface) was recorded. The titration stopped when addition of complex solution doesn't induce further change in spectral positions. The complex concentration was then plotted against the resonance angle, and the plot was fitted using GraghPad Prism 5. The acquired K_{d} values are shown in Table 1 hereunder. Figure 1 shows the titration curves for each tested ligand.

**Table 1 Complex-POPS-POPC membrane interaction dissociation constants**

| | Ligand | Clx1 | Cplx2 | Cplx4 |
|---|---|---|---|---|
| | S polarization | 1.25×10⁻⁴±3.163×10⁻⁵ | 2.092×10⁻⁵±9.509×10⁻⁶ | 1.771×10⁻⁷±9.617×10⁻⁸; |
| | | | | 8.622×10⁻⁷± 1.496×10⁻⁷; |
| K_{d} (M) | | | | 9.346×10⁻⁶±2.565×10⁻⁶; |
| | | | | 1.217×10⁻⁶±3.904x10⁷. |
| | P polarization | 1.498×10⁻⁴± 5.054×10⁻⁵ | 7.232×10⁻⁶ ± 2.178×10⁻⁶ | 1.42×10⁻⁷±2.811×10⁻⁸; |
| | | | | 2.282×10⁻⁷±2.811×10⁻⁸; |
| | | | | 3.489×10⁻⁶±1.849×10⁻⁷; |
| | | | | 6.056x10⁻⁶±1.208×10⁻⁶. |

### Example 3: Immobilization of ligands on PET film and characterization

### - Immobilization of ligands of the invention

10 cm² PET films were first cleaned with ethanol, and then immersed in a hydrolysis solution (20 mL water, 20 mL acetonitrile and 0.2 g NaOH). The hydrolysis was kept at 60 °C for 18 h. The films were cleaned with water, and then immersed in an oxidation solution (38.4 ml fresh milliQ water, 1.6 ml H₂SO₄, 2 g KMnO₄). The oxidation was kept at 60 °C for 1 h. The oxidized film was washed first with 50% HCl once and then water 3 times. The oxidized films were immersed in the activation solution (MES hydrate 390.5 mg/2 mmol, ethylcarbodiimide hydrochloride(EDC) 766.8 mg/4 mmol, N-hydroxysuccinimide(NHS) 230.18 mg/2 mmol), then kept at room temperature for 1h. The films were washed with water. The activated films were subsequently immersed in 20 mL 1 mM solution of complexes in DMSO under room temperature. 24 h later, the films were removed from the solution and washed intensively with water.

### - TBO characterization

The functionalization of the PET surface is constantly accompanied by the change of carboxyl group density on the material surface: the hydrolysis of ester bonds and the oxidation of alcohol groups increase the carboxyl density, while EDC/NHS activation and the immobilization of complexes successively decrease the carboxyl density. The absolute amount of the complexes immobilized onto PET surface is difficult to determine using established analytical methods; meanwhile the change of carboxyl group density can be easily determined by toluidine blue-o (TBO) adsorption test. Therefore, the TBO test can provide indirect evaluation of the complex density on PET surface. Briefly, a carboxyl group on material surface was first deprotonated in a basic medium, followed by the adsorption of a TBO cation. Provided that TBO reacts with carboxyl groups with the stoichiometry of 1:1, The surface density of carboxyl groups can be determined by measuring the amount of adsorbed TBO molecules. The adsorbed TBO molecules can be easily washed off with acid, and its concentration in acid can be determined by UV-Vis absorption.

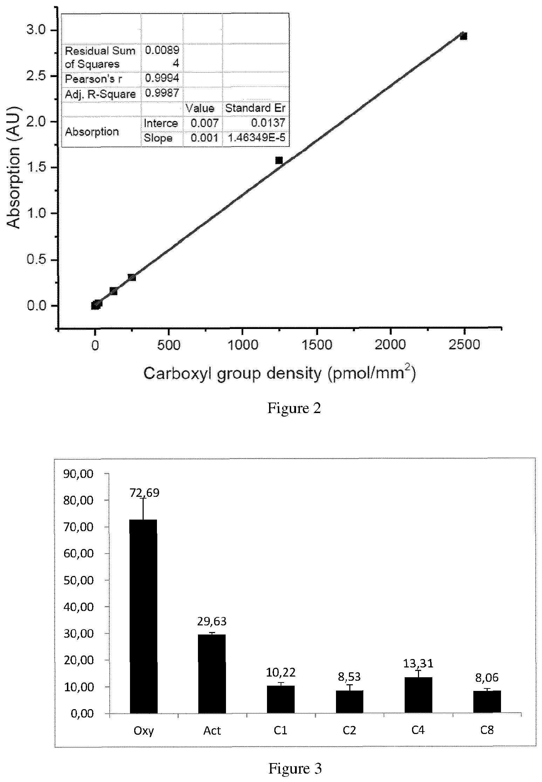

The TBO test is done following reported procedures (Chollet, C., et al., Biomol Eng, 2007. 24(5): p. 477-82.):
First, a linear correlation between TBO concentration in 50% AcOH and the solution's absorbance at 633nm (200 µL solution in each well of a transparent 96 well plate) was established using a series of TBO solutions shown in Tab. 3. In future TBO tests, 5 mL 50% acetic acid would be used to remove the TBO dye adsorbed on 1 cm² squares, and 200 µL of each solution would also be added into each individual well of a transparent 96 well plate for absorbance test. Thus TBO concentrations in Table 2 can then be further converted into molecule density on PET surface. The plot was then linearly fitted, as shown in Figure 2.

**Table 2: TBO solutions used in absorbance calibration**

| Desired TBO concentration (mM) | TBO solution used for dilution | 50% (V/V) acetic acid (µL) | Total amount |
|---|---|---|---|
| 0.5 | 100 µL of TBO 5mM | 900 | 1mL |
| 0.1 | 200 µL of TBO 5mM | 800 | 1mL |
| 0.05 | 100 µL of TBO 0.5mM | 900 | 1mL |
| 0.01 | 200 µL of TBO 0.5mM | 800 | 1mL |
| 0.005 | 100 µL of TBO 0.05mM | 900 | 1mL |
| 0.001 | 200 µL of TBO 0.05mM | 800 | 1mL |
| 0.0005 | 100 µL of TBO 0.005mM | 900 | 1mL |
| 0.0001 | 200 µL of TBO 0.005mM | 800 | 1mL |
| 0.00005 | 100 µL of TBO 0.0005mM | 900 | 1mL |
| 0.00001 | 200 µL of TBO 0.0005mM | 800 | 1mL |

After acquiring the calibration curve, the TBO test was performed:
A 5x10⁻⁴ M solution of toluidine blue-o solution was prepared by first dissolving NaOH into milli-Q water until pH 10, then dissolving toluidine blue-o to reach desired concentration. 1 cm by 1 cm PET squares were immersed in 10 mL of the TBO solution and then were kept under shaking in darkness at room temperature. 6 hours later, the supernatant was removed from tube and the stained PET surface was washed with 10 mL NaOH solution (pH 10) once and 2x10 mL pure water. The stained films were then immersed in 5 mL 50% acetic acid to remove the adsorbed TBO dye during 10 min. 200 µL of the washed solution from each sample was loaded into 96 well plate and the absorption at 633 nm is measured with a 96 well plate reader. The absorbance was then translated to the surface density of carboxyl groups and summarized in Figure 3 using the calibration curve shown in Figure 4. After the activation step, there is a drop of 43.06 pmol/mm² in COOH density, resulting from the substitution of hydrogen in COOH by NHS. A further drop in the COOH density can be observed after the grafting of the complexes onto the surface. This drop can result from 2 events: firstly, there is a reaction of amine and COOH in the presence of complexes, further eliminating the carboxyl groups on PET surface; secondly, when they are grafted on to PET surface, positively charged complexes repel the TBO+ ions, preventing them from adsorbing onto PET.

### - X-ray photoelectron spectroscopy (XPS)

A VG Scientific ESCALAB photoelectron spectrometer was used for the surface analysis with a non-monochromatized MgK 1253.6 eV source of 100 W. The area of the analytical X-ray spot on the sample surface is about 250 microns. We used a 45 degree insert angle that corresponds to 3-5 nm of analyzed depth. A flood gun was used for charge compensation. Acquisition of high resolution spectra was done at constant pass energy of 20 eV. Fitting was then realized with software provided by VG Scientific, each spectrum being referenced to carbon pollution at 284.8 eV. Binding energies values are given with a precision of ±0.2 eV.

**Table 3: The peak areas after fittings of the high resolution spectra (unit: atomic %)**

| Name | Oxy. | Act. | Cplx1 | Cplx2 | Cplx4 | Cplx8 |
|---|---|---|---|---|---|---|
| Si2p | 0.33 | 0.38 | 0.53 | 0.39 | 0.49 | 0.63 |
| S2p | 0.33 | 0.33 | 0.27 | 0.23 | 0.23 | 0.29 |
| Cl2p | 0.15 | 0.11 | 0 | 0 | 0 | 0 |
| C-C | 32.20 | 26.62 | 34.18 | 28.02 | 31.97 | 28.25 |
| C-CO | 12.36 | 14.64 | 15.46 | 18.06 | 15.08 | 23.28 |
| C-O | 12.39 | 14.44 | 13.73 | 13.55 | 14.15 | 13.69 |
| N-C=O | 0.24 | 1.55 | 2.29 | 2.19 | 1.38 | 2.85 |
| COO | 12.09 | 11.65 | 7.33 | 9.40 | 8.38 | 5.32 |
| 291.6 | 1.77 | 1.34 | 0.80 | 0.96 | 1.31 | 1.02 |
| N1s 398.0 | 0 | 0.27 | 0.13 | 0.09 | 0.23 | 0.01 |
| N1s 399.9 | 0 | 1.77 | 3.05 | 2.85 | 3.42 | 5.30 |
| N1s 401.9 | 0 | 1.05 | 0.28 | 0.29 | 0.28 | 0.40 |
| O1s 530.7 | 0 | 0 | 1.32 | 0.33 | 1.22 | 0.75 |
| O1s O=C | 13.41 | 14.02 | 11.63 | 11.81 | 11.32 | 10.37 |
| O1s O-C | 14.72 | 11.84 | 8.49 | 11.32 | 10.06 | 7.56 |
| Zn2p3 | 0 | 0 | 0.51 | 0.50 | 0.50 | 0.27 |

The peak areas after fittings of the high resolution spectra were summarized in Tab. 4. The XPS results well correspond to each treatment of PET surface: After hydrolysis and oxidation steps, the ester bonds were hydrolyzed and then oxidized into carboxyl groups; therefore the highest value of COO signal among all the samples is observed. In the activation step, NHS molecules substituted the hydrogen of carboxyl groups. In the process, number of surface carboxyl groups remains the same, while the NHS brings nitrogen onto the surface, leading to a significant increase in the N1s and N-C=O signals and no change in the -COO signal intensity. After the functionalization with complexes, multiple changes in the XPS spectra can be observed. 1^{st}, the emergence of Zn indicates the successful attachment of complexes onto material surface. 2^{nd}, the significant increase in C1s N-C=O/ C1s COO and N1s 399.9/ O1s O=C can be interpreted as the successful replacement of NHS by complexes, further confirming the covalent attachment of the complexes to PET surface. 3^{rd}, comparing the Zn content among the complex grated surfaces, Cplx1 has the most abundance on the surface while the other complexes became more and more difficult to attach to the material surface; meanwhile a comparison of N1s 399.9 signals indicate that Cplx1, Cplx2 and Cplx4 should have almost the same amount on the material surface and Cplx8 had the highest content on material surface. The two conclusions seem to be controversial with each other, but they can be explained if solubility and stability are taken into consideration: Cplx1, Cplx2 and Cplx4 are water soluble therefore the non-covalently attached molecules can be easily washed away in the washing process; Cplx8 on the other hand is insoluble in water, the adsorbed molecules will be much more difficult to remove by water. During the washing, water behaved as a complexing agent and remove the Zn(II) ion from the complex, leaving only the ligand on the surface. This effect can become more prominent with the increase of dendricity, when Zn(II) cations come closer to each other in space and experience more repelling force from each other.

For impurities appear in the XPS spectra, Si is most likely coming from the glass containers where we processed all the PET sheets; S is from the manufacturing of the material as it's concentration is relatively stable throughout the grafting procedures; lastly, Cl in oxidized and activated surface comes from the hydrochloric acid used in surface washing of PET.

### Example 4: Microvesicle capture and characterization by using cryo-SEM

### - Microvesicle preparation

Microvesicles were collected from mesenchymal stem cells. After 6 passages, TNFα was introduced into the incubation medium at a concentration of 100 ng/ml. After 36 h, the supernatant was collected, and then purified in 3 steps: 1) Removal of cellular debris: Centrifuge the incubation supernatant at 4 °C 1500 g for 15 min, take the supernatant and centrifuge at 4 °C 13000 g for another 2 min. 2) Concentrating microvesicles: Take the supernatant again, centrifuge at 4 °C 20000 g for 90 min and then take the pellet. 3) Wash the microvesicles: Re-disperse the pellet in 500 µL 1x PBS of 4 °C, centrifuge at 4 °C 20000 g for 90 min. Remove 400 µL of the supernatant without disturbing the pellet, then add another 400 µL fresh 1x PBS of 4 °C, re-disperse again, and centrifuge at 4 °C 20000 g for 90 min. Remove 450 µL of the supernatant without disturbing the pellet, then add 50 µL 1x PBS of 4 °C to redisperse the pellet. When not being used, the microvesicles were stored at - 80 °C.

### - Microvesicle staining

Stock of microvesicles was allowed to warm up to room temperature, and then 950 µL 1x PBS was used to dilute the suspension. 1 µL CellMask^{™} Deep Red Plasma membrane Stain was added into the diluted suspension to stain the microvesicles under room temperature. 15 min later, the stained suspension was centrifuged at 4 °C 20000 g for 90 min. 950 µL of the supernatant was then removed without disturbing the pellet, and another 950 µL of fresh PBS was added to redisperse the pellet. The centrifugation-redispersion step was repeated twice so as to remove the free CellMask^{™} Deep Red molecules from the PBS buffer.

### - Microvesicle characterization by fluorescence microscopy

Stained microvesicles were visible under fluorescence microscope (Leica microsystem DM5500B, microscope with a motorized, programmable stage using a CoolSnap HQ camera controlled by Metamorph 7.6). Fluorescent dots of different sizes were observed. Smaller ones should be monodispersed microvesicles, while the dots with larger diameter were suspected to be aggregations of microvesicles. Obviously, morphological studies of microvesicles were impossible to perform as their diameters are usually below the resolution of such instrument.

### - Microvesicle characterization using nanoparticle tracking analysis (NTA):

NTA experiment of prepared microvesicles was performed using NanoSight NS300 instrument. The analysis was performed under 22 °C, using 532nm laser beam as light source. The instrument was calibrated using the standard nanoparticle dispersions provided by the manufacturer before test.

For the test, stock of microvesicles was allowed to warm up to room temperature, and then 1x PBS was used to dilute the suspension to 1 mL. The suspension was then vortexed to reach an even distribution of microvesicles inside the dilution.

The NTA experiment was performed by 5 video recordings of 30s of the microvesicle dispersion flowing through the sample chamber at the syringe pump speed of 70 (AU).

The videos were simultaneously analyzed by software NTA 3.2 Dev Build 3.2.16, where the microvesicle concentration and size distribution per frame of picture were recorded.

The concentration of the microvesicles in 5 videos was determined to be 1.87×10⁸±4.69×10⁶ mL⁻¹. The uniformity of the microvesicle suspension was proved by small standard deviation in the 5 videos. Multiple subpopulations of particles of different sizes were found in the microvesicle suspension. The mean diameter of the total particles was 139.9±1.1nm, which is in good agreement with former published results, while only a small population of 2.45×10⁵ was found with the size below 50 nm, which were suspected to be exosomes.

### - Microvesicle capture

For all materials, the same procedure was used to capture the microvesicles: The stock microvesicle (stained with CellMask^{™} Deep Red for FL microscopy and nonstained for Cryo-scanning electron microscopy) dispersion was diluted to 2 mL with 1× PBS. 250µL of the dispersion was added onto a functionalized 1 cm2 PET square and was incubated under room temperature for 15 min. The PET squares were then washed with milli-Q water to remove any free vesicles adhered to the surface.

### - Observation of captured microvesicles using FL microscope

After the capturing process, fluorescence microscopy (Leica microsystem DM5500B, microscope with a motorized, programmable stage using a CoolSnap HQ camera controlled by Metamorph 7.6) of PET sheets was used to evaluate the capture ability of PET functionalized with different complexes.

### - Observation of captured microvesicles using Cryo-scanning electron microscopy (Cryo-SEM)

After the capturing process, PET sheets were mounted on freezing stub for the preparation chamber Quorum PP3000T specimen shuttle. The whole was plunged in slush nitrogen paste for cryo-fixation. After quick transfer under vacuum in the preparation chamber the samples were sublimed at -95°C during 30 min and then coated by platinum sputtering. They were at last transferred in the cryo-SEM Quanta 250 FEG chamber and kept at -140°C for observation at an accelerating voltage of 10kV.

### - Results

### • Figure 4 - Cplx1

Figure 4 shows how the microvesicles responded to the Cplx1 grafted surface. In FL micrograph (A), a small amount of donut shaped red fluorescent spots can be observed. These structures were of sizes around 1 µm, much larger than the diameter of microvesicles. On the cryo-SEM image (B), the PET surface was blank and of a smooth morphology. No vesicle structures can be observed. Both experiments indicate the PET surfaces functionalized with Cplx1 have very limited ability to capture microvesicles.

### • Figure 5 - Cplx2

Microvesicle capture using Cplx2 functionalized PET is shown in Figure 5A. The whole material surface was covered with membrane structures, although inhomogeneity was observed throughout the surface. While large areas of the surface were covered with fluorescent membranes, there were also bright dots of microvesicle size in the less fluorescent areas. Very bright membrane aggregations with size over 1 µm and of irregular shapes can also be observed. Figure 5B and C illustrates the surface of the same material observed using cryo-SEM. The majority of the PET surface was found as shown in Fig. 8B. PET was covered by coalesced membrane structures, while at the boundaries of the aggregations, holes in membrane structures and little amount of vesicle attached to PET surface can be observed.

Cplx2 functionalized surface was able to capture microvesicles. When in contact with the functionalized surface, vesicles are prone to fuse with each other into membranes. The fused membranes form web-like structures in less concentrated areas and form large aggregations at highly concentrated places. The interaction between PS and Cplx2 was strong enough to capture the vesicles, but the morphology of the microvesicles was destroyed. Contents within the vesicles were suspected to be lost during the capture process.

### • Figure 6 - Cplx4

As shown in Figure 6A and B, both FL micrograph and cryo-SEM show the microvesicles uniformly cover the whole surface functionalized with cplx4. After zooming in (Fig. 9C), microvesicles were found individually attached to the material surface, while no obvious fusion of membranes was observed. Cplx4 functionalized surface is also able to capture microvesicles. Due to the minimal intervesicular interactions, both the vesicle morphology and the contents within the microvesicles should be well preserved.

### • Figure 7 - Cplx8

FL micrograph and cryo-SEM in Fig. 7A and B both show the particles uniformly cover the whole surface functionalized with Cplx8. On the zoomed in cryo-SEM image (C), the particles were found to be of the same size as microvesicles, however irregular in shape.

Both FL micrograph and cryo-SEM confirmed that the cplx8 functionalized surface was able to capture particles in the microvesicle suspension, and the fluorescence clearly indicates that the particles consisted of phospholipids. The zoomed in cryo-SEM also show that the particles were of the same size as microvesicles without the vesicle structure as expected from microvesicles. Individual microvesicles were captured by the surface; however the vesicle membranes were destructed by the capture, leaving only the membrane on the surface, while the vesicle contents were lost.

### Example 5 : NMR Investigation of DPA-Zn Complex-PS Interaction

The zinc complex-PS interactions were studied in solution to preliminarily evaluate the binding ability of the complexes to PS. For this purpose, we adopted NMR investigation so that the peak shapes and chemical shifts can be used as evaluation criteria.

31P NMR spectroscopy was used to examine the effect on POPS when it is bound to the complexes because the chemical environment of phosphorous atom in 1-palmitoyl-2-oleoyl-sn-glycero-3-phospho-L-serine (POPS) would be the most affected should the complexes bind to the phosphate of POPS. Considering the similarity of the binding events (DPA-Zn to POPS), the changes in chemical shift can also roughly represent the strength of such interactions (in our study, it is assumed that the observed chemical shift is the mole fraction weighted average of the shifts of the free and DPA-Zn bound POPS). Indeed, when 1 equivalent of different complexes is added into 1 equivalent of POPS in solution, the changes of the 31P NMR chemical shifts varies significantly. Cplx1, Cplx2 and Cplx4 make the signal shift to high field (from 0.381 ppm for the free POPS to -1.48 ppm, -2.00 ppm and -2.88 ppm for Cplx1, Cplx2 and Cplx4 respectively), while Cplx8 shows signals at low field (0.65, 2.57, 3.70 and 6.29). From Cplx1 to Cplx4, with the increase of molecular dendricity, the change in chemical shift also increases, indicating a stronger binding ability with more binding sites. With the increase in DPA-Zn numbers, one obvious difference is the dendritic scaffolds to which the DPA-Zns are attached. To investigate the effect of the molecular scaffolds, another experiment was performed where the molar ratio of DPA-Zn units to POPS was fixed at 1 to 1. The resulting 31P NMR spectra (data not shown) show changes which are in good agreement with the assumption that DPA-Zn complexes bind to the phosphate groups on POPS, while Cplx4 has the strongest ability to bind to POPS.

## Claims

1. An *in vitro* method for capturing microvesicles present in a sample, which comprises contacting the sample with a ligand able to bind to microvesicles, said ligand comprising at least one moiety, preferably at least two moieties, of formula (I): 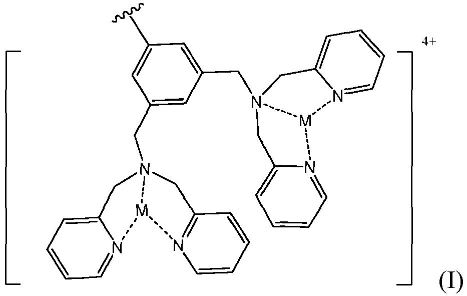 wherein M is a metal cation preferably selected from the group consisting of Zn²⁺, Mn²⁺, Co²⁺, Ni²⁺, Cu²⁺ and Fe²⁺, in conditions suitable to form a complex between said ligand and said microvesicle.

2. The in vitro method of claim 1, wherein the ligand comprises at least one moiety of formula (Ia) as follows: 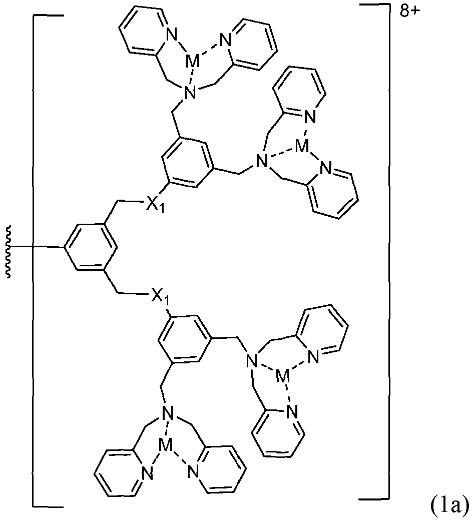 wherein:
- each X₁ is independently O, S, or NH,
- each X₂, when present, is independently O, S or NH,
- M is selected from Zn²⁺, Co²⁺, Cu²⁺ and Fe²⁺, preferably Zn²⁺.

3. The *in vitro* method of claim 2, wherein the ligand comprises the moiety of formula (Ib): 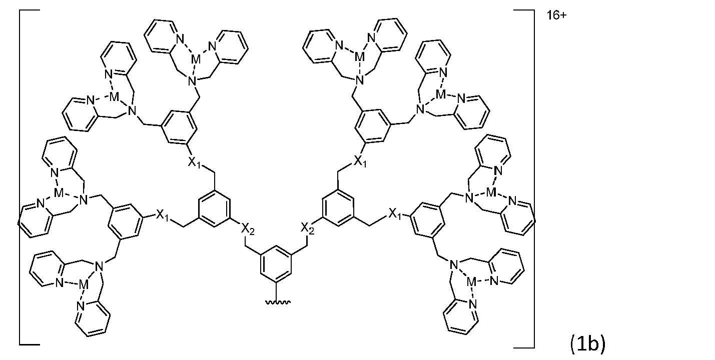 wherein:
- each X₁ is independently O, S, or NH,
- each X₂, when present, is independently O, S or NH,
- M is selected from Zn²⁺, Co²⁺, Cu²⁺ and Fe²⁺, preferably Zn²⁺.

4. The *in vitro* method of any one claims 1 to 3, wherein the ligand is a dendrimer comprising a branched core bearing a plurality of moieties of formula (I), said branched core preferably comprising a group selected from 3,5-di(hydroxymethyl) phenol, 3,5-di(thiomethyl)phenol, 3,5-di(thiomethyl) thiophenol, 3,5-dialkylphenol, 3,5-di(aminomethyl)phenol, 3,5-dialkylphenol and 3,5-di(aminomethyl) phenylamine as building block, more preferably comprising 3,5-di(hydroxymethyl) phenol as building block.

5. The *in vitro* method of any one of claim 1 to 4, wherein the ligand further comprises a mean for immobilization on a support attached at the extremity of a spacer chain, preferably the ligand is of formula (II): 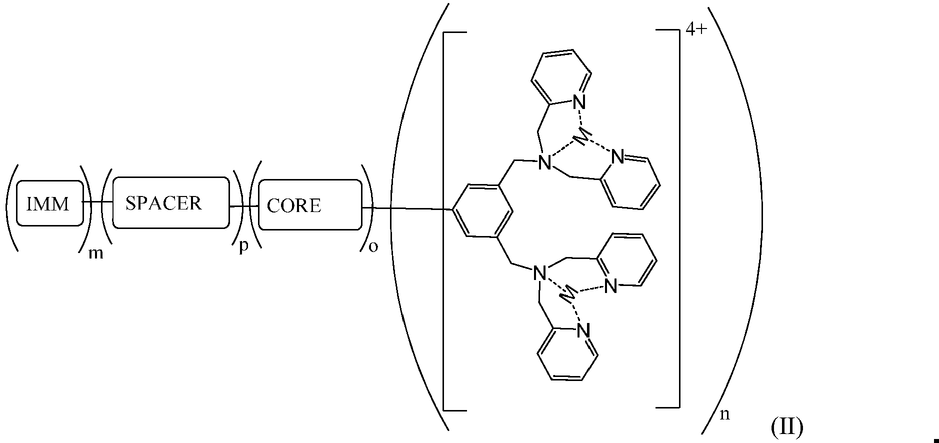 wherein:
- n is an integer from 1 to 10, preferably from 2 to 6, more preferably 2 or 4,
- m, p and o are independently 0 or 1,
- M is a divalent metal cation,
- [CORE] is a chemical entity bearing the at least one moiety of formula (I) and having a molecular weight of at most 20 000 g.mol⁻¹, preferably of at most 10 000 or 5000 g.mol⁻¹
- [IMM] is a mean for covalently or non-covalently immobilization on a support, and
- [SPACER] is selected from the group consisting of a peptide, a polypeptide, an oligo- or polysaccharide, a saturated or unsaturated hydrocarbon chain optionally interrupted by one or several heteroatoms (e.g. S, O or NH), optionally having an heteratoatom such as S, O and NH on at least one of its ends, and optionally substituted by one or several substituents such as hydroxyl, halogens, C₁-C₃ alcoxy, -CN, -CF₃, or C₁-C₃ alkyl, polymers including homopolymers, copolymers and block polymers, and combinations thereof.

6. The *in vitro* method of any one of claim 1 to 4, wherein the ligand is selected from compounds of formula (IIb) and (IIc): 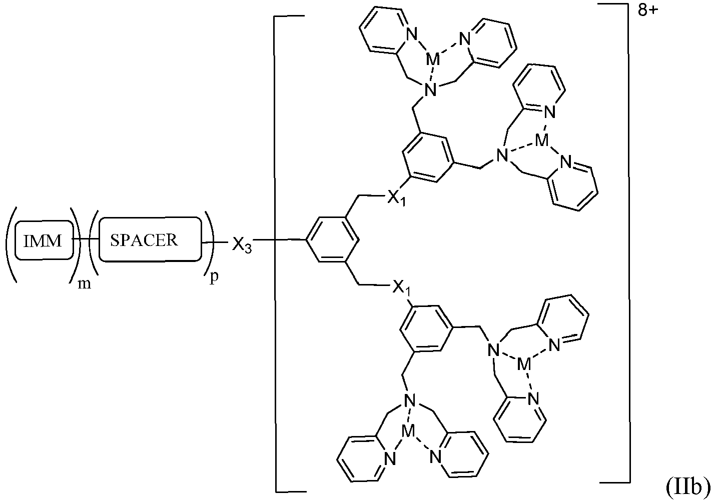 - and 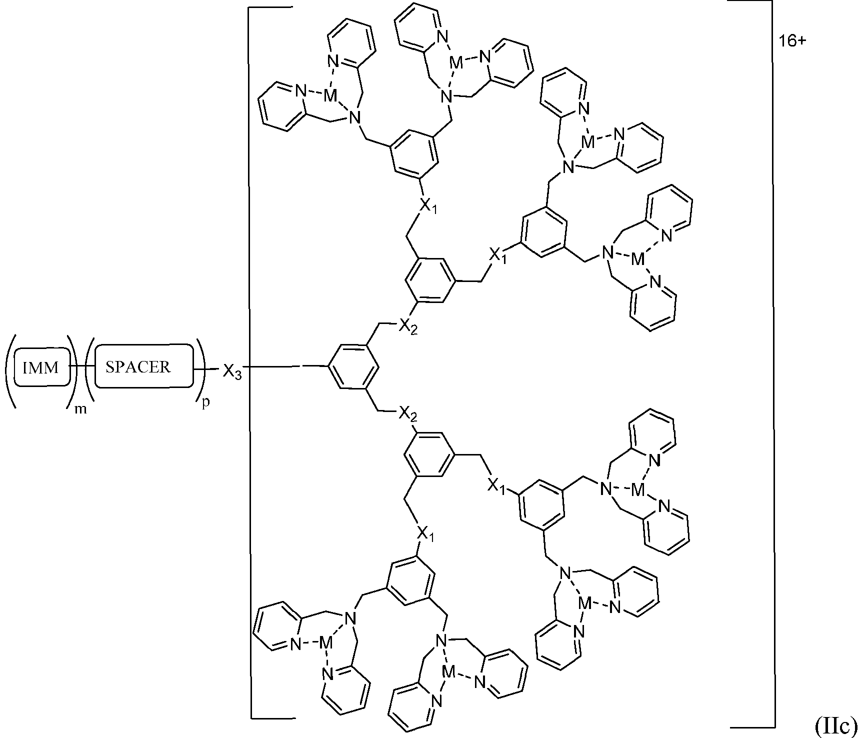 wherein
- m, p and o are independently 0 or 1,
- M is a divalent metal cation,
- [IMM] is a mean for covalently or non-covalently immobilization on a support
- [SPACER] is selected from the group consisting of a peptide, a polypeptide, an oligo- or polysaccharide, a saturated or unsaturated hydrocarbon chain optionally interrupted by one or several heteroatoms (e.g. S, O or NH), optionally having an heteratoatom such as S, O and NH on at least one of its ends, and optionally substituted by one or several substituents such as hydroxyl, halogens, C₁-C₃ alcoxy, -CN, -CF₃, or C₁-C₃ alkyl, polymers including homopolymers, copolymers and block polymers, and combinations thereof,
- each X₁ and X₂, when present, are selected from the group consisting of O, NH, and S.
- X₃ is selected from -O-, C(=O) -OC(O)-, -C(O)O-, -OC(O)O-, -S-, -SS-, -SC(O)-, - OC(S)-, NR₁-, -NR₁C(O)-, -C(O)NR₁-, NR₁C(S)-, C(S)NR₁-, -OC(O)S-, -OC(S)O-, - SC(O)O-, -OC(S)S-, -SC(O)S-, -SC(S)O-, -SC(S)S-, OC(O)NR₁-, -OC(S)NR₁-, - NR₁C(S)O-, -NR₁C(O)S-, NR₁C(O)NR₂-, -NR₁C(S)NR₂-, -SC(O)S-, -SC(S)O-, -S(O)-, -S(O)₂-, - -P(O)(R₁)-, -P(O)(OR1)-, P(O)(R₁)O-, OP(O)(OR₁)-, OP(O)(R₁)O-, NR₁P(O)(R₂)-, -NR₁P(O)(OR₂)-, NR₁P(O)(R₂)O-, OP(O)(OR₁₎₋ and OP(O)(R₁)O-wherein R₁ and R₂ are independently H or CH₃.

7. An *in vitro* method for the diagnosis, the differential diagnosis, the prognosis, the assessment of the risk of, and/or the monitoring a disorder in a subject which comprises:
(a) providing a sample from the subject susceptible to contain microvesicles,
(b) capturing the microvesicles possibly present in the sample by carrying out the method of any one of claims 1 to 6, and
(c) detecting, quantifying and/or characterizing the microvesicles captured in step (b) by complexation.
preferably wherein:
- the disorder is selected from the group consisting of diabetes and related disorders such as diabetic nephropathy, diabetic neuropathy, diabetic retinopathy, and diabetic foot syndrome, multiple sclerosis, cancer, Alzheimer's disease, Parkinson's disease, aneurysm, cerebral vasospasm, stroke, and coronary artery disease, and/or
- the sample is, or derives from, blood, blood plasma and urine.

8. A molecule able to bind to microvesicles and comprising at least one moiety of formula (1a) as follows: 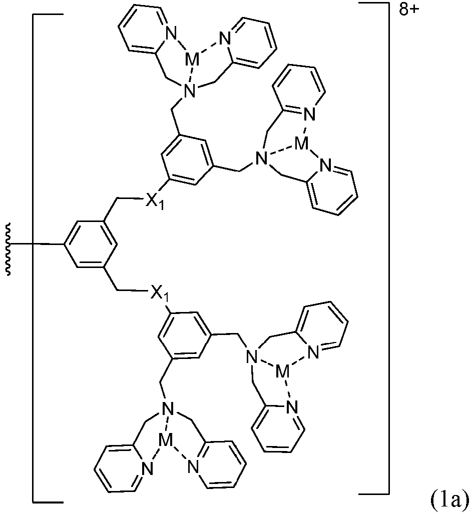 wherein:
- each X₁ is independently O, S, or NH,
- each X₂, when present, is independently O, S or NH,
- M is selected from Zn²⁺, Co²⁺, Cu²⁺ and Fe²⁺, preferably Zn²⁺.

9. The molecule according to claim 8, which comprises a moiety of formula (1b) 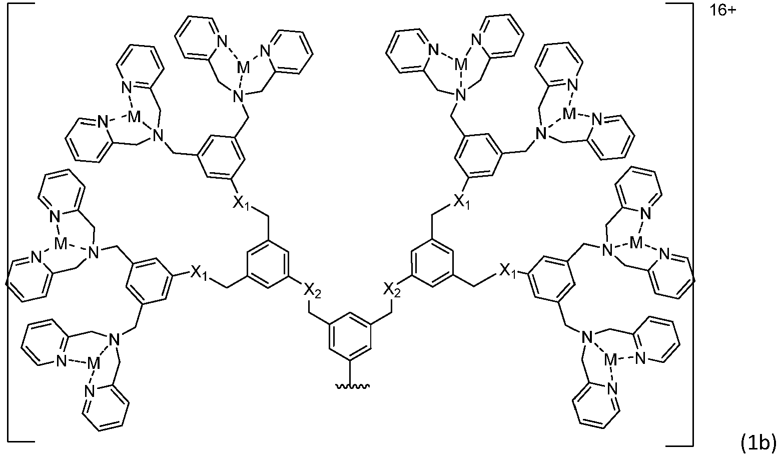 wherein:
- each X₁ is independently O, S, or NH,
- each X₂, when present, is independently O, S or NH,
- M is selected from Zn²⁺, Co²⁺, Cu²⁺ and Fe²⁺, preferably Zn²⁺.

10. The molecule of claim 8 or 9, which is a dendrimer comprising a branched core bearing a plurality of moieties of formula (I), said branched core preferably comprising a group selected from 3,5-di(hydroxymethyl) phenol, 3,5-di(thiomethyl)phenol, 3,5-di(thiomethyl) thiophenol, 3,5-dialkylphenol, 3,5-di(aminomethyl)phenol, 3,5-dialkylphenol and 3,5-di(aminomethyl) phenylamine as building block, more preferably comprising 3,5-di(hydroxymethyl) phenol as building block, and which preferably further comprises a mean for immobilization on a support attached at the extremity of a spacer chain.

11. The molecule of claim 10, which is of formula (IIb) and (IIc): 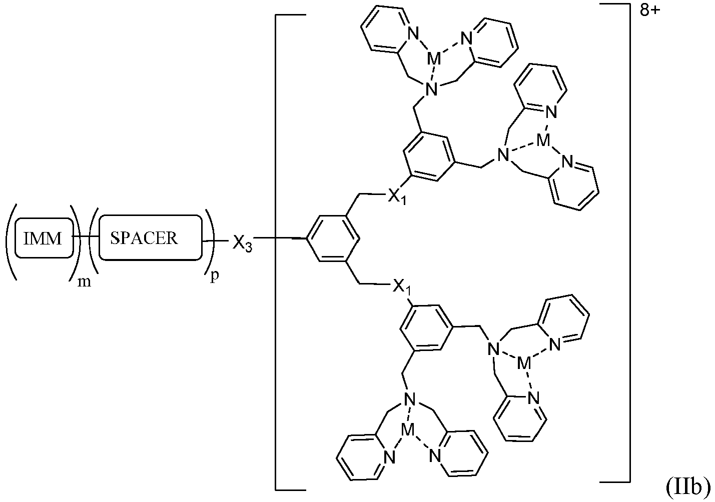
- and 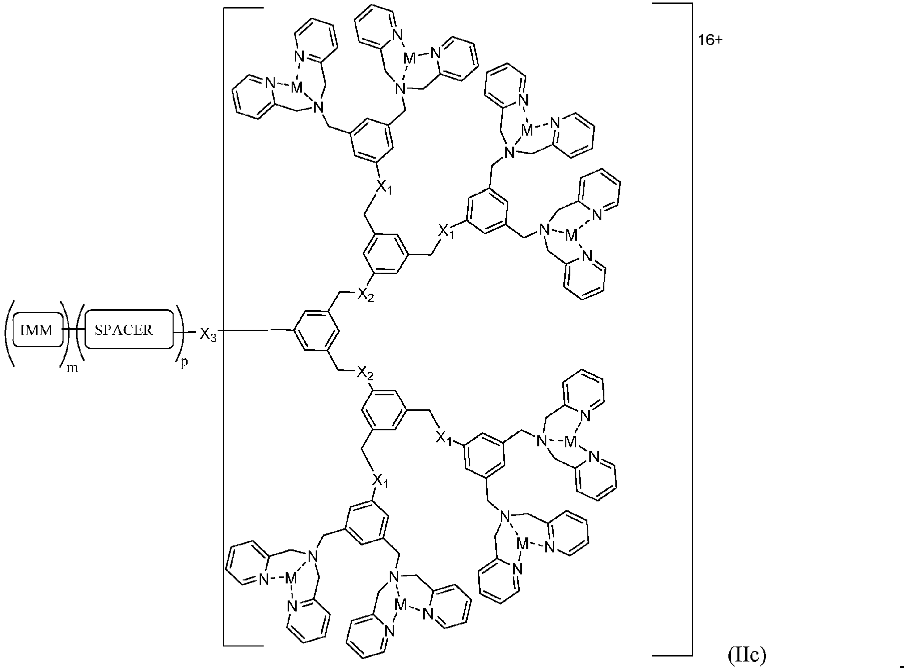 wherein
- m and p are independently 0 or 1,
- M is a divalent metal cation,
- [IMM] is a mean for covalently or non-covalently immobilization on a support, and
- [SPACER] is selected from the group consisting of a peptide, a polypeptide, an oligo- or polysaccharide, a saturated or unsaturated hydrocarbon chain optionally interrupted by one or several heteroatoms (e.g. S, O or NH), optionally having an heteratoatom such as S, O and NH on at least one of its ends, and optionally substituted by one or several substituents such as hydroxyl, halogens, C₁-C₃ alcoxy, -CN, -CF₃, or C₁-C₃ alkyl, polymers including homopolymers, copolymers and block polymers, and combinations thereof,
- each X₁ and X₂, when present, are selected from the group consisting of O, NH, and S.
- X₃ is selected from -O-, C(=O) -OC(O)-, -C(O)O-, -OC(O)O-, -S-, -SS-, -SC(O)-, -OC(S)-, NR₁-, -NR₁C(O)-, -C(O)NR₁-, NR₁C(S)-, C(S)NR₁-, -OC(O)S-, -OC(S)O-, - SC(O)O-, -OC(S)S-, -SC(O)S-, -SC(S)O-, -SC(S)S-, OC(O)NR₁-, -OC(S)NR₁-, -NR₁C(S)O-, -NR₁C(O)S-, NR₁C(O)NR₂-, -NR₁C(S)NR₂-, -SC(O)S-, -SC(S)O-, -S(O)-, -S(O)₂-, - -P(O)(R₁)-, -P(O)(OR1)-, P(O)(R₁)O-, OP(O)(OR₁)-, OP(O)(R₁)O-, NR₁P(O)(R₂)-, -NR₁P(O)(OR₂)-, NR₁P(O)(R₂)O-, OP(O)(OR₁₎₋ and OP(O)(R₁)O- wherein R₁ and R₂ are independently H or CH₃.

12. The molecule of claim 11, wherein the ligand of formula (IIb) or (IIc) is such that:
- m and p are 1,
- M is Zn²⁺, Co²⁺, Cu²⁺ or Fe²⁺, preferably Zn²⁺,
- X₁ and X₂, when present, are O,
- X₃ is O, NH, NHCO, CONH, O(C=O), (O=C)O, O(C=O)O, or NHCONH,
- [IMM]-[SPACER] is NH₂-(CH₂)ᵣ- or NH₂-[(CH₂)₂-O]ᵣ- with r an integer from 2 to 10, and
- Said molecule comprises one or several counter-anions selected from the group consisting of perchlorate, tosylate, nitrate, sulphate, sulphonate, thiosulfate, halide, hexafluorophosphate, tetraphenylborate, carbonate, and tetrafluoroborate.

13. The molecule of claim 12 which is selected from compounds of formula (IIIb) and (IIIc): 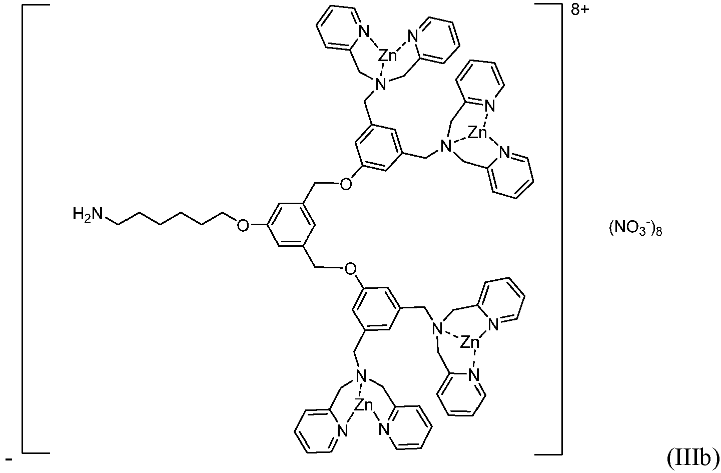 and

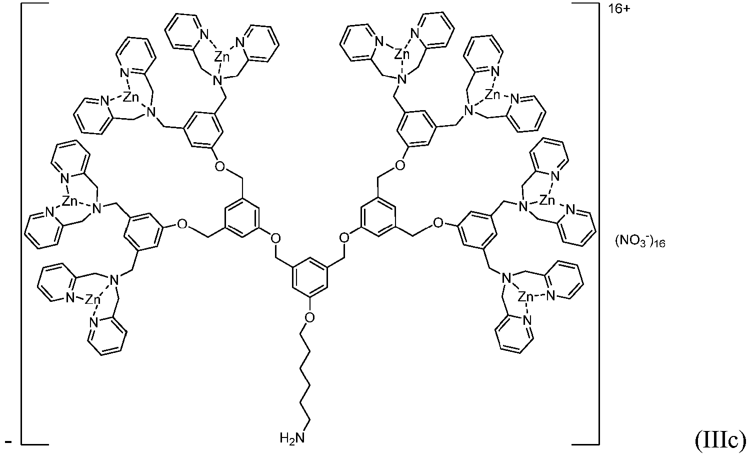

14. A support having thereon a ligand as defined in any one claims 8 to 13.

15. Use of a ligand as defined in any one of claims 8 to 13, or a support as defined in claim **14** as means for capturing microvesicles from a sample of a subject, preferably for the diagnosis, the prognostic or the monitoring of a disease in a subject such cancers, parasitic diseases, as diabetes and related disorders including diabetic nephropathy, diabetic neuropathy, diabetic retinopathy, and diabetic foot syndrome, multiple sclerosis, cancer, Alzheimer's disease, Parkinson's disease, aneurysm, cerebral vasospasm, stroke, and coronary artery disease.

## Patentansprüche

1. *In-vitro-*Verfahren zum Einfangen von in einer Probe vorhandenen Mikrovesikeln, das das Inkontaktbringen der Probe mit einem Liganden umfasst, der in der Lage ist, an Mikrovesikel zu binden, wobei der Ligand mindestens eine Gruppierung, vorzugsweise mindestens zwei Gruppierungen, der Formel (I) enthält: 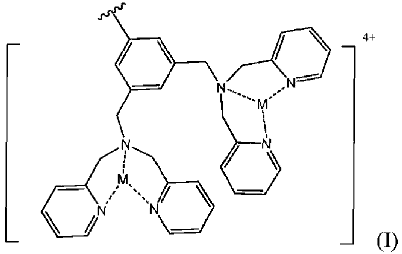 wobei
M ein Metallkation ist, das vorzugsweise aus der Gruppe, bestehend aus Zn²⁺, Mn²⁺, Co²⁺, Ni²⁺, Cu²⁺ und Fe²⁺, ausgewählt wird, unter Bedingungen, die geeignet sind, einen Komplex zwischen dem Liganden und dem Mikrovesikel zu bilden.

2. *In-vitro*-Verfahren nach Anspruch 1, wobei der Ligand mindestens eine Gruppierung der Formel (Ia) wie folgt umfasst: 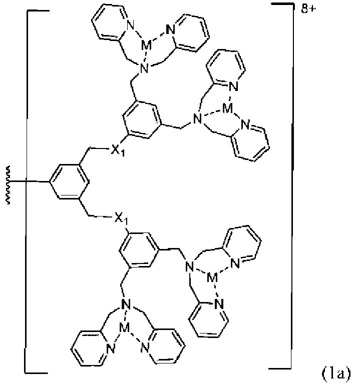 wobei:
- jedes X₁ unabhängig O, S oder NH ist,
- jedes X₂, sofern vorhanden, unabhängig O, S oder NH ist,
- M aus Zn²⁺, Co²⁺, Cu²⁺ und Fe²⁺, vorzugsweise Zn²⁺, ausgewählt wird.

3. *In-vitro*-Verfahren nach Anspruch 2, wobei der Ligand die Gruppierung der Formel (Ib) umfasst: 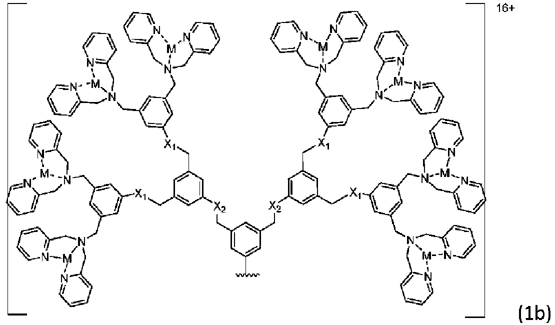 wobei:
- jedes X₁ unabhängig O, S oder NH ist,
- jedes X₂, sofern vorhanden, unabhängig O, S oder NH ist,
- M aus Zn²⁺, Co²⁺, Cu²⁺ und Fe²⁺, vorzugsweise Zn²⁺, ausgewählt wird.

4. *In-vitro*-Verfahren nach einem der Ansprüche 1 bis 3, wobei der Ligand ein Dendrimer ist, das einen verzweigten Kern umfasst, der eine Vielzahl von Gruppierungen der Formel (I) trägt, wobei der verzweigte Kern vorzugsweise eine Gruppe, ausgewählt aus 3,5-Di(hydroxymethyl)phenol, 3,5-Di(thiomethyl)phenol, 3,5-Di(thiomethyl)thiophenol, 3,5-Dialkylphenol, 3,5-Di(aminomethyl)phenol, 3,5-Dialkylphenol und 3,5-Di(aminomethyl)phenylamin, als Baustein umfasst und besonders bevorzugt 3,5-Di(hydroxymethyl)phenol als Baustein umfasst.

5. *In-vitro*-Verfahren nach einem der Ansprüche 1 bis 4, wobei der Ligand ferner ein Mittel zur Immobilisierung auf einem Träger umfasst, das am Ende einer Spacerkette befestigt ist, wobei der Ligand vorzugsweise die Formel (II) aufweist: 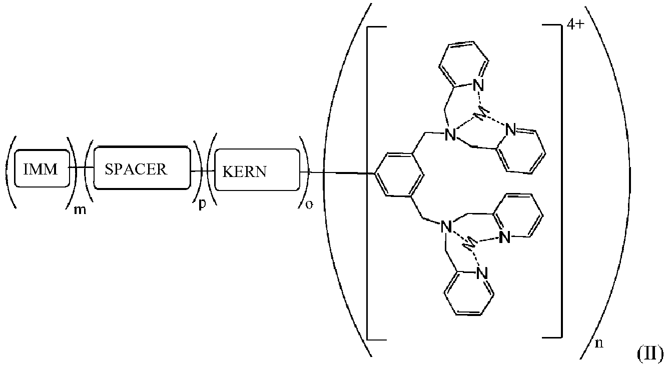 wobei:
- n eine ganze Zahl von 1 bis 10, vorzugsweise von 2 bis 6, besonders bevorzugt 2 oder 4, ist,
- m, p und o unabhängig voneinander 0 oder 1 sind,
- M ein zweiwertiges Metallkation ist,
- [KERN] eine chemische Einheit ist, die mindestens eine Gruppierung der Formel (I) trägt und ein Molekulargewicht von höchstens 20.000 g.mol⁻¹, vorzugsweise von höchstens 10.000 oder 5000 g.mol⁻¹, aufweist,
- [IMM] ein Mittel zur kovalenten oder nicht kovalenten Immobilisierung auf einem Träger, und
- [SPACER] aus der Gruppe ausgewählt wird, bestehend aus einem Peptid, einem Polypeptid, einem Oligo- oder Polysaccharid, einer gesättigten oder ungesättigten Kohlenwasserstoffkette, die optional durch ein oder mehrere Heteroatome (z. B. S, O oder NH) unterbrochen ist, optional an mindestens einem ihrer Enden ein Heteroatom wie S, O oder NH aufweist und optional durch einen oder mehrere Substituenten wie Hydroxyl, Halogene, C₁-C₃-Alkoxy, -CN, -CF₃ oder C₁-C₃-Alkyl substituiert ist, Polymeren, einschließlich Homopolymeren, Copolymeren und Blockpolymeren, sowie Kombinationen davon.

6. *In-vitro*-Verfahren nach einem der Ansprüche 1 bis 4, wobei der Ligand aus Verbindungen der Formel (IIb) und (IIc) ausgewählt ist: 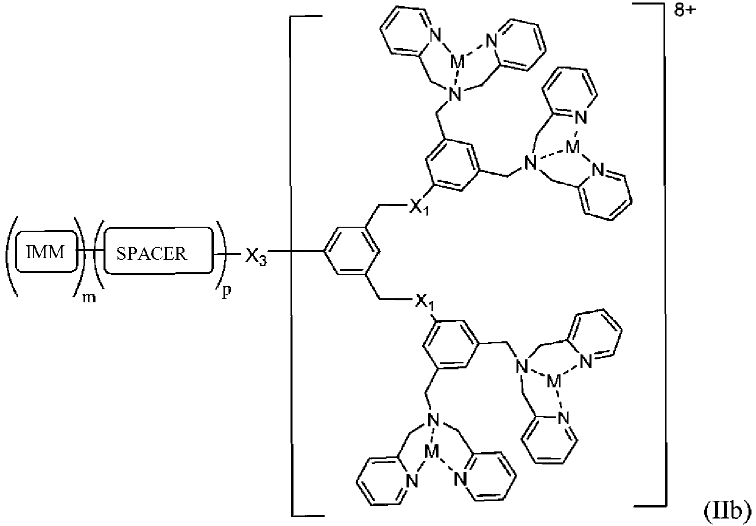
- und 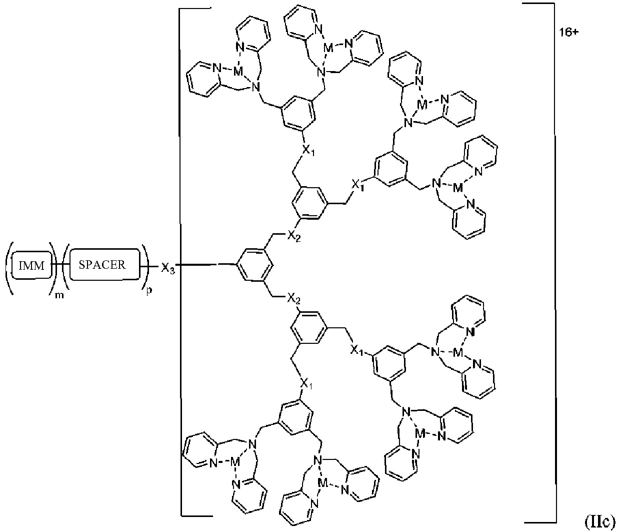 wobei
- m, p und o unabhängig voneinander 0 oder 1 sind,
- M ein zweiwertiges Metallkation ist,
- [IMM] ein Mittel zur kovalenten oder nicht kovalenten Immobilisierung auf einem Träger ist,
- [SPACER] aus der Gruppe ausgewählt wird, bestehend aus einem Peptid, einem Polypeptid, einem Oligo- oder Polysaccharid, einer gesättigten oder ungesättigten Kohlenwasserstoffkette, die optional durch ein oder mehrere Heteroatome (z, B. S, O oder NH) unterbrochen ist, optional an mindestens einem ihrer Enden ein Heteroatom wie S, O oder NH aufweist und optional durch einen oder mehrere Substituenten wie Hydroxyl, Halogene, C₁-C₃-Alkoxy, -CN, -CF₃ oder C₁-C₃-Alkyl substituiert ist, Polymeren, einschließlich Homopolymeren, Copolymeren und Blockpolymeren, sowie Kombinationen davon,
- jedes X₁ und X₂, sofern vorhanden, aus der Gruppe, bestehend aus O, NH und S, ausgewählt wird.
- X₃ aus -O-, C(=O) -OC(O)-, -C(O)O-, -OC(O)O-, -S-, -SS-, -SC(O)-, -OC(S)-, NR₁-, -NR₁C(O)-, -C(O)NR₁-, NR₁C(S)-, C(S)NR₁-, -OC(O)S-, -OC(S)O-, -SC(O)O-, -OC(S)S-, - SC(O)S-, -SC(S)O-, -SC(S)S-, OC(O)NR₁-, -OC(S)NR₁-, -NR₁C(S)O-, -NR₁C(O)S-, NR₁C(O)NR₂-, -NR₁C(S)NR₂-, -SC(O)S-, -SC(S)O-, -S(O)-, -S(O)₂-, -P(O)(R₁)-, - P(O)(OR1)-, P(O)(R₁)O-, OP(O)(OR₁)-, OP(O)(R₁)O-, NR₁P(O)(R₂)-, -NR₁P(O)(OR₂)-, NR₁P(O)(R₂)O-, OP(O)(OR₁₎- und OP(O)(R₁)O- ausgewählt wird, wobei R₁ und R₂ unabhängig voneinander H oder CH₃ sind.

7. *In-vitro*-Verfahren zur Diagnose, Differentialdiagnose, Prognose, Risikobewertung und/oder Überwachung einer Erkrankung bei einem Probanden, das umfasst:
(a) Bereitstellen einer Probe vom Probanden, die Mikrovesikel enthalten kann,
(b) Einfangen der möglicherweise in der Probe vorhandenen Mikrovesikel durch das Durchführen des Verfahrens nach einem der Ansprüche 1 bis 6, und
(c) Erkennen, Quantifizieren und/oder Charakterisieren der in Schritt (b) durch Komplexierung eingefangenen Mikrovesikel.
wobei vorzugsweise:
- die Erkrankung aus der Gruppe ausgewählt wird, bestehend aus Diabetes und verwandten Erkrankungen wie diabetischer Nephropathie, diabetischer Neuropathie, diabetischer Retinopathie und diabetischem Fußsyndrom, Multipler Sklerose, Krebs, Alzheimer-Krankheit, Parkinson-Krankheit, Aneurysma, zerebralem Vasospasmus, Schlaganfall und koronarer Herzkrankheit, und/oder
- die Probe Blut, Blutplasma und Urin ist oder von diesen stammt.

8. Molekül, das in der Lage ist, an Mikrovesikel zu binden, und mindestens eine Gruppierung der Formel (1a) wie folgt umfasst: 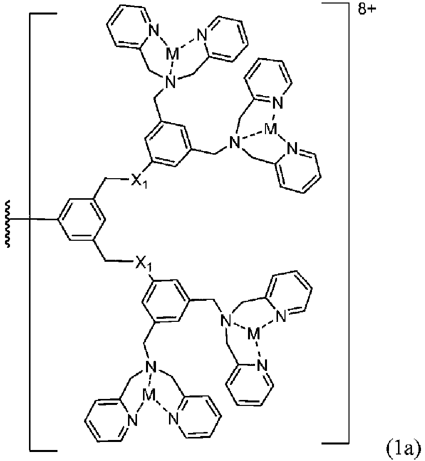 wobei:
- jedes X₁ unabhängig O, S oder NH ist,
- jedes X₂, sofern vorhanden, unabhängig O, S oder NH ist,
- M aus Zn²⁺, Co²⁺, Cu²⁺ und Fe²⁺, vorzugsweise Zn²⁺, ausgewählt wird.

9. Molekül nach Anspruch 8, das eine Gruppierung der Formel (1b) umfasst, 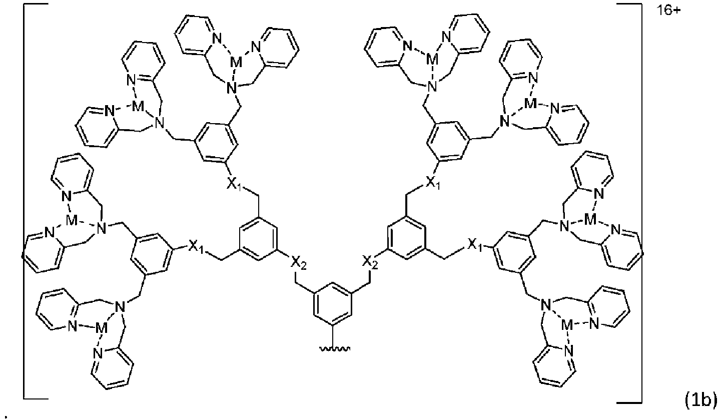 wobei:
- jedes X₁ unabhängig O, S oder NH ist,
- jedes X₂, sofern vorhanden, unabhängig O, S oder NH ist,
- M aus Zn²⁺, Co²⁺, Cu²⁺ und Fe²⁺, vorzugsweise Zn²⁺, ausgewählt wird.

10. Molekül nach Anspruch 8 oder 9, welches ein Dendrimer ist, das einen verzweigten Kern umfasst, der eine Vielzahl von Gruppierungen der Formel (I) trägt, wobei der verzweigte Kern vorzugsweise eine Gruppe, ausgewählt aus 3,5-Di(hydroxymethyl)phenol, 3,5-Di(thiomethyl)phenol, 3,5-Di(thiomethyl)thiophenol, 3,5-Dialkylphenol, 3,5-Di(aminomethyl)phenol, 3,5-Dialkylphenol und 3,5-Di(aminomethyl)phenylamin, als Baustein umfasst und besonders bevorzugt 3,5-Di(hydroxymethyl)phenol als Baustein umfasst, und welches vorzugsweise ferner ein Mittel zur Immobilisierung auf einem Träger umfasst, das am Ende einer Spacerkette befestigt ist.

11. Molekül nach Anspruch 10, das die Formel (IIb) und (IIc) aufweist: 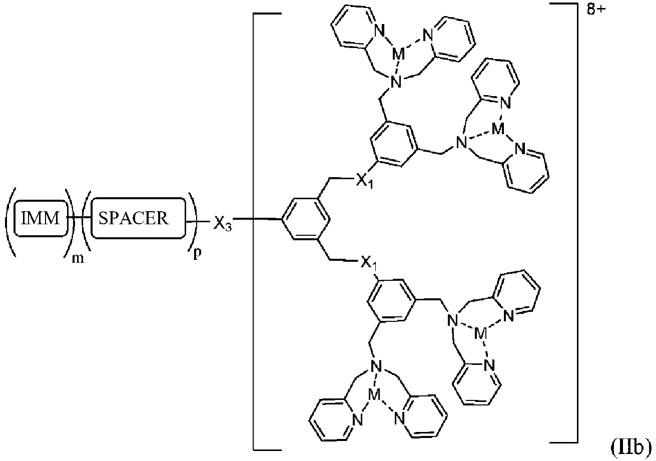
- und 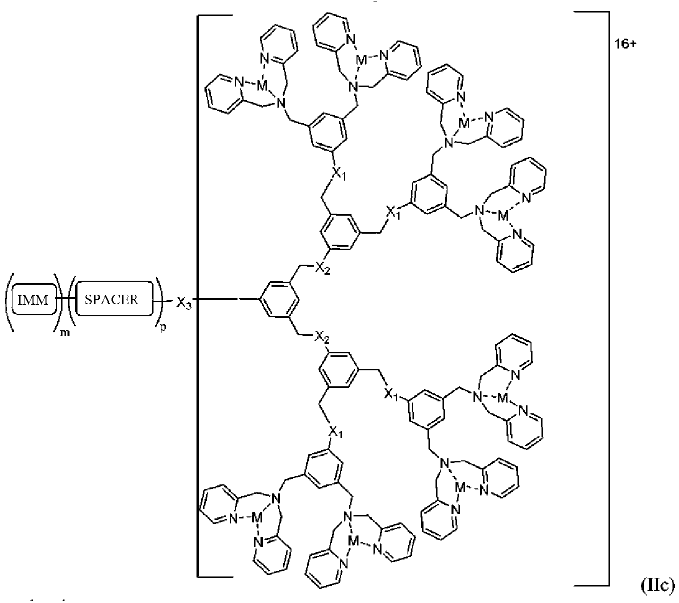 wobei
- m und p unabhängig voneinander 0 oder 1 sind,
- M ein zweiwertiges Metallkation ist,
- [IMM] ein Mittel zur kovalenten oder nicht kovalenten Immobilisierung auf einem Träger, und
- [SPACER] aus der Gruppe ausgewählt wird, bestehend aus einem Peptid, einem Polypeptid, einem Oligo- oder Polysaccharid, einer gesättigten oder ungesättigten Kohlenwasserstoffkette, die optional durch ein oder mehrere Heteroatome (z, B. S, O oder NH) unterbrochen ist, optional an mindestens einem ihrer Enden ein Heteroatom wie S, O oder NH aufweist und optional durch einen oder mehrere Substituenten wie Hydroxyl, Halogene, C₁-C₃-Alkoxy, -CN, -CF₃ oder C₁-C₃-Alkyl substituiert ist, Polymeren, einschließlich Homopolymeren, Copolymeren und Blockpolymeren, sowie Kombinationen davon,
- jedes X₁ und X₂, sofern vorhanden, aus der Gruppe, bestehend aus O, NH und S, ausgewählt wird.
- X₃ aus -O-, C(=O) -OC(O)-, -C(O)O-, -OC(O)O-, -S-, -SS-, -SC(O)-, -OC(S)-, NR₁-, -NR₁C(O)-, -C(O)NR₁-, NR₁C(S)-, C(S)NR₁-, -OC(O)S-, -OC(S)O-, -SC(O)O-, -OC(S)S-, - SC(O)S-, -SC(S)O-, -SC(S)S-, OC(O)NR₁-, -OC(S)NR₁-, -NR₁C(S)O-, -NR₁C(O)S-, NR₁C(O)NR₂-, -NR₁C(S)NR₂-, -SC(O)S-, -SC(S)O-, -S(O)-, -S(O)₂-, -P(O)(R₁)-, - P(O)(OR1)-, P(O)(R)O-, OP(O)(OR₁)-, OP(O)(R₁)O-, NR₁P(O)(R₂)-, -NR₁P(O)(OR₂)-, NR₁P(O)(R₂)O-, OP(O)(OR₁₎- und OP(O)(R₁)O- ausgewählt wird, wobei R₁ und R₂ unabhängig voneinander H oder CH₃ sind.

12. Molekül nach Anspruch 11, wobei das Ligand der Formel (IIb) oder (IIc) derart ist, dass:
- m und p 1 sind,
- M Zn²⁺, Co²⁺, Cu²⁺ oder Fe²⁺, vorzugsweise Zn²⁺, ist,
- X₁ und X₂, sofern vorhanden, O sind,
- X₃ O, NH, NHCO, CONH, O(C=O), (O=C)O, O(C=O)O oder NHCONH ist,
- [IMM]-[SPACER] NH₂-(CH₂)ᵣ- oder NH₂-[(CH₂)₂-O]ᵣ- ist, wobei r eine ganze Zahl von 2 bis 10 ist, und
- wobei das Molekül ein oder mehrere Gegenanionen umfasst, ausgewählt aus der Gruppe, bestehend aus Perchlorat, Tosylat, Nitrat, Sulfat, Sulfonat, Thiosulfat, Halogenid, Hexafluorophosphat, Tetraphenylborat, Carbonat und Tetrafluoroborat.

13. Molekül nach Anspruch 12, das aus Verbindungen der Formel (IIIb) und (IIIc) ausgewählt ist: 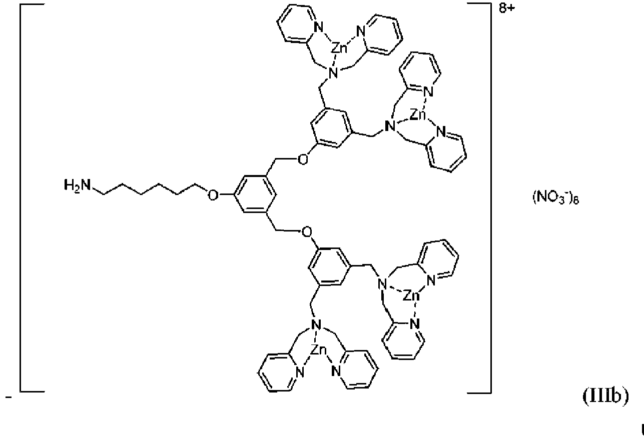 und

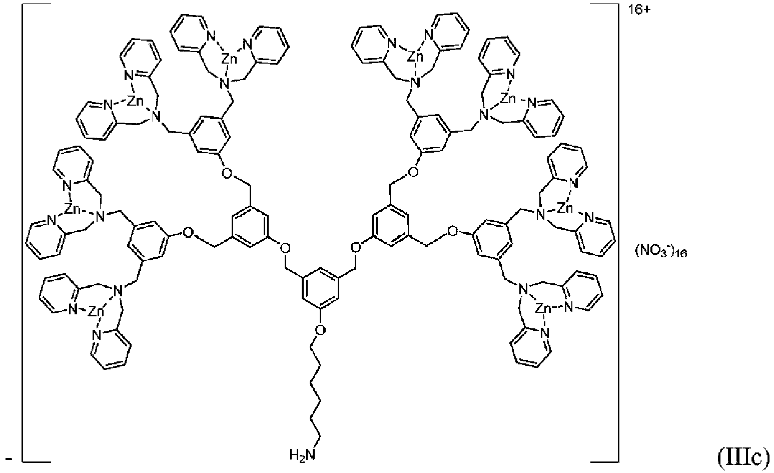

14. Träger, auf dem ein Ligand gemäß einem der Ansprüche 8 bis 13 vorhanden ist.

15. Verwendung eines Liganden nach einem der Ansprüche 8 bis 13 oder eines Trägers nach Anspruch 14 als Mittel zum Einfangen von Mikrovesikeln aus einer Probe eines Probanden, vorzugsweise zur Diagnose, Prognose oder Überwachung einer Erkrankung bei einem Probanden, wie Krebs, parasitäre Krankheiten, Diabetes und verwandte Erkrankungen, einschließlich diabetischer Nephropathie, diabetischer Neuropathie, diabetischer Retinopathie und diabetischem Fußsyndrom, Multiple Sklerose, Krebs, Alzheimer-Krankheit, Parkinson-Krankheit, Aneurysma, zerebraler Vasospasmus, Schlaganfall und koronare Herzkrankheit.

## Revendications

1. Méthode *in vitro* permettant de capturer des microvésicules présentes dans un échantillon, qui comporte la mise en contact de l'échantillon avec un ligand capable de se lier à des microvésicules, ledit ligand comportant au moins une fraction, de préférence au moins deux fractions, de formule (I) : 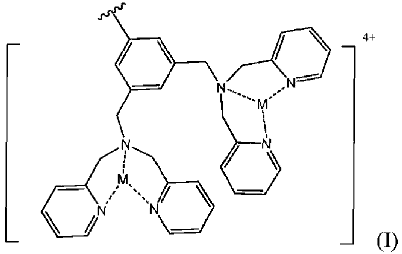 dans laquelle
M est un cation métallique de préférence choisi dans le groupe constitué de Zn²⁺, Mn²⁺, Co²⁺, Ni²⁺, Cu²⁺ et Fe²⁺, dans des conditions appropriées pour former un complexe entre ledit ligand et ladite microvésicule.

2. Méthode *in vitro* selon la revendication 1, dans laquelle le ligand comporte au moins une fraction de formule (Ia) comme suit : 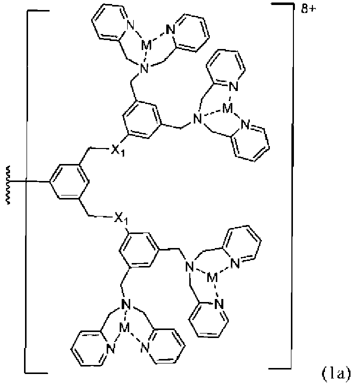 dans laquelle :
- chaque X₁ est indépendamment O, S, ou NH,
- chaque X₂, lorsqu'il est présent, est indépendamment O, S ou NH,
- M est choisi parmi Zn²⁺, Co²⁺, Cu²⁺ et Fe²⁺, de préférence Zn²⁺.

3. Méthode *in vitro* selon la revendication 2, dans laquelle le ligand comporte la fraction de formule (Ib) : 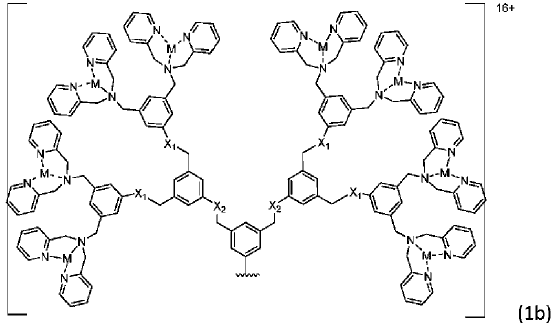 dans laquelle :
- chaque X₁ est indépendamment O, S, ou NH,
- chaque X₂, lorsqu'il est présent, est indépendamment O, S ou NH,
- M est choisi parmi Zn²⁺, Co²⁺, Cu²⁺ et Fe²⁺, de préférence Zn²⁺.

4. Méthode *in vitro* selon l'une quelconque des revendications 1 à 3, dans laquelle le ligand est un dendrimère comportant un noyau ramifié portant une pluralité de fractions de formule (I), ledit noyau ramifié comportant de préférence un groupe choisi parmi le 3,5-di(hydroxyméthyl)phénol, le 3,5-di(thiométhyl)phénol, le 3,5-di(thiométhyl)thiophénol, le 3,5-dialkylphénol, le 3,5-di(aminométhyl)phénol, le 3,5-dialkylphénol et le 3,5-di(aminométhyl)phénylamine comme élément constitutif, comportant plus préférentiellement le 3,5-di(hydroxyméthyl)phénol comme élément constitutif.

5. Méthode *in vitro* selon l'une quelconque des revendications 1 à 4, dans laquelle le ligand comporte en outre un moyen d'immobilisation sur un support fixé au niveau de l'extrémité d'une chaîne d'espacement, de préférence le ligand est de formule (II) : 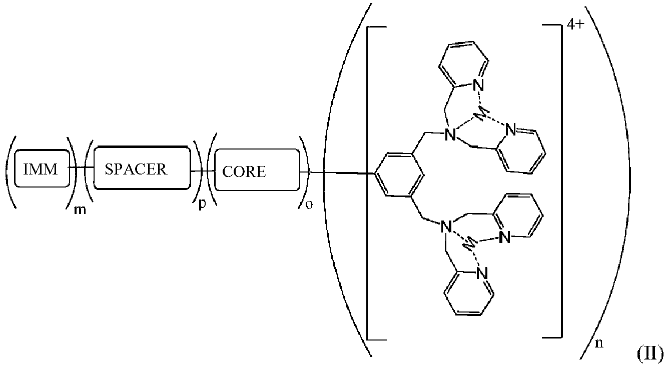 dans laquelle :
- n est un entier de 1 à 10, de préférence de 2 à 6, plus préférentiellement de 2 ou 4,
- m, p et o sont indépendamment 0 ou 1,
- M est un cation métallique divalent,
- [CORE] est une entité chimique portant au moins une fraction de formule (I) et présentant un poids moléculaire d'au plus 20 000 g.mol⁻¹, de préférence d'au plus 10 000 ou 5 000 g.mol⁻¹
- [IMM] est un moyen d'immobilisation covalente ou non covalente sur un support, et
- [SPACER] est choisi dans le groupe constitué d'un peptide, d'un polypeptide, d'un oligo- ou polysaccharide, d'une chaîne hydrocarbonée saturée ou insaturée éventuellement interrompue par un ou plusieurs hétéroatomes (par exemple S, O ou NH), présentant éventuellement un hétéroatome tel que S, O et NH sur au moins une de ses extrémités, et éventuellement substituée par un ou plusieurs substituants tels que hydroxyle, halogènes, alcoxy en C₁-C₃, -CN, -CF₃, ou alkyle en C₁-C₃, des polymères comprenant des homopolymères, copolymères et polymères séquencés, ainsi que des combinaisons de ceux-ci.

6. Méthode *in vitro* selon l'une quelconque des revendications 1 à 4, dans laquelle le ligand est choisi parmi des composés de formule (IIb) et (IIc) : 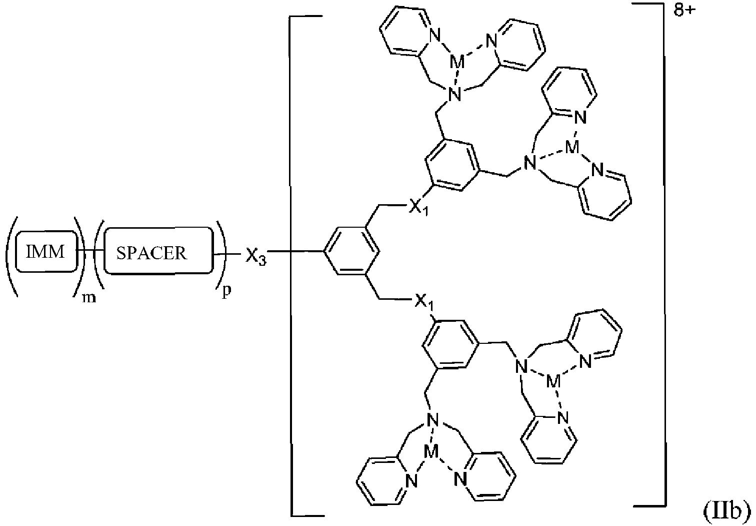
- et 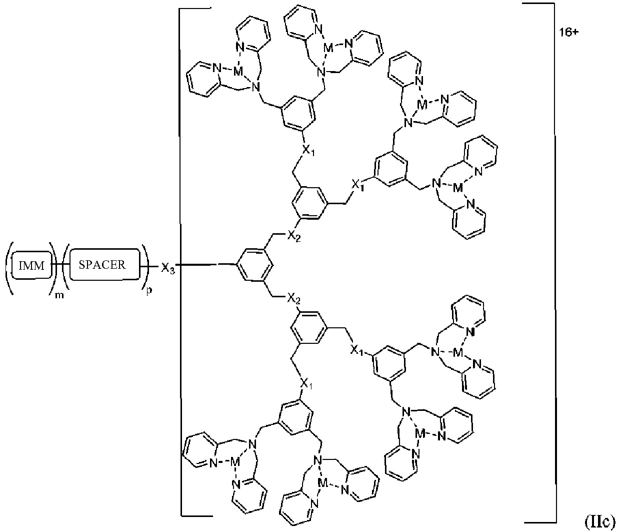 dans laquelle
- m, p et o sont indépendamment 0 ou 1,
- M est un cation métallique divalent,
- [IMM] est un moyen d'immobilisation covalente ou non covalente sur un support
- [SPACER] est choisi dans le groupe constitué d'un peptide, d'un polypeptide, d'un oligo- ou polysaccharide, d'une chaîne hydrocarbonée saturée ou insaturée éventuellement interrompue par un ou plusieurs hétéroatomes (par exemple S, O ou NH), présentant éventuellement un hétéroatome tel que S, O et NH sur au moins une de ses extrémités, et éventuellement substituée par un ou plusieurs substituants tels que hydroxyle, halogènes, alcoxy en C₁-C₃, -CN, -CF₃, ou alkyle en C₁-C₃, des polymères comprenant des homopolymères, copolymères et polymères séquencés, et des combinaisons de ceux-ci,
- chaque X₁ et X₂, lorsqu'ils sont présents, sont choisis parmi le groupe constitué de O, NH, et S.
- X₃ est choisi parmi -O-, C(=O) -OC(O)-, -C(O)O-, -OC(O)O-, -S-, -SS-, -SC(O)-, -OC(S)-, NR₁-, -NR₁C(O)-, -C(O)NR₁-, NR₁C(S)-, C(S)NR₁-, -OC(O)S-, -OC(S)O-, - SC(O)O-, -OC(S)S-, -SC(O)S-, -SC(S)O-, -SC(S)S-, OC(O)NR₁-, -OC(S)NR₁-, -NR₁C(S)O-, -NR₁C(O)S-, NR₁C(O)NR₂-, -NR₁C(S)NR₂-, -SC(O)S-, -SC(S)O-, -S(O)-, -S(O)₂-, - - P(O)(R₁)-, -P(O)(OR1)-, P(O)(R₁)O-, OP(O)(OR₁)-, OP(O)(R₁)O-, NR₁P(O)(R₂)-, - NR₁P(O)(OR₂)-, NR₁P(O)(R₂)O-, OP(O)(OR₁₎- et OP(O)(R₁)O- dans laquelle R₁ et R₂ sont indépendamment H ou CH₃.

7. Méthode *in vitro* pour le diagnostic, le diagnostic différentiel, le pronostic, l'évaluation du risque de, et/ou la surveillance d'un trouble chez un sujet qui comporte :
(a) la fourniture d'un échantillon du sujet susceptible de contenir des microvésicules,
(b) la capture des microvésicules éventuellement présentes dans l'échantillon en réalisant la méthode selon l'une quelconque des revendications 1 à 6, et
(c) la détection, la quantification et/ou la caractérisation des microvésicules capturées à l'étape (b) par complexion.
de préférence dans laquelle :
- le trouble est choisi parmi le groupe constitué du diabète et des troubles associés tels que la néphropathie diabétique, la neuropathie diabétique, la rétinopathie diabétique et le syndrome du pied diabétique, la sclérose en plaques, le cancer, la maladie d'Alzheimer, la maladie de Parkinson, l'anévrisme, le vasospasme cérébral, l'accident vasculaire cérébral et la maladie coronarienne, et/ou
- l'échantillon est du, ou provient de, sang, plasma sanguin et urine.

8. Molécule capable de se lier à des microvésicules et comportant au moins une fraction de formule (1a) comme suit : 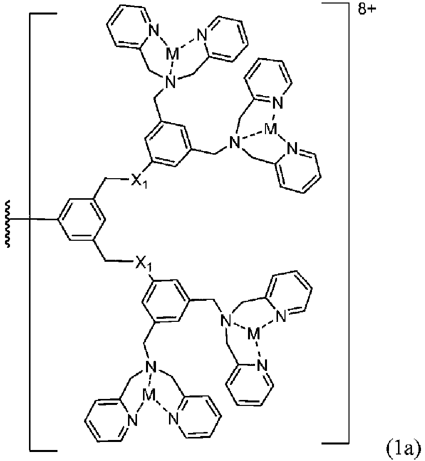 dans laquelle :
- chaque X₁ est indépendamment O, S, ou NH,
- chaque X₂, lorsqu'il est présent, est indépendamment O, S ou NH,
- M est choisi parmi Zn²⁺, Co²⁺, Cu²⁺ et Fe²⁺, de préférence Zn²⁺.

9. Molécule selon la revendication 8, qui comporte une fraction de formule (1b) 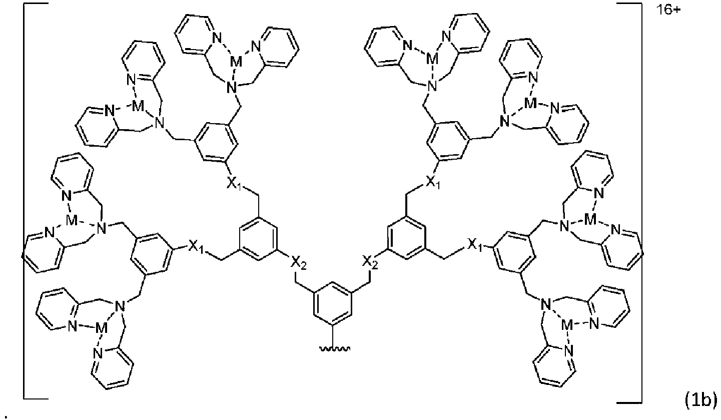 dans laquelle :
- chaque X₁ est indépendamment O, S, ou NH,
- chaque X₂, lorsqu'il est présent, est indépendamment O, S ou NH,
- M est choisi parmi Zn²⁺, Co²⁺, Cu²⁺ et Fe²⁺, de préférence Zn²⁺.

10. Molécule selon la revendication 8 ou 9, qui est un dendrimère comportant un noyau ramifié portant une pluralité de fractions de formule (I), ledit noyau ramifié comportant de préférence un groupe choisi parmi le 3,5-di(hydroxyméthyl)phénol, le 3,5-di(thiométhyl)phénol, le 3,5-di(thiométhyl)thiophénol, le 3,5-dialkylphénol, le 3,5-di(aminométhyl)phénol, le 3,5-dialkylphénol et le 3,5-di(aminométhyl)phénylamine comme élément constitutif, comportant de préférence le 3,5-di(hydroxyméthyl)phénol comme élément constitutif, et qui comporte de préférence en outre un moyen d'immobilisation sur un support fixé au niveau de l'extrémité d'une chaîne d'espacement.

11. Molécule selon la revendication 10, qui est de formule (IIb) et (IIc) : 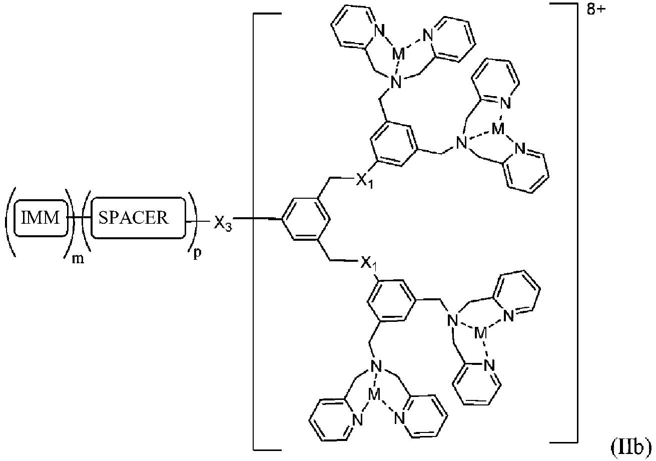
- et 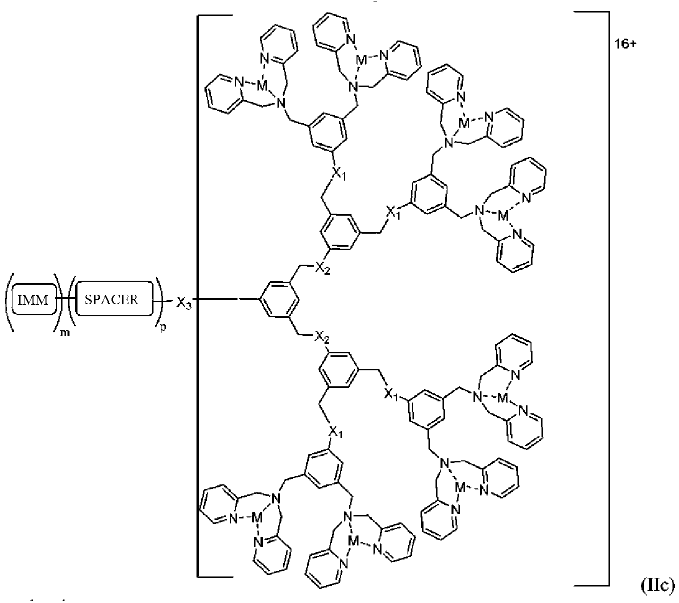 dans laquelle
- m et p sont indépendamment 0 ou 1,
- M est un cation métallique divalent,
- [IMM] est un moyen d'immobilisation covalente ou non covalente sur un support, et
- [SPACER] est choisi dans le groupe constitué d'un peptide, d'un polypeptide, d'un oligo- ou polysaccharide, d'une chaîne hydrocarbonée saturée ou insaturée éventuellement interrompue par un ou plusieurs hétéroatomes (par exemple S, O ou NH), présentant éventuellement un hétéroatome tel que S, O et NH sur au moins une de ses extrémités, et éventuellement substituée par un ou plusieurs substituants tels que hydroxyle, halogènes, alcoxy en C₁-C₃, -CN, -CF₃, ou alkyle en C₁-C₃, des polymères comprenant des homopolymères, copolymères et polymères séquencés, et des combinaisons de ceux-ci,
- chaque X₁ et X₂, lorsqu'ils sont présents, sont choisis parmi le groupe constitué de O, NH, et S.
- X₃ est choisi parmi -O-, C(=O) -OC(O)-, -C(O)O-, -OC(O)O-, -S-, -SS-, -SC(O)-, -OC(S)-, NR₁-, -NR₁C(O)-, -C(O)NR₁-, NR₁C(S)-, C(S)NR₁-, -OC(O)S-, -OC(S)O-, - SC(O)O-, -OC(S)S-, -SC(O)S-, -SC(S)O-, -SC(S)S-, OC(O)NR₁-, -OC(S)NR₁-, -NR₁C(S)O-, -NR₁C(O)S-, NR₁C(O)NR₂-, -NR₁C(S)NR₂-, -SC(O)S-, -SC(S)O-, -S(O)-, -S(O)2-, - - P(O)(R₁)-, -P(O)(OR1)-, P(O)(R₁)O-, OP(O)(OR₁)-, OP(O)(R )O-, NR₁P(O)(R₂)-, - NR₁P(O)(OR₂)-, NR₁P(O)(R₂)O-, OP(O)(OR₁₎- et OP(O)(R₁)O- dans laquelle R₁ et R₂ sont indépendamment H ou CH₃.

12. Molécule selon la revendication 11, dans laquelle le ligand de formule (IIb) ou (IIc) est tel que :
- m et p sont 1,
- M est Zn²⁺, Co²⁺, Cu²⁺ ou Fe²⁺, de préférence Zn²⁺,
- X₁ et X₂, lorsqu'ils sont présents, sont O,
- X₃ est O, NH, NHCO, CONH, O(C=O), (O=C)O, O(C=O)O, ou NHCONH,
- [IMM]-[SPACER] est NH₂-(CH₂)ᵣ- ou NH₂-[(CH₂)₂-O]ᵣ- avec r un entier de 2 à 10, et
- Ladite molécule comporte un ou plusieurs contre-anions choisis dans le groupe constitué de perchlorate, tosylate, nitrate, sulfate, sulfonate, thiosulfate, halogénure, hexafluorophosphate, tétraphénylborate, carbonate, et tétrafluoroborate.

13. Molécule selon la revendication 12 qui est choisie parmi des composés de formule (IIIb) et (IIIc) : 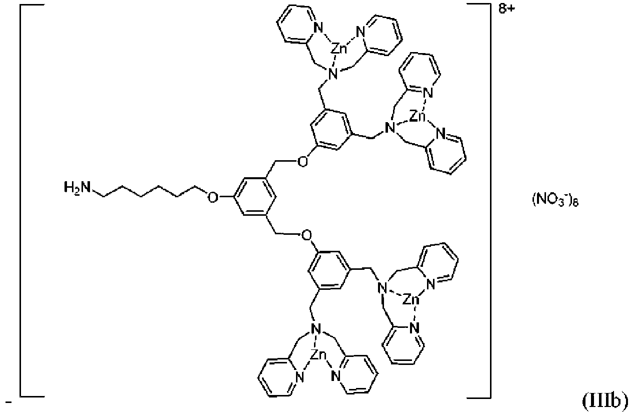 et

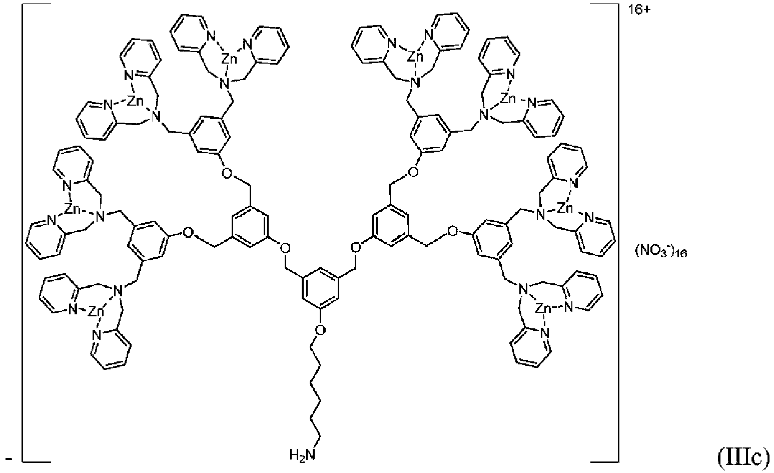

14. Support portant un ligand tel que défini dans l'une quelconque des revendications 8 à 13.

15. Utilisation d'un ligand tel que défini dans l'une quelconque des revendications 8 à 13, ou d'un support tel que défini dans la revendication 14 comme moyens de capture de microvésicules à partir d'un échantillon d'un sujet, de préférence pour le diagnostic, le pronostic ou le suivi d'une maladie chez un sujet telle que les cancers, les maladies parasitaires, le diabète et les troubles associés comprenant la néphropathie diabétique, la neuropathie diabétique, la rétinopathie diabétique et le syndrome du pied diabétique, la sclérose en plaques, le cancer, la maladie d'Alzheimer, la maladie de Parkinson, l'anévrisme, le vasospasme cérébral, l'accident vasculaire cérébral et la maladie coronarienne.
